# EUROPEAN PATENT SPECIFICATION

(11) **EP 0 804 613 B1**
(45) Date of publication and mention of the grant of the patent: **23.03.2005**
(21) Application number: 95900994.5
(22) Date of filing: 12.10.1994
(51) Int. Cl.: C12N 15/12, G01N 33/53, C12N 15/00, C07K 1/00, C07K 16/18, A61K 38/02, A61K 38/19, A61K 39/395, C07H 21/04, C12Q 1/68, C07K 14/47, A61K 48/00

(54) **DAP-2 TUMOR SUPPRESSOR GENE, PROTEIN ENCODED THEREBY AND USE OF SAID GENE AND PROTEIN**
DAP-2 TUMORSUPRESSORGEN, DURCH SIE KODIERTE PROTEIN UND VERWENDUNG BESAGTER GEN UND PROTEIN
GENE SUPPRESSEUR DE TUMEURS DAP-2, PROTEINE CODEE PAR CELUI-CI ET UTILISATION DESDITS GENE ET PROTEINE

(30) Priority: 12.10.1993 IL 10725093
(43) Date of publication of application: 05.11.1997
(73) Proprietor: YEDA RESEARCH & DEVELOPMENT COMPANY, LTD., 76100 Rehovot (IL)
(72) Inventor: KIMCHI, Adi, 43561 Raanana (IL)
(74) Representative: Desaix, Anne
(86) International application number: PCT/US1994/011598
(87) International publication number: WO 1995/010630

(56) References cited:
- EP-A- 0 475 623
- WO-A-93/07872
- US-A- 4 542 096
- US-A- 4 798 787
- US-A- 5 015 570
- L. P. DEISS ET AL: "Identification of a novel serine/threonine kinase and a novel 15-kDa protein as potential mediators of the gamma-interferon-induced cell death " EMBL DATABASE ENTRY HSDAP1, ACCESSION NUMBER X76105,11 December 1993, XP002102391 -& GENES DEVELOPMENT, vol. 9, no. 1, January 1995, pages 15-30, XP002102392
- BAKER S J ET AL: "SUPPRESSION OF HUMAN COLORECTAL CARCINOMA CELL GROWTH BY WILD-TYPE P53" SCIENCE, vol. 249, no. 4971, 24 August 1990, pages 912-915, XP000293056
- T.H.T. COETZER ET AL: "Anti-peptide antibodies to cathepsins B, L and D and type IV collagenase" JOURNAL OF IMMUNOLOGICAL METHODS., vol. 136, 1991, pages 199-200, XP002102393 NEW YORK US
- KISSIL ET AL: "Isolation of DAP3, mediator of IFN-gamma induced cell death" JOURNAL OF BIOLOGICAL CHEMISTRY, vol. 46, no. 270, 1995, page 27932 27932 XP002080572
- E. FEINSTEIN ET AL: "Assignment of DAP1 and DAPK-genes that positively mediate programmed cell death triggered by IFN-gamma-to chromosome regions 5p12.2 and 9q34.1, respectively." GENOMICS, vol. 29, 1 January 1995, pages 305-307, XP002080573
- PROC. NATL. ACAD. SCI. U.S.A., Volume 84, issued March 1987, N.V. KATRE et al., "Chemical Modification of Recombinant Interleukin 2 by Polyethylene Glycol Increases Its Potency in the Murine Meth A Sarcoma Model", pages 1487-1491.
- PROC. NATL. ACAD. SCI. U.S.A., Volume 82, issued August 1985, P.L. FAUST et al., "Cloning and Sequence Analysis of cDNA for Human Cathepsin D", pages 4910-4914.
- CELL, Volume 75, issued 03 December 1993, C. ABBADIE et al., "High Levels of C-Rel Expression are Associated with Programmed Cell Death in the Developing Avian Embryo and in Bone Marrow Cells In Vitro", pages 899-912.
- CELL, Volume 75, issued 19 November 1993, M. MIURA et al., "Induction of Apoptosis in Fibroblasts by IL-16-Converting Enzyme, a Mammalian Homolog of the C. Elegans Cell Death Gene Ced-3", pages 653-660.
- MOLECULAR AND CELLULAR BIOLOGY, Volume 11, No. 8, issued August 1991, G.P. OWENS et al., "Identification of mRNAs Associated with Programmed Cell Death in Immature Thymocytes", pages 4177-4188.
- LEUKEMIA RESEARCH, Volume 17, No. 8, issued August 1993, J. MOORE et al., "Mutant H-Ras Over-Expression Inhibits a Random Apoptotic Nuclease in Myeloid Leukemia Cells", pages 703-709.
- SCIENCE, Volume 252, issued 05 April 1991, L.P. DEISS et al., "A Genetic Tool Used to Identify Thioredoxin as a Mediator of a Growth Inhibitory Signal", pages 117-120.
- J. BIOLOGICAL CHEMISTRY, Volume 268, No. 7, issued 05 March 1993, P. WONG et al., "Genomic Organization and Expression of the Rat TRPM-2 (Clusterin) Gene, a Gene Implicated in Apoptosis", pages 5021-5031.
- J. BIOLOGICAL CHEMISTRY, Volume 268, No. 15, issued 25 May 1993, N. ITOH et al., "A Novel Protein Domain Required for Apoptosis. Mutational Analysis of Human Fas Antigen", pages 10932-10937.
- EXPERIMENTAL HEMATOLOGY, Volume 21, No. 11, issued October 1993, T. NAKANO et al., "Transfection of Interferon-gamma Gene in a Mouse Bone Marrow Stromal Preadipocyte Cell Line Causes Apoptotic Cell Death", pages 1498-1503.
- MOLECULAR NEUROSCIENCE, Volume 4, No. 4, issued April 1993, S.R. D'MELLO et al., "SGP2, Ubiquitin, 14K Lectin and RP8 mRNAs are not Induced in Neuronal Apoptosis", pages 355-358.
- NATURE, Volume 352, issued 25 July 1991, E. YONISH-ROUACH et al., "Wild-Type p53 Induces Apoptosis of Myeloid Leukaemic Cells that is Inhibited by Interleukin 6", pages 345-347.
- HENNING K. A., PH.D. THESIS Stanford University, USA. "The molecular genetics of human diseases with defective DNA damage processing", August 1993. Pubished by U.M.I. 300 N Zeeb Rd. Ann Arbor MI 48106-1346 USA. (1-800-521-0600)
- EMBL DATABASE ENTRY HS18321, ACCESSION NUMBER U18321, 09 December 1994
- INBAL ET AL: 'DAP kinase links the control of apoptosis to metastasis' NATURE vol. 390, November 1997, pages 180 - 184
- COHEN O ET AL: 'DAP-kinase is a Ca2+/calmodulin-dependent, cytoskeletal associated protein kinase, with cell death-inducing functions that depend on its catalitic activity' EMBO JOURNAL vol. 16, no. 5, 1997, pages 998 - 1008

## Description

### FIELD OF THE INVENTION

The present invention relates to the field of tumor-suppressor genes in general, and cytokine-induced cell death in particular.

### BACKGROUND OF THE INVENTION

One of the factors which determines the proliferation state of cells is the balance between the growth-promoting effects of proto-oncogenes, and the growth-constraining effects of tumor-suppressor genes.

One mechanism by which these tumor-suppressor genes exert their growth-constraining effect is by inducing the cell to undergo a physiological type of death. Such a controlled cell death is evident in a multitude of physiological conditions including metamorphosis, synaptogenesis of neurons, death of lymphocytes during receptor repertoire selection, controlled homeostasis in the bone-marrow and other proliferative tissues, and others. Such cell death is regulated by the interaction of the cell with other cells or with cell products, for example through the activity of suitable cytokines.

Genetic mutation that inactivates the suppressor genes, liberate the cell from normal growth constraint imposed by other cells, resulting in an uncontrolled growth of the cell without any relation to external signals. This uncontrolled growth is a step in tumorigenesis.

To date, only a few tumor-suppressor genes have been fully characterized including the *retinoblastoma (Rb) gene, p53, DCC, Nm23 WT-1, NF-1, APC,* and *ras suppressor* genes. A mutation in either of the above genes, probably in both alleles, which leads to either blockage of expression, or production of a faulty protein, hampers the normal control of growth and viability of cells and may thus give rise to cancer.

Growth-inhibiting cytokines have a double effect on the target cell. They can either inhibit the proliferation of the cell, and/or give rise to cell death. To date, blockage or activation of expression of known tumor-suppressor genes was shown to counteract or enhance, respectively, cytokines' inhibition of cells' growth (reviewed by A. Kimchi, 1992, *J. Cell Biochem.,* **50**:1-9) but did not have any effect on the death promoting action of cytokines. For example, the growth inhibitory response to cytokines such as TGF-β , was markedly reduced by the inactivation of the *Rb* gene, or the response to IL-6 was enhanced by introducing activated *p53* genes (Pietenpol *et al*., 1990, *Cell,* **61**:777-785; Levy *et al*., 1993, *Mol. Cell. Biol*., 13:7942-7952).

Thioredoxin, a small hydrogen carrier protein, has previously been implicated in the IFN-γ-mediated growth arrest of HeLa cells (Deiss, L.P. and Kimchi, A. (1991) Science 234:117-120).

A fragment of a cDNA sequence has been sequenced in the context of the Human Genome Project consisting of an arbitrary sequencing of expressed sequence tags of the human genome (Adams *et al., Science,* 21 June 1991, vol. 252 pp 1651-1656; EMBL no. M62147). No information is provided by the database concerning the putative function of the gene comprising this cDNA sequence.

### SUMMARY OF THE INVENTION

In the following, the term "*programmed cell death "* will be used to denote a physiological type of cell death which results from activation of some cellular mechanisms, i.e. death which is controlled by the cell's machinery. Programmed cell death may, for example, be the result of activation of the cell machinery by an external trigger, e.g. a cytokine, which leads to cell death.

The present invention is based on the pioneering finding that inhibition of expression of certain genes counteracts the cytokine-induced cell death. Namely, as long as these genes function normally, cytokine induces cell death; once the expression of said genes is inhibited, the cytokine-induced cell death is inhibited. It follows therefrom that the normal expression product of these genes is involved in programmed cell death, especially in cytokine-induced cell death. In HeLa cells, IFN-γ induces a biphasic process, which comprises an initial cytostatic phase and a subsequent cytotoxic phase (programmed cell death). The novel genes discovered in accordance with the present invention were found to affect only the later, cytotoxic phase. These genes will be referred to herein as *"DAP* (death-associated protein) *genes ".* DNA molecules comprising a coding sequence encoding the expression products of the DAP genes, or expression products having a similar biological activity, will be referred to herein at times collectively as *"DAP DNA molecules ".* The expression products of the DAP DNA molecules will be referred to herein at times collectively as *"DAP products"*.

According to one aspect of the present invention, to be referred to herein as *"the death-promoting aspect*", the above DAP DNA molecules, expression vectors comprising them, or DAP products are used for promoting death of normal or tumor cells. A particular application of the death-promoting aspect is in therapy of diseases or disorders associated with uncontrolled, pathological cell growth; e.g. cancer, psoriasis, and others. The use of DAP DNA molecules in gene therapy or DAP products if produced extracellularly, in accordance with the death-promoting aspect of the invention, may be in conjunction with cytokines, e.g. IFN-γ.

According to another aspect of the invention, to be referred to herein as "*the death-preventing aspect*", agents which prevent the expression of said DAP DNA molecules, or agents which antagonize, inhibit or neutralize the DAP products, are used for protecting cells from programmed cell death. Examples of possible applications of the death preventing aspect of the invention are in prevention of cell death in various degenerative neurological diseases, such as Alzheimer's disease or Parkinson's disease, which are associated with premature death of particular subsets of neurons; prevention of death of T-cells in AIDS patients, which death resembles programmed cell death; prevention of rejection-associated cell death in transplants which is believed to result, at least in part, from programmed cell death; protection of normal cells from the cytotoxic effects of certain anti-cancer therapies; etc.

According to a further aspect of the present invention, referred to herein at times as "*the screening aspect*", DAP DNA molecules are used in order to screen individuals for predisposition to cancer. In accordance with this aspect, the screening is carried out by comparing the sequence of each of the DAP DNA molecules to each of the respective DAP genes in the individual. The absence of a DAP gene, a partial deletion or any other difference in the sequence that indicates a mutation in an essential region, may result in a loss of function and as a consequence a predisposition for cancer. For screening, preferably a battery of different DAP genes may be used.

The DAP genes seem to play an important role in programmed cell death and the inhibition of their expression or neutralization of their expression products protects the cell from cytokine-promoted cell death. Examples of such genes are those whose sequences are depicted in Figs. 6, 8 and 12, or whose partial sequences are depicted in Figs. 13 and 14. The gene for the known protease cathepsin D, whose sequence is depicted in Fig. 15, is also revealed here for the first time as functioning as a DAP gene.

DAP DNA molecules useful in the death-promoting aspect of the invention may have the nucleic acid sequence of the DAP gene or other sequences which encode a product having a similar biological activity to that of the DAP product. Such DAP molecules include DNA molecules having a sequence other than that of the DAP gene but which, owing to the degenerative nature of the genetic code, encode the same protein or polypeptide as that encoded by the DAP gene.

It is well known that it is possible at times to modify a protein by replacing or deleting certain amino acids which are not essential for a certain biological function, or adding amino acids in a region which is not essential for the protein's biological function, without such modification essentially affecting the biological activity of the protein. Thus, a DAP DNA molecule useful in the death promoting aspect of the invention may also have a modified sequence encoding such a modified protein. The modified sequence has a sequence derived from that of the DAP gene or from that of the above degenerative sequence, in which one or more nucleic acid triplets (in the open reading frame of the sequence), has been added, deleted or replaced, with the protein product encoded thereby retaining the essential biological properties of the DAP product. Furthermore, it is known that at times, fragments of proteins retain the essential biological properties of the parent, unfragmented protein, and accordingly, a DAP DNA molecule useful in the death promoting aspect of the invention may also have a sequence encoding such fragments.

A DNA molecule useful in the death-preventing aspect of the invention may have a sequence which is an antisense sequence to that of the DAP gene, or an antisense sequence to part of the DAP gene, blocking of which is sufficient to inhibit expression of the DAP gene. The part of the gene can be either the coding of the non-coding part of the DAP gene. The mRNA transcripts of the antisense sequences hybridize to the mRNA transcripts of the DAP gene and interfere with the final protein expression. Another DNA molecule useful in the death-preventing aspect of the invention is a DNA molecule coding for a modified DAP product which is capable of inhibiting the activities of the unmodified DAP product in a dominant negative manner, such as catalytically inactive kinase (DAP-kinase) or any other modified protein whose presence in the cell interferes with the normal activity of the native protein, for example by producing faulty hereto dimers comprised of modified and unmodified proteins which are inactive and the like.

DNA molecules useful in the screening aspect of the invention comprise the sequence of a DAP gene or a sequence of a fragment thereof. Additionally, also the above antisense DNA sequences may be used in the screening aspect of the invention.

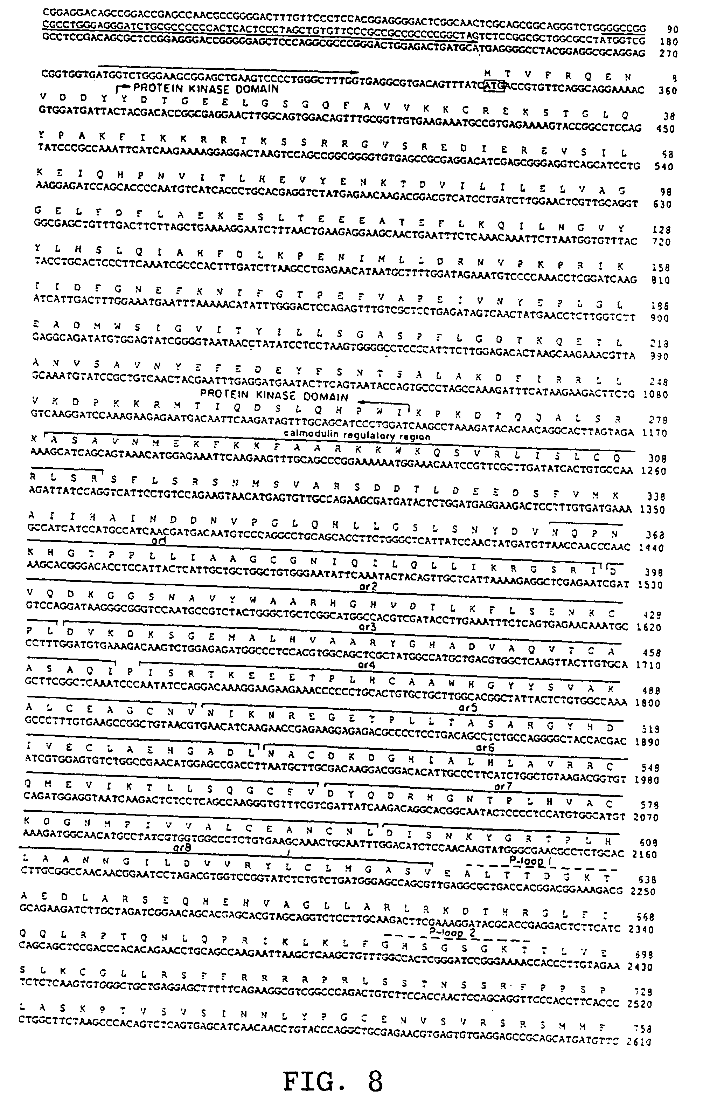

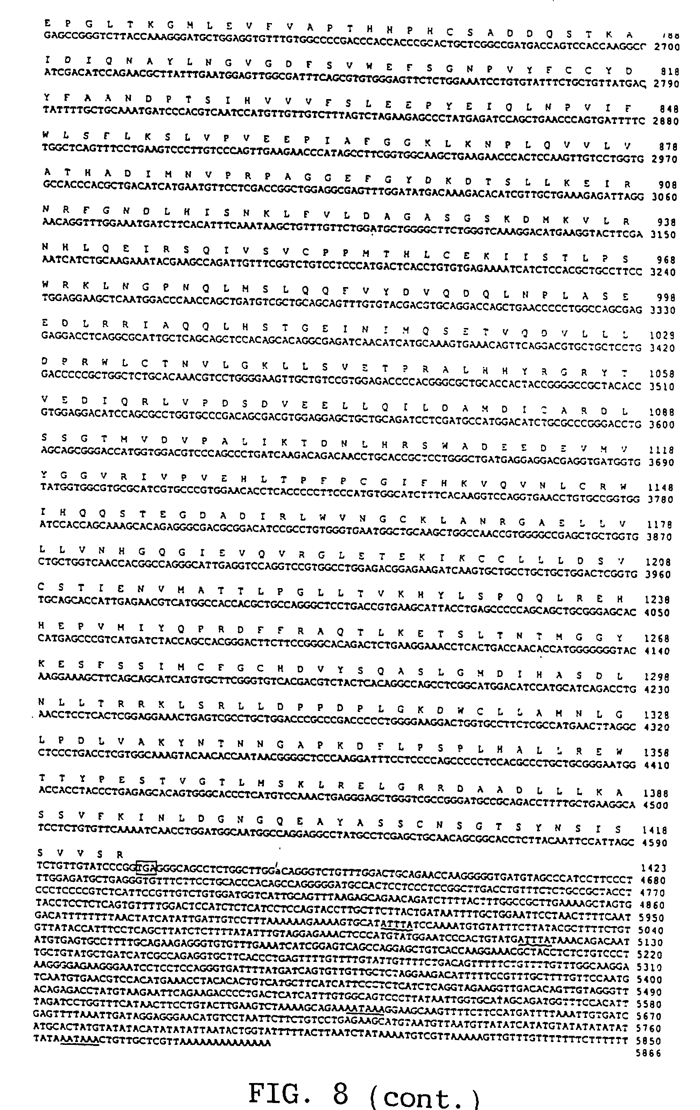

The present invention thus provides an isolated DNA molecule comprising a sequence selected from the group consisting of:
(a) a nucleic acid sequence expressed in cells, the expression product of which is involved in cytokine-induced programmed cell death, being a DNA sequence comprising a coding sequence beginning at the nucleic acid triplet at position 337-339 and ending at the triplet at position 4603-4605 of the sequence depicted in Fig. 8;
(b) a DNA sequence encoding the same protein or polypeptide encoded by the gene defined in (a);
(c) a modified DNA sequence of (a) or (b) in which one or more nucleic acid triplets has been added, deleted, or replaced, the protein or polypeptide encoded by the modified DNA sequence mediating the cytokine-induced programmed cell death similarly to the protein or polypeptide encoded by the gene as defined under (a) or (b);
(d) fragments of any of the DNA sequences of (a), (b) or (c), encoding a protein or a polypeptide having said biological activity;
(e) a sequence which is an antisense to the entire or part of the DNA molecule under (a) and capable of inhibiting the expression of said gene; and
(f) a modified DNA sequence of (a) or (b) in which one or more nucleic acid triplets has been added, deleted or replaced, the protein or polypeptide encoded by the modified sequence having dominant negative effect manifested by the ability of said protein or polypeptide to inhibit said cytokine-induced programmed cell death.

In a specific embodiment, the invention concerns an isolated DNA molecule in which either
a) one or more nucleic acid triplets, which encode amino acid which are not essential for cytokine-induced programmed cell death activity, has been deleted or replaced; or,
b) one or more nucleic acid triplets encoding amino acids has been added in a region which is not essential for cytokine-induced programmed cell death activity;
the protein or polypeptide encoded by the DNA molecule having essentially the same cytokine-induced programmed cell death activity as that encoded by any one of the DNA molecules of the invention.

An antisense sequence is the sequence beginning at position 3781-4148 of the DAP-2 gene in Fig. 8.

In another specific embodiment, the invention relates to a fragment of any one of the isolated DNA molecules of the invention encoding a protein or polypeptide retaining cytokine-induced programmed cell death activity present in the protein or polypeptide encoded by any one of the DNA molecule of the invention with the proviso that nucleotide sequence 2556-2814 of the sequence of figure 8 is excluded.

The present invention also provides a vector comprising any of the above DNA molecules, the vector comprising also sequences required for maintaining and replicating it in a host cell. Vectors in accordance with the present invention may be transfer vectors for propagating and replicating the DNA sequences in a host cell or may be expression vectors comprising also sequences required for translation of said DNA sequences into an mRNA. Examples of such expression vectors are plasmids, e.g. episomes or viruses.

Examples of episomes are those constructed by using the vehicles pTKO1, pTKO2 and pTKO3 (Deiss and Kimchi, *supra*).

The present application also provides a DAP-2 product which is a protein or polypeptide encoded by a DNA molecule of the invention, with the exception of the DNA molecules having an antisense sequence, or such a protein or polypeptide which has been chemically modified, for example, by methylation, glycosylation, etc. An example of a DAP-2 product is that having the amino acid sequence depicted in Fig. 8. The DAP-2 product is useful in the death-promoting aspect of the present invention. In accordance with this aspect, the protein may be administered to patients, in particular, to cancer patients, which administration may cause death of the transformed cells.

The present invention further provides agents which inhibit, antagonize or neutralize the DAP-2 product, which are useful in the death-preventing aspect of the invention. Such agents are for example, antibodies directed against the DAP-2 product, inhibitors or antagonists of the DAP-2 product which are able to counteract their effect and prevent the death-promoting activity of the DAP-2 product.

The present invention further provides a pharmaceutical composition comprising a pharmaceutically acceptable carrier and an active agent being selected from the group consisting of: (i) an expression vector comprising a DNA molecule of the invention; (ii) a DAP-2 product of the invention; and (iii) an antibody, inhibitor or antagonist to the DAP-2 product. The pharmaceutical composition of the present invention may also comprise means for targeting said active agent to the desired cell or tissue. Depending on the nature of the active agent, the composition is useful either in accordance with the death-promoting or the death-preventing aspect of the invention, the pharmaceutical composition may also comprise a cytokine, e.g. IFN-γ, in combination with a suitable DAP-2 product, or with an expression vector comprising a suitable DAP-2 molecule.

Further provided by the present invention is a method of treatment comprising administering said active agent to an individual. Similarly as in the pharmaceutical composition, depending on the nature of said active agent, the method is practicable in either the death-promoting aspect of the invention or the death-preventing aspect of the invention. In the death-promoting aspect of the invention, said active agent may be administered in conjunction with a cytokine, e.g. IFN-γ.

In accordance with the screening aspect of the invention, there is provided a method for detecting the absence of the DAP-2 gene, a partial deletion or a mutation (i.e. point mutation, deletion or any other mutation) in the DAP-2 gene of an individual, comprising probing genomic DNA or cDNA from the individual with a DNA probe or a multitude of DNA probes having a complete or partial sequence of the DAP-2 gene or having a sequence which is an antisense to the complete or partial sequence of the DAP-2 gene. A particular application of the screening aspect of the invention is in the screening for individuals having a predisposition to cancer, an absence of the gene or a detected mutation or deletion indicating that the individual has such predisposition. The method in accordance with the screening aspect typically comprises the following steps:
(a) obtaining a sample of either genomic DNA from cells of the individual or cDNA produced from mRNA of said cells;
(b) adding one or more DNA probes each of said probes comprising a complete or partial sequence of a DAP-2 gene, or a sequence which is an antisense sequence to the complete or partial sequence of the DAP-2 gene;
(c) providing conditions for hybridization between the DNA probe or probes and the DNA of said sample;
(d) on the basis of the hybridization determining whether the DAP-2 gene is absent or there is a match between the sequence of the DNA probe or probes and a sequence in the DNA of said sample or a mismatch, a mismatch indicating a deletion or a mutation in the genomic DNA and a predisposition to cancer in the tested individual.

A specific embodiment of the screening aspect of the present disclosure involves use of a complete or partial sequence of that shown in Fig. 8, or an antisense of the complete or partial sequence in Fig. 8.

The mutation in the DAP-2 gene indicating a possible predisposition to cancer can also be detected by the aid of appropriate antibodies which are able to distinguish between a mutated and non-functional and a normal functional DAP-2 gene product.

### DESCRIPTION OF THE DRAWINGS

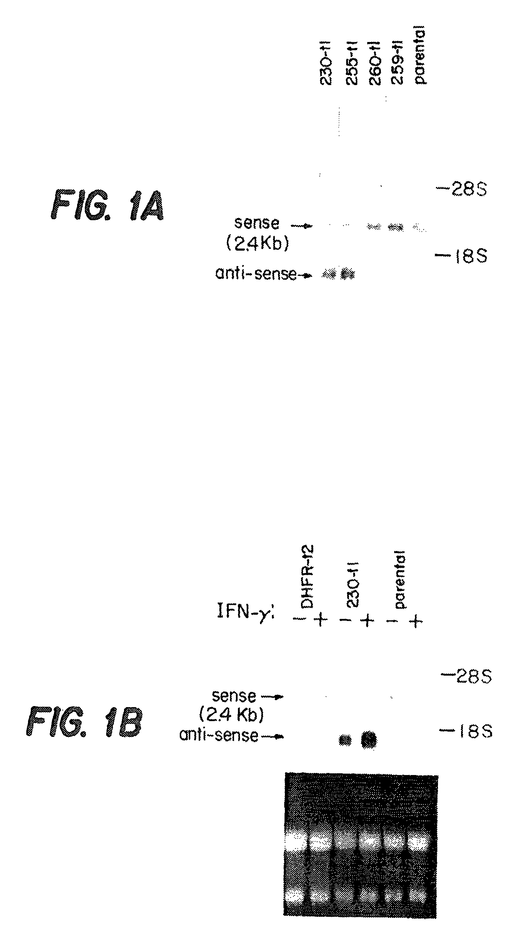

**Figs. 1 A-D** show RNA and protein expression of the DAP-1 gene, wherein:
   **Fig. 1(A)** shows a Northern blot analysis of sense and antisense mRNA obtained from HeLa cells transfected with the constructs 230, 255, 260, 259 and control cells (parental cells) and probed by labeled cDNA fragments from construct 230. Total RNA was prepared from HeLa cells either before (parental) or after transfection with pTKO1 constructs #230 or #255 (group 1), #260 (group 5) and #259 (group 3) designated 230-t1, 255-t1, 260-t1 and 259-t1, respectively. Twenty µg RNA were processed on Northern blots and DNA fragment #230 was used as a probe. The arrows point to the position of sense and antisense RNAs.
   **Fig. 1(B)** shows a Northern blot analysis of sense and antisense mRNA obtained from HeLa cells transfected with control construct (DHFR-t2), 230 construct or control cells (parental) cells treated with (+) or without (-) 750 U/ml of IFN-γ for 24 h. The RNA was extracted from the indicated HeLa cells which were grown for 4 days in the absence (-) or presence (+) of IFN-γ (750 U/ml). the Northern blot containing 20 µg RNA samples was hybridized with the cDNA insert of γ1 phage. The Ethidium Bromide staining of the mRNA samples is shown.

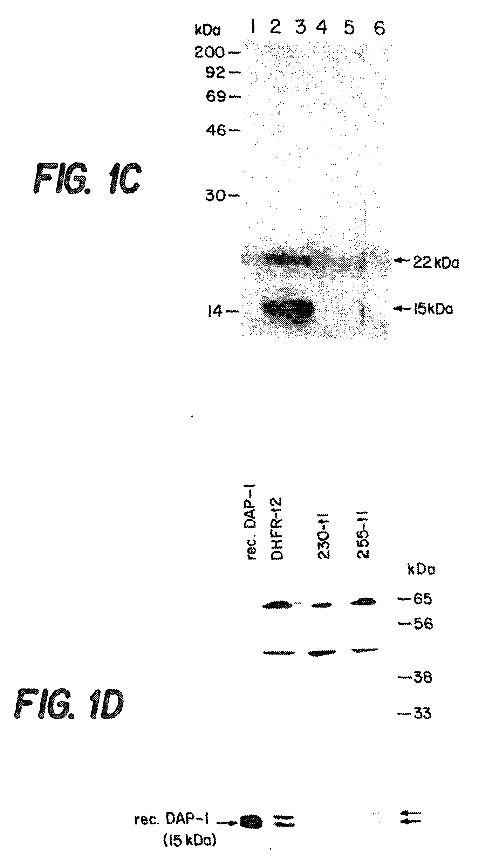

   **Fig. 1(C)** shows an SDC polyacrylamide electrophoresis gel of the expressed protein product of DAP-1 cDNA translated *in vitro* in a reticulocyte lysate preparation. *In vitro* translation of RNA (0.5 µg) transcribed from the λ1 cDNA (lane 2) and from the subclones p6, p4, p5 and p8 are shown in lanes 3-6, respectively. Lane 1 corresponds to the background obtained in the absence of RNA administration to the reticulocyte lysates. The labeled proteins were fractionated on 12% SDS polyacrylamide gels. The position of the radioactive molecular weight markers (Amersham) is marked. The two translated proteins, the major 15kDa and minor 22kDa proteins, are indicated by arrows.
   **Fig. 1(D)** shows an immunoblot analysis of recombinant and cellular 15kDa DAP-1 protein. Bacterially produced DAP-1 protein (300 ng) and the indicated HeLa cell extracts (350 µg) were fractionated on SDS polyacrylamide gels (12%), blotted to nitrocellulose and reacted with affinity purified antibodies generated against the 15kDa DAP-1. The cells were treated with IFN-γ (750 U/ml) for 4 days before their extraction. The two arrows point to the position of the cellular DAP-1 protein. The antibodies also recognize two non-relevant bands of 60 and 45 kDa that are not modulated by the antisense RNA expression. Quantitation of the reduction in DAP-1 protein was done by densitometric analysis. The calibration of the protein content in each slot was done by referring to the signals of the non-relevant bands. The prestained protein markers (Sigma) are marked.

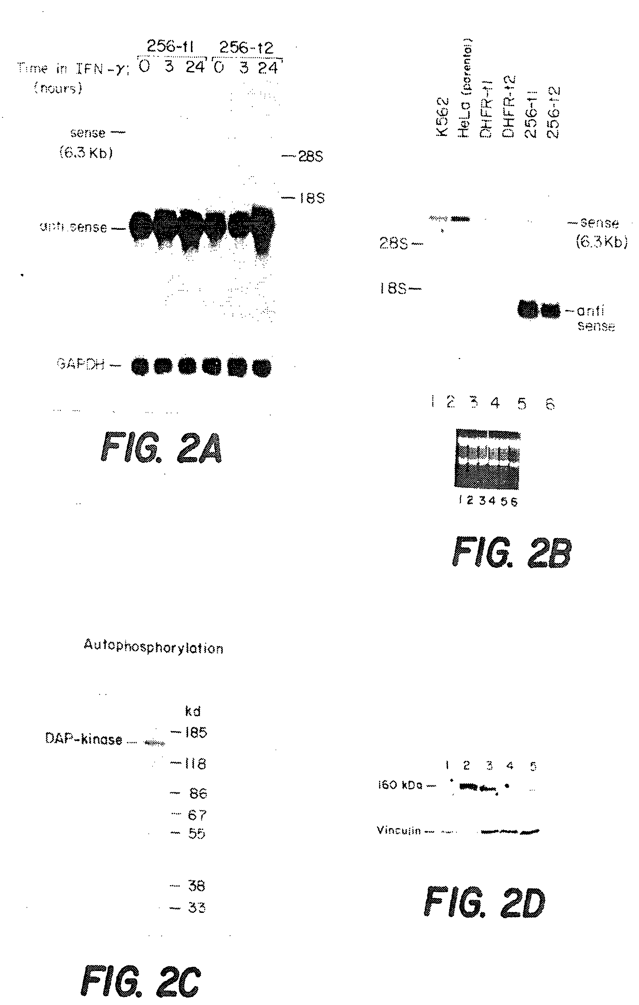

**Figs. 2 A-D** show RNA and protein expression of the DAP-2 gene, wherein:
   **Fig. 2(A)** shows a Northern blot analysis of sense and antisense mRNA obtained from two clones of HeLa cells transfected with the control constructs (DHFR-t1 and DHFR-t2) and two clones of cells transfected with the 256 construct (t1 and t2). Total RNA was prepared from the 256-t1 and 256-t2 HeLa cell transfectants either before (0 hours) or at 3 and 24 hours after treatment with IFN-γ (750 U/ml) and 20 µg samples were processed on Northern blots. Fragment #256 was used as a probe. The position of the sense and antisense mRNAs is indicated. The GAPDH mRNA levels were used for the calibration of the RNA amounts in each blot.
   In **Fig. 2(B)** the blot consists of total RNA (20 µg) from K562 cells, parental HeLa cells, the two DHFR-transfected HeLa cell populations and the two HeLa cell populations that were transfected with the pTKO1-256. The blot was hybridized with the cDNA insert of λ29. The Ethidium Bromide staining of the RNA samples is shown.
   **Fig. 2(C)** shows an *in vitro* phosphorylation assay. Cell lysates were prepared from COS-7 cells either before (lane 1) or after transfection with the PECE-FLAG expression vector that carries the coding region of the λ29 cDNA (lane 2). Samples of 400 µg were immunoprecipitated with anti-FLAG™ (M2) monoclonal antibodies (IBI) and subjected to phosphorylation assays.
   **Fig. 2(D)** shows immunoblot analysis of recombinant and cellular DAP-2 protein. The COS-7 cells were transiently transfected with the PECE-FLAG-DAP-2 expression vector. Samples of cell lysates, 100 µg from COS-7 cells and 400 µg from HeLa cells, were fractionated on SDS polyacrylamide gels (7.5%), immunoblotted and reacted with affinity purified polyclonal antibodies raised against the N-terminal DAP-2 peptide. In the lower panel the blot was reacted with monoclonal antibodies against vinculin (Sigma Immunochemicals). Lanes: 1, non-transfected COS-1 cells; 2, transfected COS-1 cells; 3, DHFR-t1 cells; 4, 256-t1 cells; 5, 256-t2 cells. In lane 2 the same 160 kDa protein was also detected with anti-FLAG™ (M2) monoclonal antibodies (IBI) (not shown).

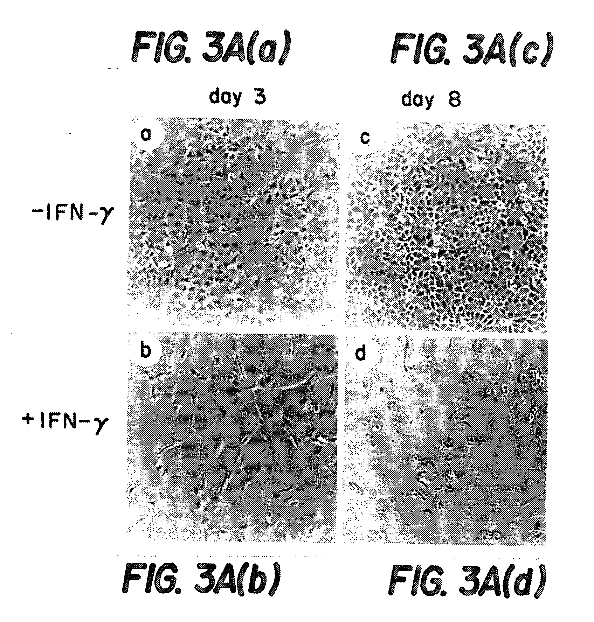

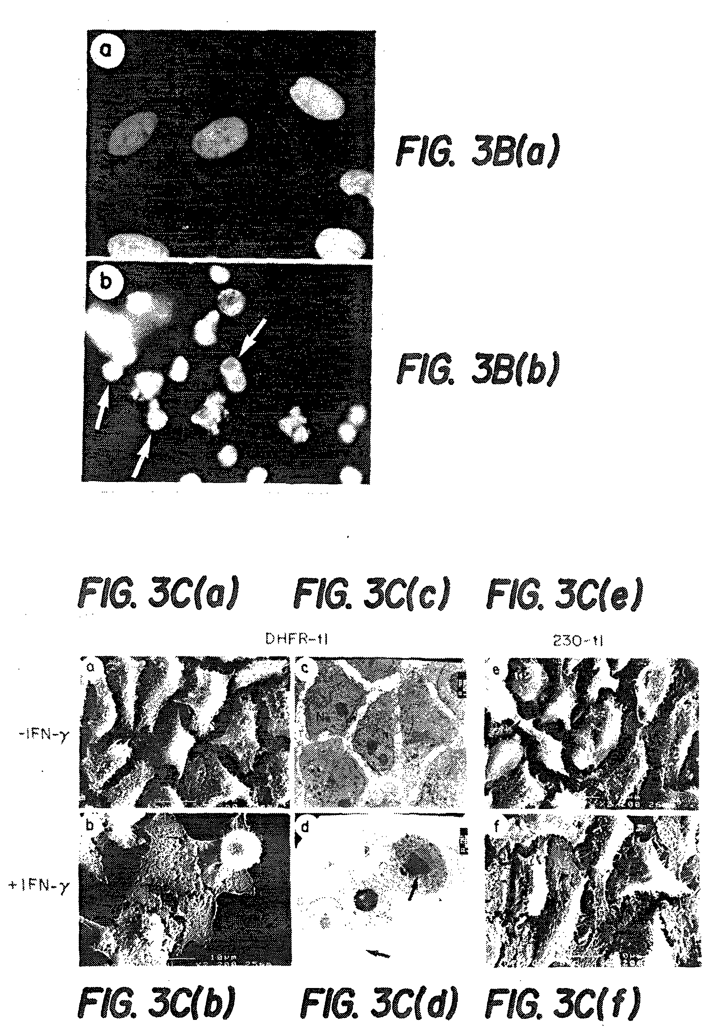

**Figs. 3 A-C** show morphological features of the cytostatic and cytotoxic responses to IFN-γ in HeLa cells. All cultures were seeded at an initial density of 10,000 cells per cm².
   **Fig. 3(A)** shows light microscopy of HeLa cells transfected with pTKO1-DHFR construct (DHFR-t1 cells), on days 3 and 8 of culturing in the absence (a,c) or the presence (b,d) of IFN-γ (750 U/ml). (Magnification x 400). Note the absence of refractile mitotic cells during the cytostatic phase of responses to IFN-γ (in b) and the appearance of round cells that were detached from the substratum during the killing phase (in d).
   **Fig. 3(B)** shows staining of DNA with DAPI; a. DHFR-t1 non-treated cells removed by trypsinization and mounted on glass slides. b. Detached DHFR-t1 cells collected 7 days after IFN-γ treatment. Nuclei with condensed or fragmented chromatin are indicated by arrows. (Magnification x 1000).
   **Fig. 3(C)** shows scanning and transmission electron micrographs of cells transfected with the control construct DHFR-t1 and the 230-t1 construct. DHFR-t1 HeLa cell populations (a-d) and the 230-t1 antisense transfected cells (e and f), were cultured either in the absence (a, c, e) or in the presence (b, d, f) of IFN-γ (750 U/ml). (a,b,e,f), scanning electron micrographs were taken after 7 days using GSM 6400 SEM (Jeol). Bars=10 mm (x2200 magnitude in all the four samples). (c and d), transmission electron micrographs taken after 7 days using TEM (Philips 410) at a magnitude of x2800. The condensed nuclei and the surface blebs are indicated by arrows.

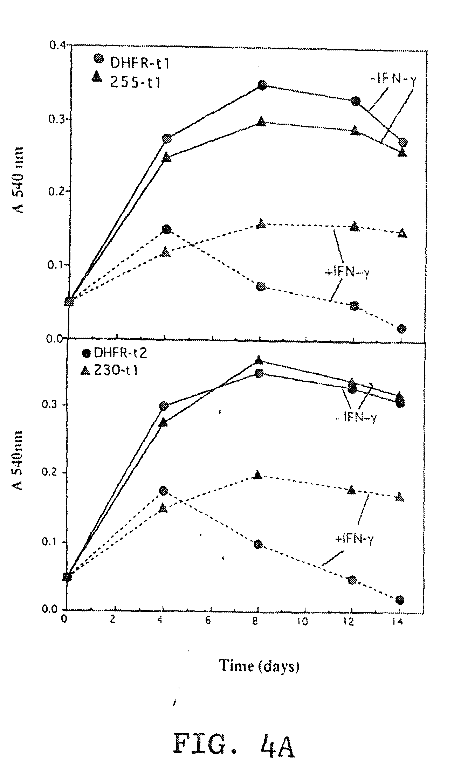

**Figs. 4 A-C** show that the antisense RNA expression from plasmids of groups 1 and 2 reduces the susceptibility of HeLa cells to the killing effects of IFN-γ but has no effect on early IFN-γ signalling.

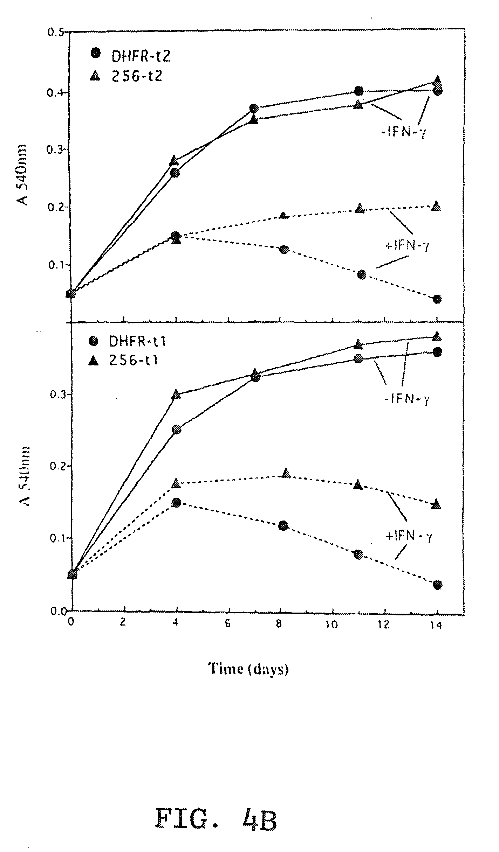

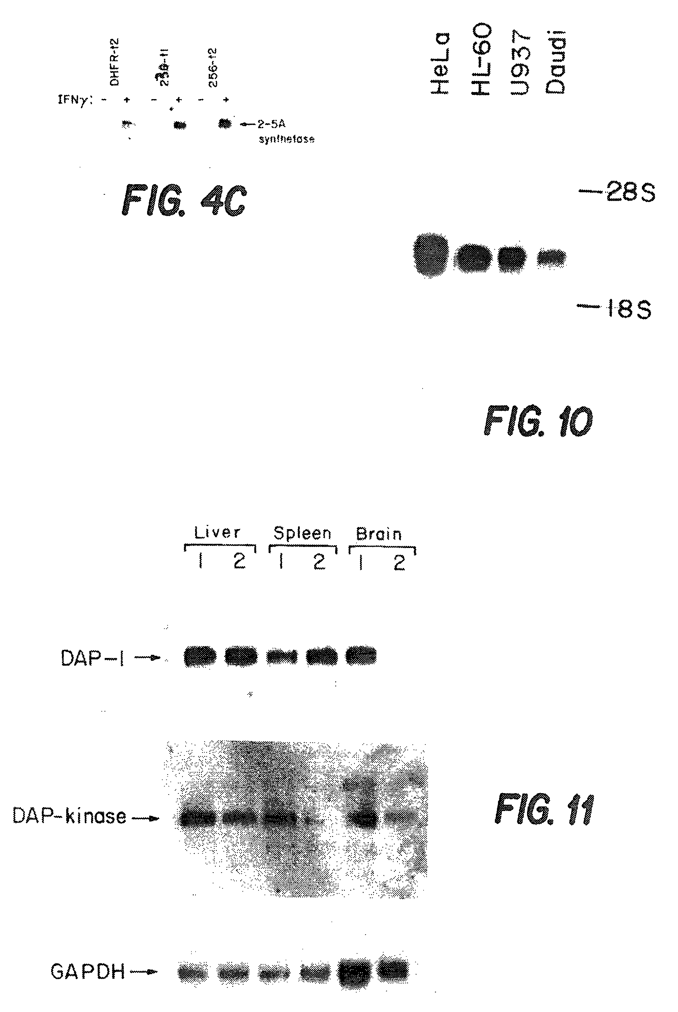

   **Figs. 4 (A-B)** show the number of viable cells as determined by light absorption at 540 nm, as a function of time; the cells being transfected either with the control construct DHFR-t1 (• - 1(A) and 1(B)); the 255 or 230 construct (▲ - 1(A)) or with two clones t1 and t2 of the 256 construct (▲ - 1(B)). The results are shown both for cell growth with (+) and without (-) administration of 750 U/ml of IFN-γ. Each point is the average of a quadruplicate determination with a SD that ranged between 2-5%.
   **Fig. 4(C)** shows a Northern blot analysis of 2-5A synthetase gene induction. The indicated HeLa cell transfectants were incubated for 24 hours in the presence (+) or absence (-) of IFN-γ (750 U/ml). Twenty mg of total RNA were analyzed. The cDNA of the 2-5A synthetase was used as probe.

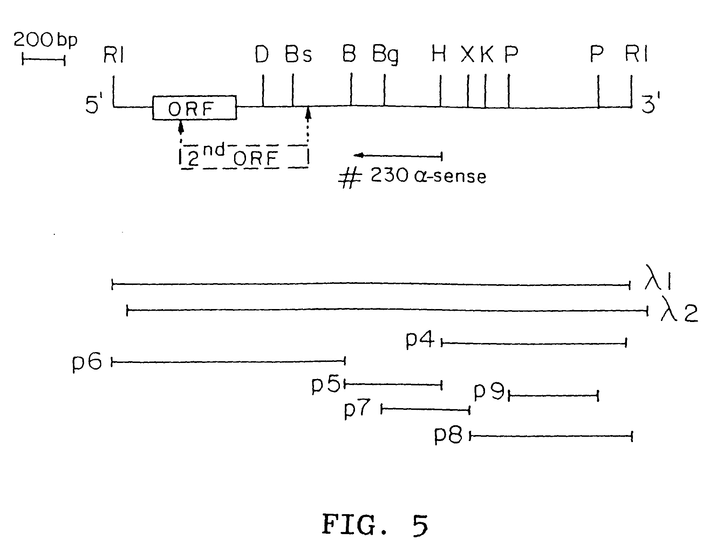

**Fig. 5** shows the restriction map of the λ1 cDNA clone that carries the DAP-1 cDNA.

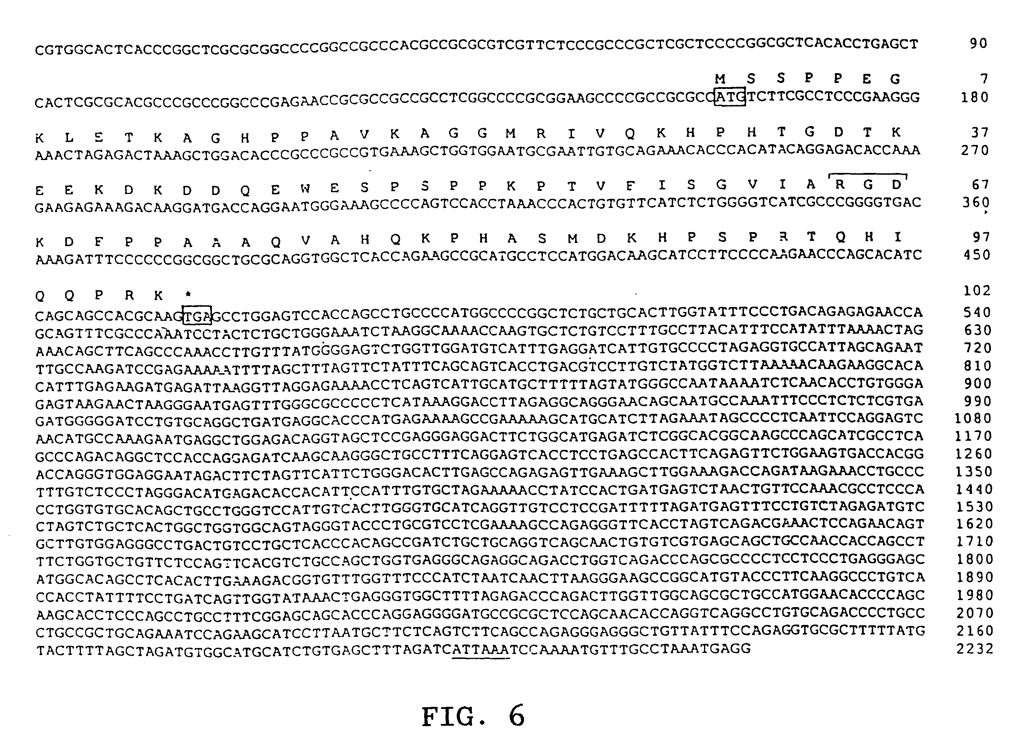

**Fig. 6** shows the DNA sequence and predicted amino acid sequence of DAP-1.

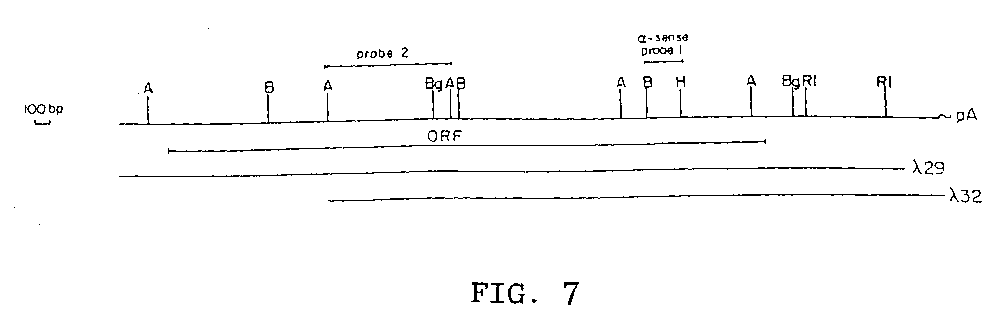

**Fig. 7** shows the restriction map of the λ29 cDNA clone, that carries the DAP-2 cDNA.
**Fig. 8** shows the DNA sequence and predicted amino acid sequence of DAP-2.

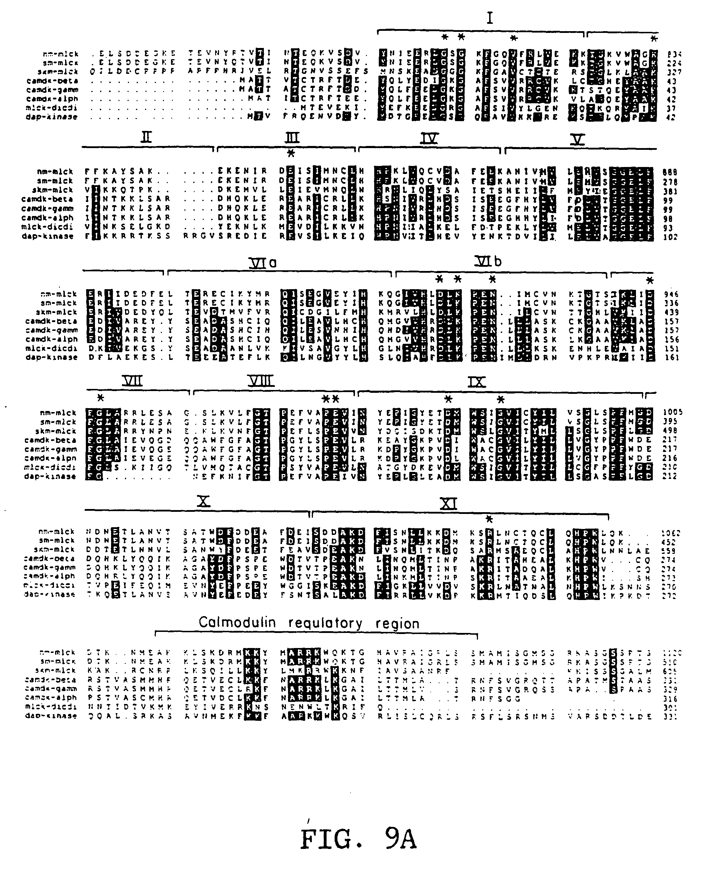

**Figs. 9 A-C** show DAP-2 sequence homologies to other serine/threonine kinases and alignment of the ankyrin repeats of DAP-2, wherein:
   In **Fig. 9(A)** the protein kinase domain sequences of the DAP-2 are aligned with the corresponding domains of other calmodulin-dependent kinases. The kinase subdomain structure (numbered I-XI) and the region implicated in calmodulin recognition and binding (designated as calmodulin regulatory region) are indicated. The obligatory conserved amino acids within the kinase domain are labeled with asterisks. Numbers at the right mark positions relative to the N-terminus of primary translational products of each kinase. Solid background indicates identical amino acids within the compared kinases. Stippled background indicates positions where the amino acids are not identical but similar. nm-mlck - non-muscle myosin light chain kinase (chicken); sm-mlck - smooth muscle myosin light chain kinase (chicken); skm-mlck - skeletal muscle myosin light chain kinase (rat); camdk-alph,-beta,-gamm - calcium/calmodulin dependent protein kinase II - α-, β- and γ- subunits, respectively; mlck-dicdi - dictyostelium discoidium (slime mold) myosin light chain kinase.

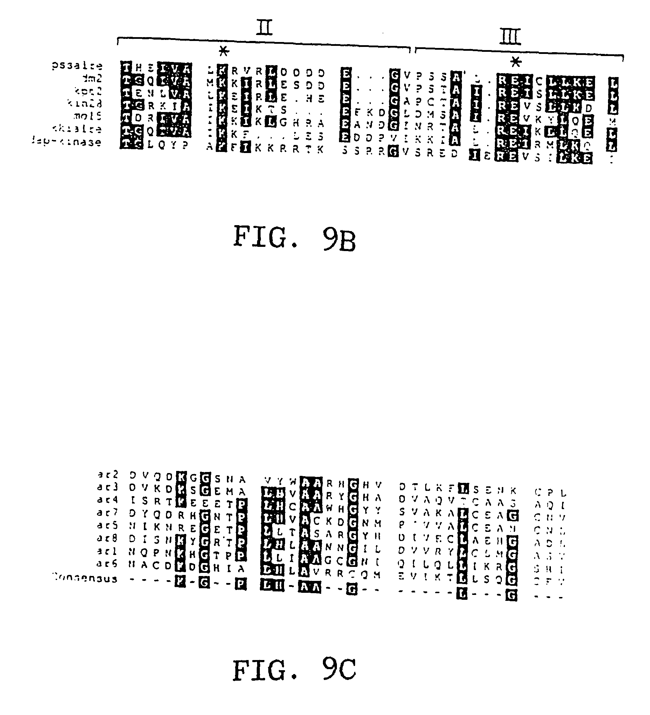

   **Fig. 9(B)** shows alignment of kinase subdomains II and III of DAP-2 and the corresponding domains of different cell cycle dependent kinases. dm2 - Drosophila CDC2 homologue; pssalre - Human serine/threonine kinase PSSALRE; kpt2 - Human serine/threonine protein kinase PCTAIRE-2; kin28 - yeast (*S*.*cerevisiae*) putative protein kinase; mo15 - Xenopus protein kinase related to cdc2 that is a negative regulator of meiotic maturation; kkialre - human serine/threonine protein kinase KKIALRE.
   **Fig. 9(C)** shows alignment of DAP-2 ankyrin repeats. Solid background indicates identical amino acids. A consensus sequence of the DAP-2 ankyrin repeats is shown at the bottom. The position of each individual repeat along the cDNA is illustrated in Fig. 9(B). ar 1-8, ankyrin repeats.

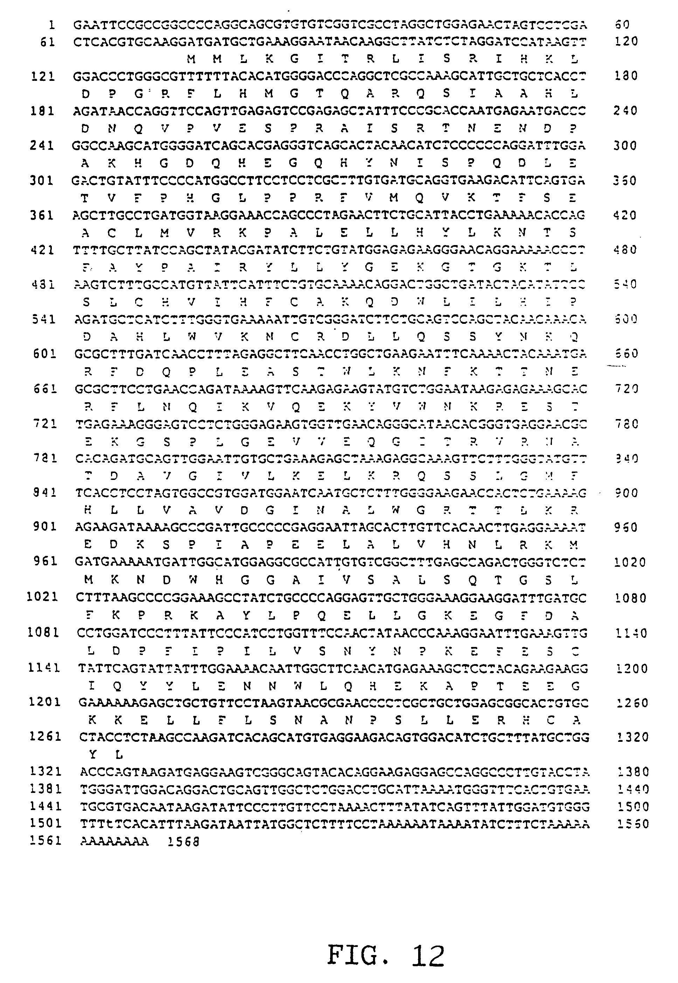

**Fig. 10** shows Northern blot analysis of mRNA obtained from several hematopoietic cells probed with labeled DAP-1 cDNA.
**Fig. 11** shows Northern blot analysis of mRNA obtained from liver, spleen or brain of normal embryos (2) and embryos with Down Syndrome (1) both probed with the labeled cDNA or DAP-1 or DAP-2. In order to evaluate levels of total mRNA, GAPDH was used (bottom).
**Fig. 12** shows the DNA sequence and predicted amino acid sequence of DAP-3.

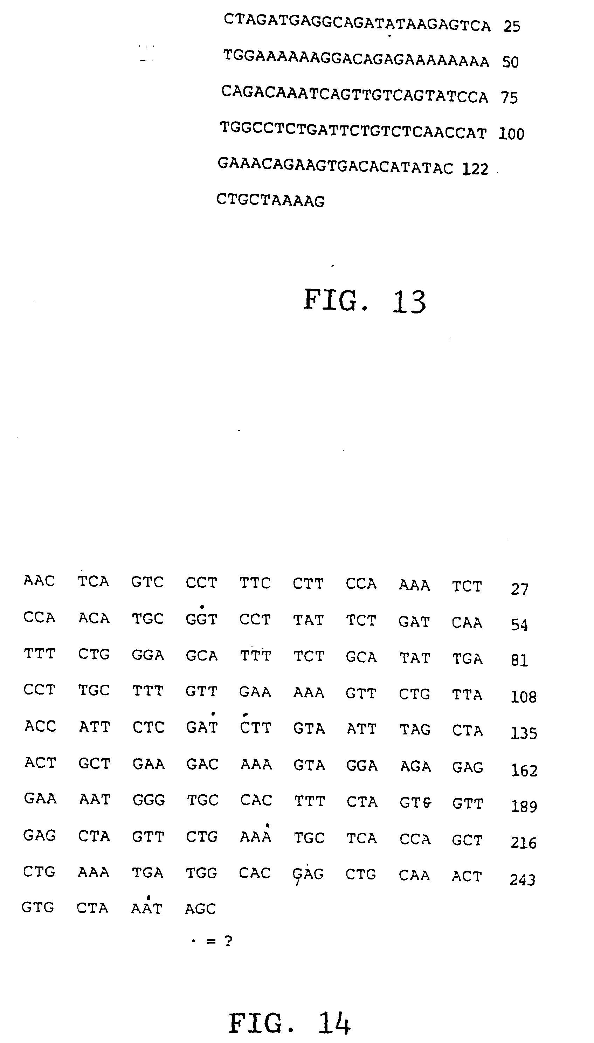

**Fig. 13** shows a partial DNA sequence of DAP-4.
**Fig. 14** shows a partial DNA sequence of DAP-5.

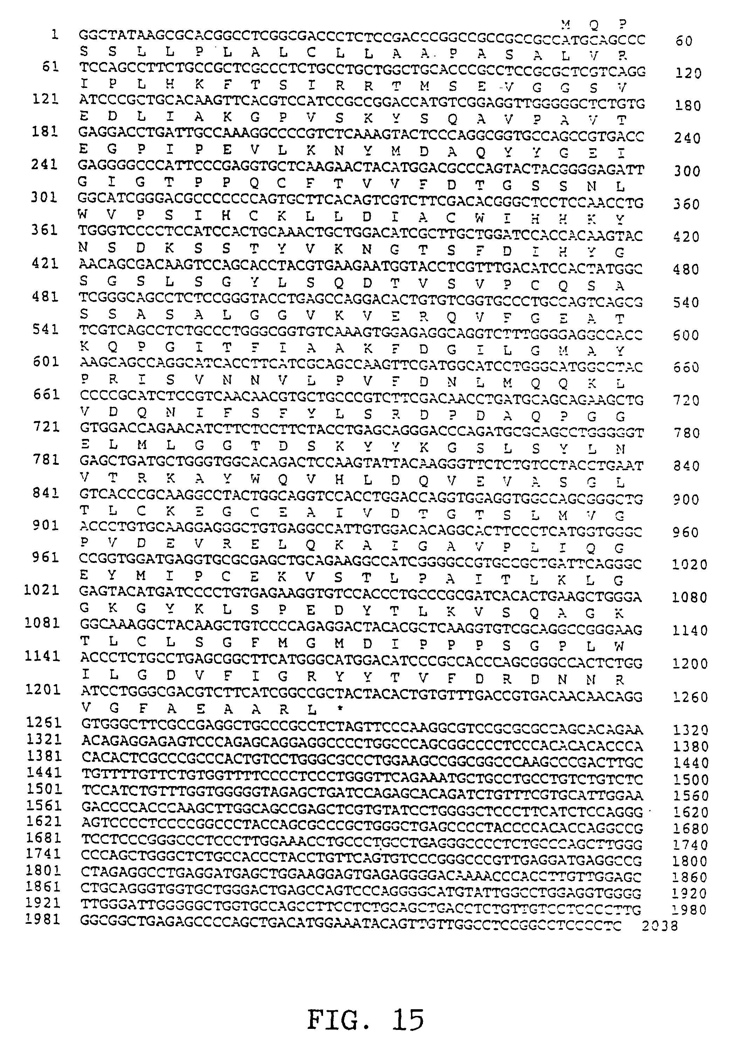

**Fig. 15** shows the DNA sequence and amino acid sequence of cathepsin D.

### DETAILED DESCRIPTION OF THE INVENTION

### I. Isolation of antisense cDNA's that protect cells from the cytotoxic effects of IFN-γ

### (A) Experimental procedure

### (A₁) Obtaining cDNA clones

A cDNA library (100µg DNA) was generated from a mixture of mRNA's harvested before and at 1, 2, 4, 12, 24 and 48 hours after treatment of HeLa cells with IFN-γ (200 U/ml). It was cloned in antisense orientation into the EBV-based pTKO1 expression vector, as previously described in detail (Deiss and Kimchi, *supra*). The resulting expression library of about 10⁵ independent clones was introduced into 8X10⁶ HeLa cells (10⁶ cells per 9 cm plate) by the calcium phosphate transfection technique. In order to determine the efficacy of transfection, a fraction of the transfectants was selected with hygromycin B (200 µg/ml, Calbiochem). The resulted efficacy was around 5%. In parallel, the majority of the transfected cells were plated at a cell density of 1500 cells per cm² and were selected with both hygromycin B (200 µg/ml) and IFN-γ (750 U/ml). Selective media was changed every 3-4 days. After 28 days the cells that survived and/or grew in the presence of IFN-γ were expanded for 2 weeks and pooled. The extrachromosal DNA was obtained according to the method of Hirt (Hirt, B. (1967) *J. Mol*. *Biol*., **26**:365), cleaved with the restriction enzyme DpnI and introduced into *Escherichia coli* HB101 host cells. The cleavage with Dpnl ensured that only episomal DNA that have replicated in HeLa cells was transfected into bacteria.

A few bacterial clones were obtained by the above procedure which included DNA antisense sequences, some of which were able to protect the cells from the death-promoting effects of IFN-γ.

### (A₂) Classification of the antisense cDNA clones

Plasmid DNAs were prepared from 10 individual bacterial clones. PCR amplified cDNA inserts were generated from each plasmid using specific primers that correspond to the immediate flanking sequence of the cDNA insertion sites in the pTKO1 vector. The size of the cDNA inserts ranged between 300 to 800 bp. The PCR fragments were used as labeled probes to search on Southern blots for possible cross hybridization between some of the rescued antisense cDNA clones.

### (B) Results

### (B₁) Classification of Clones

The above 10 cDNA clones were classified into six distinct nonoverlapping groups, some constituting several members (clones) and some constituting of a single member. Those clones relevant for the present invention are shown in the following Table 1:

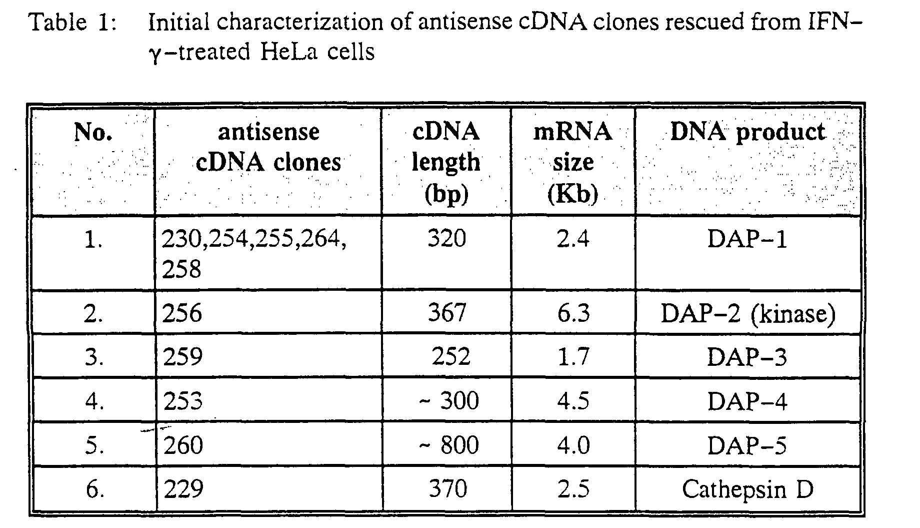

Inserts 230, 254, 255, 264 and 258 of group 1 seemed to be completely identical to one another. The PCR fragments were sequenced and the results were compared with sequences present in the EMBA nucleic acid database. All inserts of groups 1 through 5 were found to be novel.

### (B₂) Detection of mRNA

The DNA fragments thus obtained were used to detect and determine the expression level in HeLa cells of mRNA which hybridized to these fragments. 20 µg of total RNA from the parental HeLa cells were fractionated on gels, blotted and reacted with the different probes. Each probe recognized a single mRNA transcript of a different size (Table 1). Expression levels of mRNA's reactive with group 2 were low while those reactive with group 1 were relatively high.

### II. Second transfection by isolated antisense cDNA

### Levels of expression of antisense RNA in secondary transfectants

### (A) Experimental procedure

To ensure that the above isolated antisense cDNA's are sufficient in order to protect cells from the death promoting effects of IFN-γ, subconfluent monolayers of HeLa cells were transfected with 40 µg DNA of the individual rescued pTKO1 plasmids (in duplicates) and subjected to the single selection of hygromycin B. Pools of approximately 10⁴ hygromycin resistant clones were generated from each transfection and were kept as 6 duplicates of stable polyclonal populations. The sensitivities of the above clones to an application of IFN-γ was then determined.

The vector pTKO1-DHFR (Deiss and Kimchi, *supra*) which carried a non-relevant construct served as control. The control vector was introduced in parallel into HeLa cells and produced two independent polyclonal population of stable transfectants designated DHFR-t1 and t2.

The double stranded cDNA fragments from construct 230 and 256 (from groups 1 and 2, respectively) were used as probes in Northern blot analysis in order to detect mRNA transcripts both in non-transfected and transfected HeLa cells. These two specific cDNA inserts were labelled by commonly used commercial labelling kits. They were subcloned into Bluescript™ vectors (Stratagene, USA) to facilitate both the preparation of the cDNA inserts and the production of single stranded RNA probes therefrom.

### (B) Results

### Constructs 230 (group 1)

As can be seen in Fig. 1A the cDNA insert in this construct hybridized to a single endogenous 2.4 Kb mRNA transcript, both in non-transfected and transfected HeLA cells. In stable transfectants containing the antisense constructs of clones 230 and 255, an additional composite antisense transcript was detected by this 230 probe. It consisted of 320 bases of the original cDNA insert and 800 additional bases of sequences derived from the expression cassette (SV40 early promoter together with sequences till the polyadenylation signal). One of the RNA labeled strands produced by the Bluescript™ vector hybridized exclusively to the endogenous 2.4 Kb mRNA while the complementary strand hybridized only to the 1.1 Kb RNA confirming that the latter is indeed an antisense mRNA (data not shown).

The amount of the antisense RNA in clones 230 and 255 exceeded the sense mRNA levels by 3 to 6 fold (Figs. 1A, 1B). After IFN-γ treatment the level of antisense expression was further elevated due to the presence of IFN-γ-stimulated response element (ISRE) in the pTKO1 vector (Deiss and Kimchi, *supra*), thus leading to 15 fold excess of antisense over sense transcripts (Fig. 1B). The endogenous 2.4 Kb mRNA level was neither modulated by IFN-γ, nor influenced by the high antisense expression.

### Construct 256 (group 2)

As can be seen in Figs. 2A and 2B, the construct of the 256 clone (367 bp in size) hybridized on Northern blots to a single endogenous 6.3 Kb mRNA transcript which was expressed in all tested cells at relatively low levels. In the 256-t1 and t2 transfected cells it also hybridized to a composite 1.2 Kb RNA that consisted of 367 bases of the cDNA insert and 800 bases of sequences derived from the expression cassette in the vector (Fig. 2). The antisense orientation of fragment #256 in the pTKO1 vector was confirmed upon sequencing of the sense cDNA clone (Fig. 7). The amount of the antisense RNA expressed from pTKO-1 plasmid #256 in non-treated HeLa cells exceeded the sense mRNA levels by more than 100 fold. Moreover, due to the presence of IFN-stimulated response element (ISRE) in the pTKO1 vector, the levels of antisense mRNA expression were further elevated after IFN-γ treatment (Fig. 3).

### III. Response of cells transfected with antisense cDNAs to IFN-γ

### (A) Experimental procedure

The HeLa polyclonal population transfected with the individual antisense cDNAs were cultured in the presence of both hygromycin B and IFN-γ (750 U/ml). Growth and viability parameters were examined: (1) under the light microscope, (2) by electron microscopy, and (3) by DAPI staining (0.5 µg/ml; Sigma). For more detailed quantitation, a neutral red uptake assay was performed: the different polyclonal HeLa cell populations were cultivated in 96-well microtiter plates at subconfluent cell densities and then treated with IFN-γ (750 U/ml) or left untreated. All the cells were continuously maintained in a hygromycin B-containing medium to select for transfected cells. The two DHFR-transfected HeLa cell populations (t1, t2), prepared as described above, served as control cultures that display the typical growth sensitivity curves to IFN-γ. The examined antisense cDNA transfected cells were the 230-t1, 255-t1 (group 1) and 256-t1, 256-t2 (group 2). Viable cells were stained with neutral-red and the dye uptake was quantified by measuring O.D. at 540 nm in quadruplicates during the 14 days of the experiment.

### (B) Results

The microscopic examination of parental and control DHFR-transfected HeLa cells revealed that IFN-γ triggered a biphasic pattern of responses. The cells stopped proliferating during the first four days of IFN-γ treatment but still remained viable (in trypan-blue exclusion tests) and displayed a flattened morphology characteristic of the cystostatic responses to IFN-γ (Fig. 3A, b). The reduction in the proliferation rate during this period was also measured by a sharp decline (by more than 90%) in the thymidine uptake into DNA (not shown). This type of IFN-γ-induced proliferation arrest was then followed by massive cell death that occurred in a non-synchronous fashion over a period of an additional 10 days. The cells gradually reduced their size, rounded up and detached from the plates (Figs. 3A, d). Staining of DNA with DAPI after detachment of cells from the substratum revealed gross changes in the nuclear morphology characteristic of programmed cell death. This included nuclear pyknosis, chromatin condensation, sometimes detected preferentially at the nuclear periphery, and chromatin segmentation (Fig. 3B, b). Transmission electron micrographs of the IFN-γ-treated cells prior to their detachment revealed other morphological changes including the disappearance of surface microvilli, surface blebbing, budding off cytoplasmic projections and cytoplasmic disintegration, in addition to the nuclear pyknosis and chromatin condensation (details shown in Fig. 3C, d). The antisense RNA expression from pTKO-1 plasmid of group 1 reduced the susceptibility of the cells to the killing effects of IFN-γ: more cells survived on the plates and the abovementioned death associated morphological changes appeared at much lower frequency (compare the scanning electron micrographs of the IFN-γ-treated DHFR-transfected cells in Fig. 3C, b to the IFN-γ-treated 230-t1 cells in Fig. 3C, f). Similar microscopic observations, showing protection from the IFN-γ-induced cell death, were also made with respect to three other clones from the aforementioned groups of antisense cDNAs, i.e. 2, 3, and 7 (not shown).

A neutral-red uptake assay was then performed to determine more accurately, on a quantitative basis, both the typical biphasic responses of control cultures to IFN-γ and the reduced susceptibility of the antisense expressing cultures to the IFN-γ-induced cell death. The two DHFR-transfected HeLa cell populations (t1, t2) served as the control cultures in this assay and the antisense cDNA transfected cells examined were the 230-t1, 255-t1 (group 1) (Fig. 4A) and 256-t1, 256-t2 (group 2) (Fig. 4B). In the absence of IFN-γ, all the transfected HeLa cells behaved the same and displayed practically identical growth curves suggesting that the antisense RNA expression had no effects on the normal growth of cells. Another feature that was not changed by the antisense RNA expression was the extent of the cytostatic responses to IFN-γ. As shown in Figs. 4A and 4B, IFN-γ has similarly reduced the proliferation rate of all the transfected cultures and they all displayed the same extent of reduction in the neutral-red dye uptake during the first 4 days (before cell death starts to be microscopically evident). After 4 days of treatment the picture changed drastically. While almost all control cells died during the subsequent days of IFN-γ treatment leading to minimal values of the neutral-red dye uptake on day 14, a significant fraction of cells that expressed antisense RNA survived in the presence of IFN-γ, as reflected by the sustained values of the dye uptake. The resistance to the IFN-γ-induced cell killing was very similar in all the four tested cultures that expressed the two different antisense RNAs (Figs. 4A, 4B). These data indicate that expression of antisense RNA from groups 1 and 2 protects the HeLa cells exclusively from the IFN-γ-induced cell death and not from its cytostatic action. It is noteworthy that the antisense RNA expression did not affect the early biochemical steps in the signaling of IFN-γ as deduced from the normal mRNA induction by IFN-γ of the 2-5A synthetase gene in these transfected cells (Fig. 4C). Altogether, it is concluded that among all criteria tested only the death inducing effects of IFN-γ were interrupted by the antisense RNA expression.

### IV. Responses of cells transfected with antisense constructs to necrotic cell death

It became interesting at this stage to check whether the antisense RNA expression can also protect the HeLa cells from a necrotic type of cell death. For this, the effect of TNF-α added in combination with cycloheximide (CHX) was examined in the various HeLa cell populations. Unlike the effect of IFN-γ, the cell death that was induced by TNF-α + CHX in HeLa cells was very rapid (50% killing after 3 hours) and displayed typical features of necrosis such as swelling of the cells before their lysis. As shown in Table 2, while the antisense RNA expression from groups 1 and 2 protected the cells from the IFN-γ-induced cell killing, there was no protection from the TNF-α-induced necrotic cell death. All the examined HeLa cell transfectants were killed by the TNF + CHX combination with similar time kinetics and at the same efficiency. Northern blot analysis demonstrated that the levels of the antisense mRNA transcripts in 256-t1 cells were not reduced by the TNF + CHX treatment at 5 hours (not shown) thus excluding the possibility that loss of the antisense RNA expression, caused by the treatment, may be the reason for lack of protective effects from the necrotic cell death. This further suggests a certain specificity of the protective mechanisms regarding the type of cell killing.

**Table 2:**

| **Expression of antisense RNA (from groups 1 and 2) protects from the IFN-γ-induced programmed cell death but not from the TNF-induced necrotic cell death.** (A=540 nm) | | | | | | |
|---|---|---|---|---|---|---|
| | | DHFR -t1 | DHFR-t2 | 230-t1 | 255-t1 | 256-t1 |
| 14 days | No treatment | 0.396 | 0.345 | 0.385 | 0.324 | 0.336 |
| | IFN-γ | 0.026 | 0.017 | 0.136 | 0.158 | 0.159 |
| 5 hours | No treatment | N.D. | 0.148 | 0.130 | N.D. | 0.140 |
| | TNF-α + CHX | N.D. | 0.053 | 0.026 | N.D. | 0.022 |
| 20 hours | No treatment | 0.211 | 0.248 | 0.223 | 0.173 | 0.190 |
| | TNF-α + CHX | 0.002 | 0.001 | 0.003 | 0.0015 | 0.002 |

Each treatment was done in quadruplicates and the average values of dye uptake, measured by the OD at 1=540 nm, is presented at the indicated time intervals. The SD was between 2-4%. N.D, not done.

### V. Cloning of DAP-1 cDNA and determination of amino acid sequence.

An HL-60 cDNA library constructed in λgt10 vector was screened with the cDNA insert of pTKO1-230. Two independent clones, λ1 and λ2, almost completely overlapping and carrying cDNA inserts of about 2.3 Kb were analysed. λ1 cDNA clone encompasses the 5'-untranslated region, short coding region(s) and a relatively long 3'-untranslated region that constitutes more than 60% of the cDNA clone (Fig. 5).

The nucleotide sequence of the cDNA carried by λ1 and its predicted amino acid pattern are presented in Fig. 6. This cDNA is 2232 bp long and contains a potential polyadenylation signal ATTAAA at its 3' end. The open reading frame (ORF) is very short, starting from the initiation codon at nucleotide positions 160-162 and ending at termination codon TGA at positions 466-468. This ORF is preceded by an extremely GC-rich 5'-untranslated region and potentially codes for a protein consisting of 102 amino acids with calculated MW of 11.2 kDa. The amino acid composition predicts a basic protein (isoelectric point = 10), rich in prolines (15%) which displays two blocks of charged residues, one in the middle and the other at the 3' end of the protein. The high proline content may cause some anomalies in the protein's migration on gels. Search for motifs ("Motifs" program; GCG Software Package) indicated that the protein contains two potential sites for casein kinase II phosphorylation at positions 3 and 36, a single potential protein kinase C phosphorylation site at the C-terminus (position 91) and a consensus phosphorylation site of the cdks at position 51. In addition, the protein contains the consensus sequence RGD at position 65-67, a tripeptide that in some proteins plays a role in cell adhesion, and a potential SH3 binding motif, SPSPP, at position 49-53 (Cowburn (1994) Struc. Biol. 1, 489-491). No indications for the presence of signal peptide or transmembranal domain have been found ( SAPS prediction; Brendel et al., (1992) PNAS USA, 89:2002-2006). The amino acid sequence showed no significant homology to known proteins.

Fragment #230 was used as a probe on Southern blots containing human genomic DNA, digested with various restriction enzymes that do not cut it. A single band was visualized upon hybridization with DNA cleaved with EcoRI, BamHI, PstI and XbaI, suggesting the existence of a single copy gene (not shown). This new gene was termed DAP-1 (Death Associated Protein-1).

*In vitro* translation assays in reticulocyte lysates confirmed that the predicted ORF codes for the major 15kDa protein translated from the cloned 2.4 Kb transcript. The full length cDNA insert as well as four subclones that span different regions of the molecule (i.e., p6, p5, p8, and p4; see Fig. 5) were transcribed and translated in vitro. Among all the tested subclones, only the 5' 1 Kb portion of the DAP-1 cDNA (p6) directed the *in vitro* synthesis of proteins (Fig. 1C). The major translated product migrated on gels as a 15 kDa protein. Mutation at the ATG codon at position 160-162 (ATG to GGC) completely eliminated the synthesis of the 15 kDa protein, thus confirming the position of the start point of this protein (data not shown). In addition to the 15 kDa protein product, a second protein of 22 kDa was also translated at lower efficiency from λ1 and the p6 cDNAs (Fig . 1C). Its translation was not influenced by the elimination of the ATG codon at position 160 but the protein was shortened to a size of 16 and 18 kDa upon cleavage of the p6 subclone with DraI and BstYI restriction endonucleases, respectively (not shown; for restriction map see Fig. 5). These criteria fit another potential open reading frame, which is detected in the nucleotide sequence in a different phase with respect to the first ORF (Fig. 6). It starts at the ATG codon (positions 287-289) and ends at termination codon TGA (positions 816-818). It has the potential to code for a protein consisting of 176 amino acids with a calculated molecular weight of 19.9 kDa, and has no significant homology to any known proteins.

To analyse the expression of the major DAP-1 protein in cells, rabbit polyclonal antibodies were prepared against the bacterially produced 15kDa protein. The affinity purified antibodies recognized on immunoblots two closely migrating proteins in extracts of HeLa cells; the lower band co-migrated on gels with the bacterially produced 15 kDa DAP-1 protein. The slower migrating form may represent a post-translationally modified version of the protein. In the HeLa cell transfectants, 230-t1 and 255-t1, expressing the elevated levels of antisense RNA that develop in the presence of IFN-γ (15 to 1 ratio), the DAP-1 protein levels were reduced by 75% and 78%, respectively, as compared to the DHFR-tranfected cultures (Fig. 1D). The two upper non specific bands (that are not competed with excess of the bacterially produced DAP-1) were not affected by the antisense expression, thus supporting the selectivity of the effect.

### VI. Cloning of DAP-2 and determination of amino acid sequence

As mentioned above, expression studies indicated that the doublestranded cDNA fragment #256 (367 bp in size) hybridized on Northern blots to an endogenous 6.3 Kb mRNA transcript. The same single 6.3Kb mRNA transcript was detected in HeLa (parental and transfectants) and in K562 cells when the full length cDNA (see below) was used as a probe on Northern blots (Fig. 2B). The cDNA insert from pTKO1-256 was therefore used to screen a K562 cDNA library.

Approximately 4x10⁶ pfu were screened with the #256 cDNA insert and 40 positive clones were isolated after two rounds of sequential walking screening. The sequencing was performed on an Applied Bio-systems DNA sequencer 373 A. Sequence uniqueness and relatedness were determined using FASTA (GCG software package) at the nucleotide level and FASTA, BLASTP, and BLOCKS programs at the amino acid level (S. Henikoff and J. G. Henikoff, Nucleic Acids Res. 19, 6565 (1991).

Two clones, λ29 and λ32, were chosen for sequencing (Fig. 7). The resulting composite sequence of both cDNAs consists of 5886 nucleotides and contains a poly A tail that starts at position 5872 and is preceded by two polyadenylation signals AATAAA (Fig. 8). The 3'-untranslated region also contains two ATTTA instability motifs found in the 3'-noncoding portions of short-lived mRNAs (G. Shaw and R. Kamen, Cell 46, 659 (1986)). The mRNA contains a single long open reading frame that starts at position 337, ends at position 4605 and potentially codes for a protein of 1423 amino acids (Fig. 8). The calculated molecular weight of the protein product is about 160 kDa. Affinity purified polyclonal antibodies were raised against the N-terminal 20 amino acid peptide of the protein. These antibodies recognized on immunoblots a 160kDa recombinant protein that was produced in COS-1 cells after transfection with a vector that expressed the entire coding region of the cDNA (Fig. 2D). These antibodies reacted in HeLa cells with an endogenous protein of the same size. In the antisense RNA expressing cells, 256-t1 and 256-t2, the steady state levels of the 160kDa protein were 10 and 5 fold lower than in the DHFR control cells while a non relevant protein, vinculin, displayed similar expression levels in all HeLa cell transfectants (Fig. 2D). Thus, expression of anti-sense RNA from pTKO-1 plasmid #256 in HeLa cells resulted in a significant reduction in the amount of the corresponding protein.

We were able to define several known domains and motifs that are present in this protein. Its extreme N-terminus is composed of a protein kinase domain that spans 255 amino acids from position 13-267. On the basis of its structure, it is likely to be a serine/threonine type of protein kinase having a classical composition of XI subdomains with all conserved motifs present (Fig. 8) (S. K. Hanks and A. M. Quinn, Methods Enzymol. 200, 38 (1991)). This novel kinase was termed DAP-2 or DAP-kinase (Death Associated Protein-kinase).

The kinase domain falls into a family of that of calmodulin-dependent kinases. The homology to known kinase domains that constitute this group, including the myosin light chain kinases, ranges between 34%-49% ( Fig. 9A). Three main differences distinguish the kinase domain of DAP-kinase from other members of calmodulin-dependent kinase family: 1) Subdomain II is relatively long and has a stretch of basic amino acids (KKRRTKSSRR); 2) Subdomain III mostly resembles that of the cell cycle dependent kinases (Fig. 9B). Interestingly, the typical sequences of the cell cycle dependent kinases ( PSTAIRE, PSSALRE, PCTAIRE, KKIALRE) are located in subdomain III; and 3) Subdomain VII is extremely short and consists of only 7 amino acids.

Right downstream to the kinase domain there is an additional stretch of homology that is present in almost all members of the family of calmodulin-dependent kinases, and was implicated in calmodulin-recognition and binding; B. P. Herring, J. T. Stull, P. J. Gallagher, J. Biol. Chem. 265, 1724 (1990); M. O. Shoemaker *et al*., J.Cell. Biol. 111, 1107 (1990); F.H. Cruzalegui *et al*., Proc. Nath. Acad. Sci. USA 89, 12127 (1992)). Downstream of the calmodulin-recognition domain, an ankyrin repeats domain was identified spanning 265 amino acids from position 365 to 629. It is composed of 8 repeats of 33 amino acids each, not separated by spacers except for a single proline residue that separates three N-terminal repeats from five C-terminal ones (Figs. 8 and 9C). Ankyrin repeats are involved in protein-protein interactions in a variety of proteins (P. Michaely and V. Bennett, Trends in Cell Biology 2, 127 (1992)), but were not described before in the context of serine/threonine kinases. One tyrosine kinase carrying ankyrin repeats has been recently identified in Hydra vulgaris (T.A. Chan *et al*., Oncogene 9, 1253 (1994)). In the DAP-kinase, the 8 ankyrin repeats may mediate the interaction with a putative effector or a regulatory molecule, or influence the substrate selectivity and/or stability of the kinase-substrate interactions.

Immediately downstream to ankyrin repeats there are two subsequent potential P-loop motifs, ALTTDGKT and GHSGSGKT, identified through the consensus sequence, G[A]XXXXGKT[S]. Comparison of DAP-kinase potential P-loop motifs to the corresponding consensus sequences within seven ATP or GTP-binding protein families demonstrates that only the 3' P-loop has some similarity to P-loop consensus of elongation factors, ATP synthase b-subunits and thymidine kinase. Actually, a stretch of 33 amino acids following the eighth ankyrin repeat that encompasses the putative 5' P-loop, may represent a ninth ankyrin repeat that is less conserved than others. DAP-kinase also carries multiple potential sites for post-translational modifications, and has neither transmembranal domain nor signal peptide. The Prosite bank search, using the program Motifs (GCG Software Package) revealed that the DAP-kinase protein contains a consensus sequence for the C-terminal amidation site at position 1376 (this suggests that 47 C-terminal amino acids can be cleaved from the protein body ). It also contains consensus sequences for six N-glycosylation sites, and potential phosphorylation sites for CAMP-dependent kinase (six), casein kinase II (twenty eight) and protein kinase C (twenty).

Altogether, the deduced amino acid sequence of the DAP-kinase suggests that a very unique type of calmodulin-regulated serine/threonine kinase has been rescued. The combination of serine/threonine kinase domain, ankyrin repeats and additional possible ATP/GTP binding sites outside the kinase domain in one protein (Fig. 10) has not been previously described. A size of 160 kDa is rare among serine/threonine kinases and DAP-kinase is actually the largest calmodulin-dependent kinase known to date. The ability of DAP-kinase to bind calmodulin, recently confirmed in yeast two hybrid system (not shown), is consistent with the notion that in many cases programmed cell death is Ca²⁺ dependent (S. Sen, Biol. Rev. Camb. Philos. Soc. 67, 287 (1992); S. Lee, S. Christakos, M. B. Small, Curr. Opin. Cell. Biol. 5, 286 (1993)). Moreover, it has been recently reported that calmodulin antagonists inhibited the glucocorticoid-induced apoptosis (D. R. Dowd, D. P. Mac, B. S. Komm, M. R. Haussler, R. Miesfeld, J. Biol. Chem. 266, 18423 (1991)), and that inhibitors of myosin light chain kinases blocked the TNF-induced apoptotic cell death (S.C. Wright, H. Zheng, J. Zhong, F.M.Torti, J.W. Larrick, J. Cell. Biochem. 53, 222 (1993)).

In order to verify that DAP-2 is truely a kinase, COS cells were transiently transfected with an expression vector (PECE-FLAG) that carries a fragment of the 129 cDNA that encompasses the entire coding region (from the abovementioned start ATG to the first EcoRI site at the 3' end). Cell lysates were immunoprecipitated by anti-FLAG monoclonal antibodies and washed immunoprecipitates were assayed for *in-vitro* autophosphorylation in the presence of calmodulin and Ca²⁺. As shown in Fig. 2C, a single phosphorylated band of 160 kDa appeared upon fractionation of the *in-vitro* reaction products on polyacrylamide gels. This experiment provides the first direct proof that the recombinant protein has intrinsic kinase activity, as suggested by the predicted amino acid structure.

### Vll. Expression of DAP-1 and DAP-2 proteins in various cells and tissues

Examination of a variety of cell lines and tissues revealed that these two genes are likely to be ubiquitously expressed. Fig. 10 shows the Northern blot analysis of RNA from different hematopoietic cells probed with the DAP-1 cDNA. The 2.4 Kb mRNA transcript of this gene was detected in granulocytes (HL-60) B lymphoid (Daudi) and macrophage (U937) cells. The expression levels in the hematopoietic cells was lower than in HeLa cells. Fig. 11 shows results of examination of the mRNA expression in human embryonic tissues: brain, spleen (predominantly B cells) and liver (predominantly erythrocytes). Again the single 2.4 Kb mRNA transcript was detected in these tissues by the DAP-1 cDNA probe.

The DAP-2 cDNA probe 2 recognized the 6.3 Kb mRNA encoded by this gene in these different tissues (Fig. 11). The embryonal liver and spleen tissues from Down syndrome seemed in this blot to express higher levels of the DAP-2 gene (compared to the GAPDH levels) while the brain tissue from Down syndrome contained higher levels of DAP-1 mRNA than the corresponding normal brain.

### VIII. Cloning and sequencing of DAP-3, DAP-4 and DAP-5

Clone 259 (DAP-3) was sequenced and used to screen a K562 λgt10 cDNA library as described above for DAP-1 and DAP-2. The sequence of the (almost) full length cDNA of DAP-3 and the deduced amino acid sequence is shown in Fig. 12.

Clones 253 (DAP-4) and 260 (DAP-5) were partially sequenced as described above for DAP-1 and DAP-2, and the results are shown in Figs. 13 and 14, respectively.

### IX. Identification of DAP-7

The initial microscopic observations, performed on the different HeLa cells that had been transfected with the individual rescued pTKO1 clones (described in Table 1), indicated that plasmid pKTO1-229 (group 7) conveyed similar effects to those conferred by the plasmids from group 1. It reduced the susceptibility of the cells to the IFN-γ-induced cell death but not to its cytostatic effects.

The cDNA carried by plasmid pTKO1 - 229 was identified upon sequencing as a BamHl-Hindlll fragment of human cathepsin D cDNA, which was present in the expression vector in the antisense orientation. The DNA probe, corresponding to fragment #229, hybridized as expected to a single endogenous 2.5 Kb mRNA, both in control and in the transfected HeLa cells. The steady state levels of cathepsin D sense mRNA were not affected by the IFN-γ treatment. In the pTKO1-229 transfected cells the DNA probe also hybridized to the composite antisense RNA. The levels of antisense cathepsin D RNA were stimulated 5-fold in response to IFN-γ due to the presence of an ISRE enhancer element in the pTKO1 expression vector (not shown).

The cathepsin D protein was identified on immunoblots using commercially available polyclonal antibodies (Oncogene Science). It was found that in the control clones, IFN-γ prevented the appearence of the mature 34kDa chain while the 48kDa active single chain precursor was retained at abnormal high levels in these cells. It appears that this single chain precursor is the specific cathepsin form that functions during cell death.

Cathepsin D is an aspartic protease that is found normally in lysosomes where it functions in protein catabolism. Yet, in some pathological situations it has been suggested that this protease can function in the cytosol, and its activity was associated with degenerative brain changes, muscular dystrophy and connective tissue disease pathology ( Matus and Green (1987); Biochemistry, 26, 8083-8036). The present invention shows for the first time that the expression of this protease is indispensable for the execution of programmed cell death that is induced by IFN-γ. Thus, cathepsin D joins the growing list of proteases that play a key role in different scenarios of programmed cell death.

The DNA sequence and amino acid sequence of cathepsin D are shown in Fig. 15 (Faust, P.L. *et al*. (1985) PNAS USA 82, 4910-4914).

## Claims

1. An isolated DNA molecule comprising a nucleic acid sequence expressed in cells, the expression product of which is involved in cytokine-induced programmed cell death, said sequence being a coding sequence beginning at the nucleic acid triplet at position 337-339 and ending at the triplet at position 4603-4605 of the sequence depicted in Fig. 8.

2. An isolated DNA molecule comprising a nucleic acid sequence expressed in cells, the expression product of which is involved in cytokine-induced programmed cell death, said sequence being a coding sequence encoding the same expression product involved in cytokine-induced programmed cell death as that encoded by the coding sequence as defined in Claim 1.

3. An isolated DNA molecule, **characterized in that** it comprises a modified coding sequence of the DNA molecules according to either of claims 1 or 2,in which either
a) one or more nucleic acid triplets, which encode amino acids which are not essential for cytokine-induced programmed cell death activity, have been deleted or replaced; or,
b) one or more nucleic acid triplets encoding amino acids have been added in a region which is not essential for cytokine-induced programmed cell death activity;
the expression product encoded by said modified coding sequence having the same cytokine-induced programmed cell death activity as that of the expression product encoded by any one of the coding sequences as defined in either of Claims 1 or 2, respectively.

4. A fragment of any one of the isolated DNA molecules of Claims 1-3, said fragment encoding a protein or polypeptide retaining cytokine-induced programmed cell death activity present in the protein or polypeptide encoded by any one of the coding sequences of either of Claims 1 or 2 with the proviso that nucleotide sequence 2556-2814 of the sequence of figure 8 is excluded.

5. A molecule which comprises an antisense sequence complementary in sequence to the mRNA transcribed from the entire or part of any one of the DNA molecules according to claim 1, wherein said antisense sequence is capable of inhibiting the expression of the coding sequence as defined in Claim 1.

6. A modified DNA sequence of Claim 1 in which one or more nucleic acid triplets have been added, deleted or replaced, the protein or polypeptide encoded by the modified sequence having dominant negative effect, being capable of inhibiting the function of the protein or polypeptide involved in cytokine-induced programmed cell death encoded by the coding sequence of Claim 1.

7. A vector comprising a DNA sequence as defined in Claim 1 and sequences required for propagating and replicating the DNA sequence in a host cell.

8. A vector according to Claim 7, being an expression vector comprising also sequences required for transcription of said DNA into an mRNA.

9. A vector according to Claim 8, being an episomal plasmid constructed by using vectors pTKO1, pTKO2 and pTKO3.

10. A protein or polypeptide comprising an amino acid sequence encoded by a DNA molecule as defined in any of Claims 1-4.

11. A protein or polypeptide according to Claim 10, wherein one or more amino acids have been chemically modified.

12. A protein according to Claim 10, having the amino acid sequence as depicted in Fig. 8.

13. An antibody raised against a protein according to any one of Claims 10-12 or against an immunogenic portion thereof.

14. A pharmaceutical composition for promoting or preventing death of target cells, comprising a pharmaceutically acceptable carrier and an active agent being an expression vector which comprises a DNA sequence being under expression control allowing the expression in target cells, said DNA sequence being selected from the group consisting of:
a) a coding sequence beginning at the nucleic acid triplet at position 337-339 and ending at the triplet at position 4603-4605 of the sequence depicted in Fig. 8;
b) a DNA sequence other than the DNA defined under (a) which encodes the same expression product encoded by the DNA sequence defined in (a);
c) a modified DNA sequence of (a) or (b) in which, either; one or more nucleic acid triplets encoding amino acids which are not essential for cytokine-induced programmed cell death activity, have been deleted or replaced; or, one or more nucleic acid triplets encoding amino acids have been added in a region which is not essential for cytokine-induced programmed cell death activity; the protein or polypeptide encoded by the modified DNA sequence having the same cytokine-induced programmed cell death activity as that encoded by any one of the DNA molecules of either of (a) or (b), respectively; or,
d) fragments of any of the DNA sequences of (a), (b) or (c), encoding a protein or a polypeptide having the same cytokine-induced programmed cell death activity as that of the expression product encoded by any one of the DNA molecules of either (a) or (b).

15. A pharmaceutical composition for the treatment of cancer, comprising a pharmaceutically acceptable carrier and an active agent, being an expression vector comprising a DNA sequence as defined in Claim 14.

16. A pharmaceutical composition for the treatment of cancer, comprising a pharmaceutically acceptable carrier and an active ingredient, being a protein or polypeptide encoded by any of the DNA sequences as defined in Claim 14.

17. A pharmaceutical composition according to Claim 14, further comprising a cytokine.

18. A pharmaceutical composition for use in inhibiting cell destruction caused by cytokine-induced programmed cell death, comprising a pharmaceutically acceptable carrier and an active agent being:
a) a DNA sequence which is an antisense to the entire or part of the DNA sequence comprising a coding sequence beginning at nucleic acid triplet at position 337-339 and ending at the triplet at position 4603-4605 of the sequence depicted in Fig. 8; wherein said antisense is capable. of inhibiting the expression of said coding sequence beginning at nucleic acid triplet at position 337-339 and ending at the triplet at position 4603-4605 of the sequence depicted in Fig. 8; or,
b) an antibody raised against any of the proteins or polypeptides encoded by the coding sequences as defined in Claims 1 or 2;

19. An *in vitro* method for the screening for a predisposition to cancer in a sample of either genomic DNA from cells of an individual or cDNA produced from mRNA of said cells; said method comprising:
a) adding one or more DNA probes, said probes comprising a DNA sequence selected from the group consisting of:
i. a DNA sequence encoding an expression product which is involved in cytokine-induced programmed cell death, and comprising a coding sequence beginning at the nucleic acid triplet at position 337-339 and ending at the triplet at position 4603-4605 of the sequence depicted in Fig. 8;
ii. a DNA sequence encoding an expression product which is involved in cytokine-induced programmed cell death, other than the DNA defined under (i), which encodes the same expression product encoded by the coding sequence defined in (i);
iii. a modified DNA sequence of (i) or (ii) in which either; one or more nucleic acid triplets, which encode amino acid which are not essential for cytokine-induced programmed cell death activity, have been deleted or replaced; or, one or more nucleic acid triplets, encoding amino acids, have been added in a region which is not essential for cytokine-induced programmed cell death activity; the protein or polypeptide encoded by the modified DNA sequence having the same cytokine-induced programmed cell death activity as that of the expression product encoded by any one of the DNA molecules of either of (i) or (ii), respectively;
iv. fragments of any of the DNA sequences of (i), (ii) or (iii), encoding a protein or a polypeptide having the same cytokine-induced programmed cell death activity as that of the expression product encoded by any one of the DNA molecules of either of (i) or (ii);
v. a sequence which is an antisense to the entire or part of the DNA molecule as defined in (i), (ii), (iii) or (iv), the expression product of which is involved in cytokine-induced programmed cell death, said antisense being capable of inhibiting the expression of said DNA molecule encoding the expression product;
vi. a modified DNA sequence of a DNA molecule as defined in (i) in which one or more nucleic acids have been added, deleted or replaced, the protein or polypeptide encoded by the modified sequence having dominant negative effect manifested by the ability of said protein or polypeptide to inhibit said cytokine-induced programmed cell death; and,
b) providing conditions for hybridization between the DNA probe or probes and the DNA of said sample;
c) determining on the basis of the hybridization whether DAP-2 gene is absent, whether there is a match between the sequence of the DNA probe or probes and a sequence in the DNA of said sample or whether there is a mismatch, a mismatch or absence indicating a deletion or a mutation in the genomic DNA and a predisposition to cancer of the tested individual.

20. An *in vitro* method for detecting a mutation in a DAP-2 gene encoding a product involved in cytokine-induced programmed cell death comprising probing a sample constituted by genomic DNA or cDNA from an individual with one or more DNA probes comprising a complete or partial sequence of a DNA sequence comprising a coding sequence beginning at the nucleic acid triplet at position 337-339 and ending at the triplet 4603-4605 of the sequence depicted in Fig. 8.

21. A use of an expression vector according to Claim 8 in the preparation of a pharmaceutical composition for the treatment of cancer.

22. A use of an expression vector comprising a DNA sequence selected from the group consisting of:
a) an antisense sequence as defined in Claim 5;
b) a modified DNA sequence, as defined in Claim 6;
in the preparation of a pharmaceutical composition which inhibits cell destruction caused by cytokine-induced programmed cell death.

23. A use, as an active agent of an antibody directed against any of the proteins or polypeptides encoded by the DNA sequences defined in Claim 1 or 2, in the preparation of a pharmaceutical composition which inhibits cell destruction caused by cytokine-induced programmed cell death.

## Patentansprüche

1. Isoliertes DNA-Molekül, umfassend eine in Zellen exprimierte Nukleinsäuresequenz, deren Expressionsprodukt an dem Zytokin-induzierten programmierten Zelltod beteiligt ist, wobei die Sequenz eine kodierende Sequenz ist, die an dem Nukleinsäuretriplett an der Position 337-339 der in Fig. 8 dargestellten Sequenz beginnt und an dem Triplett an der Position 4603-4605 endet.

2. Isoliertes DNA-Molekül, umfassend eine in Zellen exprimierte Nukleinsäuresequenz, deren Expressionsprodukt an dem Zytokin-induzierten programmierten Zelltod beteiligt ist, wobei die Sequenz eine kodierende Sequenz ist, die das gleiche, an dem Zytokin-induzierten programmierten Zelltod beteiligte Expressionsprodukt kodiert wie dasjenige, das von der, wie in Anspruch 1 definierten, kodierenden Sequenz kodiert wird.

3. Isoliertes DNA-Molekül, **dadurch gekennzeichnet, dass** es eine modifizierte kodierende Sequenz der DNA-Moleküle nach einem der Ansprüche 1 oder 2 umfasst, in der entweder
a) eines oder mehrere Nukleinsäuretripletts, die Aminosäuren kodieren, die nicht essentiell für die Zytokin-induzierte programmierter-Zelltod-Aktivität sind, deletiert oder ersetzt wurden; oder
b) eines oder mehrere, für Aminosäuren kodierende Nukleinsäuretripletts in einer Region, die nicht essentiell für die Zytokin-induzierte programmierter-Zelltod-Aktivität ist, hinzugefügt wurden;
wobei das von der modifizierten kodierenden Sequenz kodierte Expressionsprodukt die gleiche Zytokin-induzierte programmierter-Zelltod-Aktivität aufweist wie diejenige des Expressionsprodukts, das von einer beliebigen der, wie in einem der Ansprüche 1 bzw. 2 definierten, kodierten Sequenzen kodiert ist.

4. Fragment eines beliebigen der isolierten DNA-Moleküle der Ansprüche 1-3, wobei das Fragment ein Protein oder Polypeptid kodiert, das eine Zytokin-induzierte programmierter-Zelltod-Aktivität bewahrt, die in dem von einer beliebigen der kodierenden Sequenzen nach einem der Ansprüche 1 oder 2 kodierten Protein oder Polypeptid vorhanden ist, mit der Maßgabe, dass die Nukleotidsequenz 2556-2814 der Sequenz von Figur 8 ausgeschlossen ist.

5. Molekül, das eine Antisensesequenz umfasst, deren Sequenz komplementär zu der von dem gesamten oder einem Teil eines beliebigen der DNA-Moleküle nach Anspruch 1 transkribierten mRNA ist, wobei die Antisensesequenz fähig ist, die Expression der, wie in Anspruch 1 definierten, kodierenden Sequenz zu inhibieren.

6. Modifizierte DNA-Sequenz nach Anspruch 1, in der eines oder mehrere Nukleinsäuretripletts hinzugefügt, deletiert oder ersetzt wurden, wobei das von der modifizierten Sequenz kodierte Protein oder Polypeptid einen dominant negativen Effekt hat und fähig ist, die Funktion des an dem Zytokin-induzierten programmierten Zelltod beteiligten, von der kodierenden Sequenz von Anspruch 1 kodierten Proteins oder Polypeptids zu inhibieren.

7. Vektor, umfassend eine wie in Anspruch 1 definierte DNA-Sequenz und Sequenzen, die für die Vermehrung und Replikation der DNA-Sequenz in einer Wirtszelle erforderlich sind.

8. Vektor nach Anspruch 7, der ein Expressionsvektor ist, der auch Sequenzen umfasst, die für die Transkription der DNA in eine mRNA erforderlich sind.

9. Vektor nach Anspruch 8, der ein unter der Verwendung der Vektoren pTKO1, pTKO2 und pTKO3 konstruiertes, episomales Plasmid ist.

10. Protein oder Polypetid, umfassend eine von einem, wie in einem beliebigen der Ansprüche 1-4 definierten, DNA-Molekül kodierte Aminosäuresequenz.

11. Protein oder Polypeptid nach Anspruch 10, worin eine oder mehrere Aminosäuren chemisch modifiziert wurden.

12. Protein nach Anspruch 10, das die wie in Fig. 8 dargestellte Aminosäuresequenz aufweist.

13. Antikörper, der gegen ein Protein nach einem beliebigen der Ansprüche 10-12 oder gegen einen immunogenen Teil davon erzeugt wurde.

14. Pharmazeutische Zusammensetzung für das Fördern oder Verhindern des Tods von Zielzellen, umfassend einen pharmazeutisch akzeptablen Träger und einen aktiven Wirkstoff, der ein Expressionsvektor ist, der eine DNA-Sequenz umfasst, die sich unter einer Expressionskontrolle befindet, die die Expression in Zielzellen erlaubt, wobei die DNA-Sequenz ausgewählt ist aus der Gruppe, bestehend aus:
a) einer kodierenden Sequenz, die an dem Nukleinsäuretriplett an der Position 337-339 der in Fig. 8 dargestellten Sequenz beginnt und an dem Triplett an der Position 4603-4605 endet;
b) einer anderen DNA-Sequenz als der unter (a) definierten DNA, die das gleiche Expressionsprodukt kodiert wie dasjenige, das von der in (a) definierten DNA-Sequenz kodiert ist;
c) einer modifizierten DNA-Sequenz nach (a) oder (b), in der entweder: ein oder mehrere Nukleinsäuretripletts, die für Aminosäuren kodieren, die für die Zytokin-induzierte programmierter-Zelltod-Aktivität nicht essentiell sind, deletiert oder ersetzt wurden; oder ein oder mehrere Nukleinsäuretripletts, die Aminosäuren kodieren, in einer Region hinzugefügt wurden, die für die Zytokin-induzierte programmierter-Zelltod-Aktivität nicht essentiell ist; wobei das von der modifizierten DNA-Sequenz kodierte Protein oder Polypetid die gleiche Zytokin-induzierte programmierter-Zelltod-Aktivität aufweist wie dasjenige, das von einem beliebigen der DNA-Moleküle von einem von (a) bzw. (b) kodiert ist; oder
d) Fragmenten einer beliebigen der DNA-Sequenzen von (a), (b) oder (c), die ein Protein oder Polypeptid kodieren, das die gleiche Zytokin-induzierte programmierter-Zelltod-Aktivität aufweist wie diejenige des Expressionsprodukts, das von einem beliebigen der DNA-Moleküle von entweder (a) oder (b) kodiert ist.

15. Pharmazeutische Zusammensetzung für die Behandlung von Krebs, umfassend einen pharmazeutisch akzeptablen Träger und einen aktiven Wirkstoff, der ein Expressionsvektor ist, der eine wie in Anspruch 14 definierte DNA-Sequenz umfasst.

16. Pharmazeutische Zusammensetzung für die Behandlung von Krebs, umfassend einen pharmazeutisch akzeptablen Träger und einen aktiven Bestandteil, der ein von einer beliebigen der, wie in Anspruch 14 definierten, DNA-Sequenzen kodiertes Protein oder Polypeptid ist.

17. Pharmazeutische Zusammensetzung nach Anspruch 14, weiter umfassend ein Zytokin.

18. Pharmazeutische Zusammensetzung zur Verwendung bei der Inhibierung der durch Zytokin-induzierten programmierten Zelltod verursachten Zellzerstörung, umfassend einen pharmazeutisch akzeptablen Träger und einen aktiven Wirkstoff, der ist:
a) eine DNA-Sequenz, die eine Antisense zu der gesamten oder einem Teil der DNA-Sequenz ist, die eine kodierende Sequenz umfasst, die an dem Nukleinsäuretriplett an der Position 337-339 der in Fig. 8 dargestellten Sequenz beginnt und an dem Triplett an der Position 4603-4605 endet; wobei die Antisense fähig ist, die Expression der kodierenden Sequenz zu inhibieren, die an dem Nukleinsäuretriplett an der Position 337-339 der in Fig. 8 dargestellten Sequenz beginnt und an dem Triplett an der Position 4603-4605 endet; oder
b) ein Antikörper, der gegen ein beliebiges der Proteine oder Polypeptide erzeugt wurde, die von den, wie in den Ansprüchen 1 oder 2 definierten, kodierenden Sequenzen kodiert sind.

19. *In-vitro*-Verfahren für das Screenen auf eine Veranlagung für Krebs in einer Probe von entweder genomischer DNA aus Zellen eines Individuums oder cDNA, die aus mRNA der Zellen hergestellt ist, wobei das Verfahren umfasst:
a) Zugeben einer oder mehrerer DNA-Sonden, wobei die Sonden eine DNA-Sequenz umfassen, die ausgewählt ist aus der Gruppe, bestehend aus:
i. einer DNA-Sequenz, die ein Expressionsprodukt kodiert, das an dem Zytokin-induzierten programmierten Zelltod beteiligt ist, und eine kodierende Sequenz umfasst, die an dem Nukleinsäuretriplett an der Position 337-339 der in Fig. 8 dargestellten Sequenz beginnt und an dem Triplett an der Position 4603-4605 endet;
ii. einer DNA-Sequenz, die ein Expressionsprodukt kodiert, das an dem Zytokin-induzierten programmierten Zelltod beteiligt ist, die eine andere als die unter (i) definierte DNA ist, die das gleiche Expressionsprodukt kodiert wie dasjenige, das von der in (i) definierten, kodierenden Sequenz kodiert ist;
iii. einer modifizierte DNA-Sequenz von (i) oder (ii), in der entweder: ein oder mehrere Nukleinsäuretripletts, die für Aminosäuren kodieren, die für die Zytokin-induzierte programmierter-Zelltod-Aktivität nicht essentiell sind, deletiert oder ersetzt wurden; oder ein oder mehrere Nukleinsäuretripletts, die Aminosäuren kodieren, in einer Region hinzugefügt wurden, die für die Zytokin-induzierte programmierter-Zelltod-Aktivität nicht essentiell ist; wobei das von der modifizierten DNA-Sequenz kodierte Protein oder Polypetid die gleiche Zytokin-induzierte programmierter-Zelltod-Aktivität aufweist wie diejenige des Expressionsprodukts, das von einem beliebigen der DNA-Moleküle von einem von (i) bzw. (ii) kodiert ist;
iv. Fragmenten einer beliebigen der DNA-Sequenzen von (i), (ii) oder (iii), die ein Protein oder Polypeptid kodieren, das die gleiche Zytokin-induzierte programmierter-Zelltod-Aktivität aufweist wie diejenige des Expressionsprodukts, das von einem beliebigen der DNA-Moleküle von entweder (i) oder (ii) kodiert ist;
v. einer Sequenz, die eine Antisense zu dem gesamten oder einem Teil des wie in (i), (ii), (iii) oder (iv) definierten DNA-Moleküls ist, dessen Expressionsprodukt an dem Zytokin-induzierten programmierten Zelltod beteiligt ist, wobei die Antisense fähig ist, die Expression des DNA-Moleküls, das das Expressionsprodukt kodiert, zu inhibieren;
vi. einer modifizierten DNA-Sequenz eines wie in (i) definierten DNA-Moleküls, worin eine oder mehrere Nukleinsäuren zugegeben, deletiert oder ersetzt wurden, wobei das von der modifizierten Sequenz kodierte Protein oder Polypeptid einen dominant negativen Effekt hat, der sich durch die Fähigkeit des Proteins oder Polypeptids, den Zytokin-induzierten programmierten Zelltod zu inhibieren, manifestiert; und
b) Bereitstellen von Bedingungen für die Hybridisierung zwischen der DNA-Sonde oder -Sonden und der DNA der Probe;
c) Bestimmen auf der Basis der Hybridisierung, ob das DAP-2-Gen abwesend ist, ob es eine Übereinstimmung zwischen der Sequenz der DNA-Sonde oder -Sonden und einer Sequenz der DNA der Probe gibt oder ob es eine Fehlpaarung gibt, wobei eine Fehlpaarung oder Abwesenheit eine Deletion oder eine Mutation in der genomischen DNA und eine Veranlagung des getesteten Individuums für Krebs anzeigt.

20. *In-vitro*-Verfahren zum Nachweis einer Mutation in einem DAP-2-Gen, das ein an dem Zytokin-induzierten programmierten Zelltod beteiligtes Produkt kodiert, umfassend Untersuchen einer aus genomischer DNA oder cDNA aus einem Individuum bestehenden Probe mit einer oder mehreren DNA-Sonden, die eine gesamte oder teilweise Sequenz einer DNA-Sequenz umfassen, die eine kodierende Sequenz umfasst, die an dem Nukleinsäuretriplett an der Position 337-339 der in Fig. 8 dargestellten Sequenz beginnt und an dem Triplett 4603-4605 endet.

21. Verwendung eines Expressionsvektors nach Anspruch 8 bei der Herstellung einer pharmazeutischen Zusammensetzung für die Behandlung von Krebs.

22. Verwendung eines Expressionsvektors, der eine DNA-Sequenz umfasst, die ausgewählt ist aus der Gruppe, bestehend aus:
a) einer wie in Anspruch 5 definierten Antisensesequenz;
b) einer wie in Anspruch 6 definierten, modifizierten DNA-Sequenz;
bei der Herstellung einer pharmazeutischen Zusammensetzung, die die von dem Zytokin-induzierten programmierten Zelltod verursachte Zellzerstörung inhibiert.

23. Verwendung eines Antikörpers, der gegen ein beliebiges Protein oder Polypeptid gerichtet ist, das von den in Anspruch 1 oder 2 definierten DNA-Sequenzen kodiert ist, als aktiver Wirkstoff bei der Herstellung einer pharmazeutischen Zusammensetzung, die die von dem Zytokin-induzierten programmierten Zelltod verursachte Zellzerstörung inhibiert.

## Revendications

1. Molécule d'ADN isolée comprenant une séquence d'acide nucléique exprimée dans des cellules, dont le produit d'expression est impliqué dans la mort cellulaire programmée induite par les cytokines, ladite séquence étant une séquence codante débutant au triplet d'acide nucléique à la position 337-339 et se terminant au triplet à la position 4603-4605 de la séquence représentée sur la figure 8.

2. Molécule d'ADN isolée comprenant une séquence d'acide nucléique exprimée dans des cellules, dont le produit d'expression est impliqué dans la mort cellulaire programmée induite par les cytokines, ladite séquence étant une séquence codante codant pour le même produit d'expression impliqué dans la mort cellulaire programmée induite par les cytokines que celui codé par la séquence codante telle que définie dans la revendication 1.

3. Molécule d'ADN isolée, **caractérisée en ce qu'**elle comprend une séquence codante modifiée des molécules d'ADN selon l'une ou l'autre des revendications 1 et 2, dans laquelle :
a) un ou plusieurs triplets d'acide nucléique, qui codent pour des acides aminés qui ne sont pas essentiels à l'activité de mort cellulaire programmée induite par les cytokines, ont été délétés ou remplacés ; ou
b) un ou plusieurs triplets d'acide nucléique codant pour des acides aminés ont été ajoutés dans une région qui n'est pas essentielle à l'activité de mort cellulaire programmée induite par les cytokines ;
le produit d'expression codé par ladite séquence codante modifiée ayant la même activité de mort cellulaire programmée induite par les cytokines que le produit d'expression codé par l'une quelconque des séquences codantes telles que définies dans l'une ou l'autre des revendications 1 et 2, respectivement.

4. Fragment de l'une quelconque des molécules d'ADN isolées selon les revendications 1 à 3, ledit fragment codant pour une protéine ou un polypeptide conservant l'activité de mort cellulaire programmée induite par les cytokines présente dans la protéine ou le polypeptide codé par l'une quelconque des séquences codantes de l'une ou l'autre des revendications 1 et 2 à condition que la séquence nucléotidique 2556-2814 de la séquence de la figure 8 soit exclue.

5. Molécule qui comprend une séquence antisens de séquence complémentaire à l'ARNm transcrit à partir de la totalité ou d'une partie de l'une quelconque des molécules d'ADN selon la revendication 1, ladite séquence antisens étant susceptible d'inhiber l'expression de la séquence codante telle que définie dans la revendication 1.

6. Séquence d'ADN selon la revendication 1 modifiée dans laquelle un ou plusieurs triplets d'acide nucléique ont été ajoutés, délétés ou remplacés, la protéine ou le polypeptide codé par la séquence modifiée ayant un effet négatif dominant, étant susceptible d'inhiber la fonction de la protéine ou du polypeptide impliqué dans la mort cellulaire programmée induite par les cytokines codé par la séquence codante selon la revendication 1.

7. Vecteur comprenant une séquence d'ADN telle que définie dans la revendication 1 et des séquences nécessaires à la propagation et à la réplication de la séquence d'ADN dans une cellule hôte.

8. Vecteur selon la revendication 7, étant un vecteur d'expression comprenant également des séquences nécessaires à la transcription dudit ADN en un ARNm.

9. Vecteur selon la revendication 8, étant un plasmide épisomique construit à l'aide des vecteurs pTKO1, pTKO2 et pTKO3.

10. Protéine ou polypeptide comprenant une séquence d'acides aminés codée par une molécule d'ADN telle que définie dans l'une quelconque des revendications 1 à 4.

11. Protéine ou polypeptide selon la revendication 10, dans lequel un ou plusieurs acides aminés ont été chimiquement modifiés.

12. Protéine selon la revendication 10, ayant la séquence d'acides aminés telle que représentée sur la figure 8.

13. Anticorps levé contre une protéine selon l'une quelconque des revendications 10 à 12 ou contre une partie immunogène de celle-ci.

14. Composition pharmaceutique destinée à favoriser ou prévenir la mort de cellules cibles, comprenant un support pharmaceutiquement acceptable et un agent actif étant un vecteur d'expression qui comprend une séquence d'ADN étant sous contrôle d'expression, permettant une expression dans les cellules cibles, ladite séquence d'ADN étant choisie dans le groupe constitué par :
(a) une séquence codante débutant au triplet d'acide nucléique à la position 337-339 et se terminant au triplet à la position 4603-4605 de la séquence représentée sur la figure 8 ;
(b) une séquence d'ADN distincte de l'ADN défini en (a) qui code pour le même produit d'expression codé par la séquence d'ADN définie en (a) ;
(c) une séquence d'ADN de (a) ou (b) modifiée dans laquelle soit un ou plusieurs triplets d'acide nucléique codant pour des acides aminés qui ne sont pas essentiels à l'activité de mort cellulaire programmée induite par les cytokines ont été délétés ou remplacés, soit un ou plusieurs triplets d'acide nucléique codant pour des acides aminés ont été ajoutés dans une région qui n'est pas essentielle à l'activité de mort cellulaire programmée induite par les cytokines, la protéine ou le polypeptide codé par la séquence d'ADN modifiée ayant la même activité de mort cellulaire programmée induite par les cytokines que celui codé par l'une quelconque des molécules d'ADN de (a) ou (b), respectivement ; ou
(d) des fragments de l'une quelconque des séquences d'ADN de (a), (b) ou (c), codant pour une protéine ou un polypeptide ayant la même activité de mort cellulaire programmée induite par les cytokines que le produit d'expression codé par l'une quelconque des molécules d'ADN de (a) ou (b).

15. Composition pharmaceutique destinée au traitement du cancer, comprenant un support pharmaceutiquement acceptable et un agent actif étant un vecteur d'expression comprenant une séquence d'ADN telle que définie dans la revendication 14.

16. Composition pharmaceutique destinée au traitement du cancer, comprenant un support pharmaceutiquement acceptable et un principe actif étant une protéine ou un polypeptide codé par l'une quelconque des séquences d'ADN telles que définies dans la revendication 14.

17. Composition pharmaceutique selon la revendication 14, comprenant en outre une cytokine.

18. Composition pharmaceutique à utiliser pour l'inhibition de la destruction de cellules causée par la mort cellulaire programmée induite par les cytokines, comprenant un support pharmaceutiquement acceptable et un agent actif étant :
(a) une séquence d'ADN qui est un antisens de la totalité ou d'une partie de la séquence d'ADN comprenant une séquence codante débutant au triplet d'acide nucléique à la position 337-339 et se terminant au triplet à la position 4603-4605 de la séquence représentée sur la figure 8, ledit antisens étant susceptible d'inhiber l'expression de ladite séquence codante débutant au triplet d'acide nucléique à la position 337-339 et se terminant au triplet à la position 4603-4605 de la séquence représentée sur la figure 8 ; ou
(b) un anticorps levé contre l'un quelconque des protéines ou polypeptides codés par les séquences codantes telles que définies dans la revendication 1 ou 2.

19. Procédé *in vitro* de criblage pour une prédisposition à un cancer dans un échantillon d'ADN génomique provenant de cellules d'un individu ou d'ADNc produit à partir de l'ARNm desdites cellules, ledit procédé consistant à :
(a) ajouter une ou plusieurs sondes d'ADN, lesdites sondes comprenant une séquence d'ADN choisie dans le groupe constitué par :
(i) une séquence d'ADN codant pour un produit d'expression qui est impliqué dans la mort cellulaire programmée induite par les cytokines et comprenant une séquence codante débutant au triplet d'acide nucléique à la position 337-339 et se terminant au triplet à la position 4603-4605 de la séquence représentée sur la figure 8 ;
(ii) une séquence d'ADN codant pour un produit d'expression qui est impliqué dans la mort cellulaire programmée induite par les cytokines distincte de l'ADN défini en (i), qui code pour le même produit d'expression codé par la séquence codante définie en (i) ;
(iii) une séquence d'ADN de (i) ou (ii) modifiée dans laquelle un ou plusieurs triplets d'acide nucléique, qui codent pour des acides aminés qui ne sont pas essentiels à l'activité de mort cellulaire programmée induite par les cytokines, ont été délétés ou remplacés, ou un ou plusieurs triplets d'acide nucléique codant pour des acides aminés ont été ajoutés dans une région qui n'est pas essentielle à l'activité de mort cellulaire programmée induite par les cytokines, la protéine ou le polypeptide codé par la séquence d'ADN modifiée ayant la même activité de mort cellulaire programmée induite par les cytokines que le produit d'expression codé par l'une quelconque des molécules d'ADN de (i) ou (ii), respectivement ;
(iv) des fragments de l'une quelconque des séquences d'ADN de (i), (ii) ou (iii), codant pour une protéine ou un polypeptide ayant la même activité de mort cellulaire programmée induite par les cytokines que le produit d'expression codé par l'une quelconque des molécules d'ADN de (i) ou (ii) ;
(v) une séquence qui est un antisens de la totalité ou d'une partie de la molécule d'ADN telle que définie en (i), (ii), (iii) ou (iv), dont le produit d'expression est impliqué dans la mort cellulaire programmée induite par les cytokines, ledit antisens étant susceptible d'inhiber l'expression de ladite molécule d'ADN codant pour le produit d'expression ; et
(vi) une séquence d'ADN modifiée d'une molécule d'ADN telle que définie en (i) dans laquelle un ou plusieurs acides nucléiques ont été ajoutés, délétés ou remplacés, la protéine ou le polypeptide codé par la séquence modifiée ayant un effet négatif dominant se manifestant par la capacité de ladite protéine ou dudit polypeptide à inhiber ladite mort cellulaire programmée induite par les cytokines ; et
(b) fournir des conditions d'hybridation entre la sonde ou les sondes d'ADN et l'ADN dudit échantillon ;
(c) déterminer sur la base de l'hybridation si le gène DAP-2 est absent, s'il existe un appariement entre la séquence de la sonde ou des sondes d'ADN et une séquence dans l'ADN dudit échantillon ou s'il existe un mésappariement, un mésappariement ou une absence indiquant une délétion ou une mutation de l'ADN génomique et une prédisposition à un cancer de l'individu testé.

20. Procédé *in vitro* de détection d'une mutation dans un gène DAP-2 codant pour un produit impliqué dans la mort cellulaire programmée induite par les cytokines consistant à sonder un échantillon constitué par l'ADN génomique ou l'ADNc d'un individu avec une ou plusieurs sondes d'ADN comprenant une séquence complète ou partielle d'une séquence d'ADN comprenant une séquence codante débutant au triplet d'acide nucléique à la position 337-339 et se terminant au triplet 4603-4605 de la séquence représentée sur la figure 8.

21. Utilisation d'un vecteur d'expression selon la revendication 8 pour la préparation d'une composition pharmaceutique destinée au traitement du cancer.

22. Utilisation d'un vecteur d'expression comprenant une séquence d'ADN choisie dans le groupe constitué par :
(a) une séquence antisens telle que définie dans la revendication 5 ; et
(b) une séquence d'ADN modifiée telle que définie dans la revendication 6 ;
pour la préparation d'une composition pharmaceutique qui inhibe la destruction de cellules causée par la mort cellulaire programmée induite par les cytokines.

23. Utilisation comme agent actif d'un anticorps dirigé contre l'un quelconque des protéines ou polypeptides codés par les séquences d'ADN définies dans la revendication 1 ou 2, pour la préparation d'une composition pharmaceutique qui inhibe la destruction de cellules causée par la mort cellulaire programmée induite par les cytokines.


### ANNEX I

### SEQUENCE LISTING

(1) GENERAL INFORMATION:
   (i) APPLICANT:
      (A) NAME: YEDA RESEARCH AND DEVELOPMENT COMPANY LTD.
      (B) STREET: The Weizmann Institute of Science - P.O. Box 95
      (C) CITY: REHOVOT
      (E) COUNTRY: ISRAEL
      (F) POSTAL CODE (ZIP): 76100
   (ii) TITLE OF INVENTION: TUMOR SUPPRESSOR GENES, PROTEINS ENCODED THEREBY AND USE OF SAID GENES AND PROTEINS
   (iii) NUMBER OF SEQUENCES: 26
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: EP 95900994.5
   (vi) PRIOR APPLICATION DATA:
      (A) APPLICATION NUMBER: PCT/US94/11598
      (B) FILING DATE: 12-OCT-1994
   (vi) PRIOR APPLICATION DATA:
      (A) APPLICATION NUMBER: IL 107250
      (B) FILING DATE: 12-OCT-1993
(2) INFORMATION FOR SEQ ID NO: 1:
   (i) SEQUENCE CHARACTERISTICS:
      (A) LENGTH: 10 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 1:
   Lys Lys Arg Arg Thr Lys Ser Ser Arg Arg
   1               5                   10
(2) INFORMATION FOR SEQ ID NO: 2:
   (i) SEQUENCE CHARACTERISTICS:
      (A) LENGTH: 7 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 2:
   Pro Ser Thr Ala Ile Arg Glu
   1               5
(2) INFORMATION FOR SEQ ID NO: 3:
   (i) SEQUENCE CHARACTERISTICS:
      (A) LENGTH: 7 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 3:
   Pro Ser Ser Ala Leu Arg Glu
   1               5
(2) INFORMATION FOR SEQ ID NO: 4:
   (i) SEQUENCE CHARACTERISTICS:
      (A) LENGTH: 7 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 4:
   Pro Cys Thr Ala Ile Arg Glu
   1               5
(2) INFORMATION FOR SEQ ID NO: 5:
   (i) SEQUENCE CHARACTERISTICS:
      (A) LENGTH: 7 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 5:
   Lys Lys Ile Ala Leu Arg Glu
   1               5
(2) INFORMATION FOR SEQ ID NO: 6:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 6:
   Ala Leu Thr Thr Asp Gly Lys Thr
   1               5
(2) INFORMATION FOR SEQ ID NO: 7:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO: 7:
   Gly His Ser Gly Ser Gly Lys Thr
   1               5
(2) INFORMATION FOR SEQ ID NO: 8:
   (i) SEQUENCE CHARACTERISTICS:
      (A) LENGTH: 8 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Protein
      (B) LOCATION:1..2
      (D) OTHER INFORMATION:/note= "G at this location can be A"
   (ix) FEATURE:
      (A) NAME/KEY: Protein
      (B) LOCATION:7..8
      (D) OTHER INFORMATION:/note= "T at this location can be S"
   (xi) SEQUENCE DESCRIPTION: SEQ ID NO: 8:
   Gly Xaa Xaa Xaa Xaa Gly Lys Thr
   1               5
(2) INFORMATION FOR SEQ ID NO: 9:
   (i) SEQUENCE CHARACTERISTICS:
      (A) LENGTH: 2232 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION:160..468
      (D) OTHER INFORMATION:/note= "prevention of IFN-2 promoted cell death"
   (xi) SEQUENCE DESCRIPTION: SEQ ID NO: 9:
   CGTGGCACTC ACCCGGCTCG CGCGGCCCCG GCCGCCCACG CCGCGCGTCG TTCTCCCGCC      60
   CGCTCGCTCC CCGGCGCTCA CACCTGAGCT CACTCGCGCA CGCCCGCCCG GCCCGAGAAC     120
   CGCGCCGCCG CCTCGGCCCC GCGGAAGCCC CGCCGCGCC ATG TCT TCG CCT CCC       174
                                              Met Ser Ser Pro Pro
                                               1               5
   GAA GGG AAA CTA GAG ACT AAA GCT GGA CAC CCG CCC GCC GTG AAA GCT      222
   Glu Gly Lys Leu Glu Thr Lys Ala Gly His Pro Pro Ala Val Lys Ala
                    10                  15                  20
   GGT GGA ATG CGA ATT GTG CAG AAA CAC CCA CAT ACA GGA GAC ACC AAA      270
   Gly Gly Met Arg Ile Val Gln Lys His Pro His Thr Gly Asp Thr Lys
                25                  30                  35
   GAA GAG AAA GAC AAG GAT GAC CAG GAA TGG GAA AGC CCC AGT CCA CCT      318
   Glu Glu Lys Asp Lys Asp Asp Gln Glu Trp Glu Ser Pro Ser Pro Pro
            40                  45                  50
   AAA CCC ACT GTG TTC ATC TCT GGG GTC ATC GCC CGG GGT GAC AAA GAT      366
   Lys Pro Thr Val Phe Ile Ser Gly Val Ile Ala Arg Gly Asp Lys Asp
        55                  60                  65
   TTC CCC CCG GCG GCT GCG CAG GTG GCT CAC CAG AAG CCG CAT GCC TCC      414
   Phe Pro Pro Ala Ala Ala Gln Val Ala His Gln Lys Pro His Ala Ser
    70                  75                  80                  85
   ATG GAC AAG CAT CCT TCC CCA AGA ACC CAG CAC ATC CAG CAG CCA CGC      462
   Met Asp Lys His Pro Ser Pro Arg Thr Gln His Ile Gln Gln Pro Arg
                    90                  95                 100
   AAG TGA GCCTGGAGTC CACCAGCCTG CCCCATGGCC CCGGCTCTGC TGCACTTGGT       518
   Lys  *
   ATTTCCCTGA CAGAGAGAAC CAGCAGTTTC GCCCAAATCC TACTCTGCTG GGAAATCTAA     578
   GGCAAAACCA AGTGCTCTGT CCTTTGCCTT ACATTTCCAT ATTTAAAACT AGAAACAGCT     638
   TCAGCCCAAA CCTTGTTTAT GGGGAGTCTG GTTGGATGTC ATTTGAGGAT CATTGTGCCC     698
   CTAGAGGTGC CATTAGCAGA ATTTGCCAAG ATCCGAGAAA AATTTTAGCT TTAGTTCTAT     758
   TTCAGCAGTC ACCTGACGTC CTTGTCTATG GTCTTAAAAA CAAGAAGGCA CACATTTGAG     818
   AAGATGAGAT TAAGGTTAGG AGAAAACCTC AGTCATTGCA TGCTTTTTAG TATGGGCCAA     878
   TAAAATCTCA ACACCTGTGG GAGAGTAAGA ACTAAGGGAA TGAGTTTGGG CGCCCCCTCA     938
   TAAAGGACCT TAGAGGCAGG GAACAGCAAT GCCAAATTTC CCTCTCTCGT GAGATGGGGG     998
   ATCCTGTGCA GGCTGATGAG GCACCCATGA GAAAAGCCGA AAAAGCATGC ATCTTAGAAA    1058
   TAGCCCCTCA ATTCCAGGAG TCAACATGCC AAAGAATGAG GCTGGAGACA GGTAGCTCCG    1118
   AGGGAGGACT TCTGGCATGA GATCTCGGCA CGGCAAGCCC AGCATCGCCT CAGCCCAGAC    1178
   AGGCTCCACC AGGAGATCAA GCAAGGGCTG CCTTTCAGGA GTCACCTCCT GAGCCACTTC    1238
   AGAGTTCTGG AAGTGACCAC GGACCAGGGT GGAGGAATAG ACTTCTAGTT CATTCTGGGA    1298
   CACTTGAGCC AGAGAGTTGA AAGCTTGGAA AGACCAGATA AGAAACCTGC CCTTTGTCTC    1358
   CCTAGGGACA TGAGACACCA CATTCCATTT GTGCTAGAAA AACCTATCCA CTGATGAGTC    1418
   TAACTGTTCC AAACGCCTCC CACCTGGTGT GCACAGCTGC CTGGGTCCAT TGTCACTTGG    1478
   GTGCATCAGG TTGTCCTCCG ATTTTTAGAT GAGTTTCCTG TCTAGAGATG TCCTAGTCTG    1538
   CTCACTGGCT GGTGGCAGTA GGGTACCCTG CGTCCTCGAA AAGCCAGAGG GTTCACCTAG    1598
   TCAGACGAAA CTCCAGAACA GTGCTTGTGG AGGGCCTGAC TGTCCTGCTC ACCCACAGCC    1658
   GATCTGCTGC AGGTCAGCAA CTGTGTCGTG AGCAGCTGCC AACCACCAGC CTTTCTGGTG    1718
   CTGTTCTCCA GTTCACGTCT GCCAGCTGGT GAGGGCAGAG GCAGACCTGG TCAGACCCAG    1778
   CGCCCCTCCT CCCTGAGGGA GCATGGCACA GCCTCACACT TGAAAGACGG TGTTTGGTTT    1838
   CCCATCTAAT CAACTTAAGG GAAGCCGGCA TGTACCCTTC AAGGCCCTGT CACCACCTAT    1898
   TTTCCTGATC AGTTGGTATA AACTGAGGGT GGCTTTTAGA GACCCAGACT TGGTTGGCAG    1958
   CGCTGCCATG GAACACCCCA GCAAGCACCT CCCAGCCTGC CTTTCGGAGC AGCACCCAGG    2018
   AGGGGATGCC GCGCTCCAGC AACACCAGGT CAGGCCTGTG CAGACCCCTG CCCTGCCGCT    2078
   GCAGAAATCC AGAAGCATCC TTAATGCTTC TCAGTCTTCA GCCAGAGGGA GGGCTGTTAT    2138
   TTCCAGAGGT GCGCTTTTTA TGTACTTTTA GCTAGATGTG GCATGCATCT GTGAGCTTTA    2198
   GATCATTAAA TCCAAAATGT TTGCCTAAAT GAGG                               2232
(2) INFORMATION FOR SEQ ID NO: 10:
   (i) SEQUENCE CHARACTERISTICS:
      (A) LENGTH: 103 amino acids
      (B) TYPE: amino acid
      (D) TOPOLOGY: linear
   (ii) MOLECULE TYPE: protein
   (xi) SEQUENCE DESCRIPTION: SEQ ID NO: 10:
   Met Ser Ser Pro Pro Glu Gly Lys Leu Glu Thr Lys Ala Gly His Pro
     1               5                  10                  15
   Pro Ala Val Lys Ala Gly Gly Met Arg Ile Val Gln Lys His Pro His
                20                  25                  30
   Thr Gly Asp Thr Lys Glu Glu Lys Asp Lys Asp Asp Gln Glu Trp Glu
            35                  40                  45
   Ser Pro Ser Pro Pro Lys Pro Thr Val Phe Ile Ser Gly Val Ile Ala
        50                  55                  60
   Arg Gly Asp Lys Asp Phe Pro Pro Ala Ala Ala Gln Val Ala His Gln
    65                  70                  75                  80
   Lys Pro His Ala Ser Met Asp Lys His Pro Ser Pro Arg Thr Gln His
                    85                  90                  95
   Ile Gln Gln Pro Arg Lys  *
               100
(2) INFORMATION FOR SEQ ID NO: 11:
   (i) SEQUENCE CHARACTERISTICS:
      (A) LENGTH: 5886 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION:337..4605
      (D) OTHER INFORMATION:/note= "prevention of IFN-2 promoted cell death"
   (xi) SEQUENCE DESCRIPTION: SEQ ID NO: 11:
   CGGAGGACAG CCGGACCGAG CCAACGCCGG GGACTTTGTT CCCTCCACGG AGGGGACTCG      60
   GCAACTCGCA GCGGCAGGGT CTGGGGCCGG CGCCTGGGAG GGATCTGCGC CCCCCACTCA     120
   CTCCCTAGCT GTGTTCCCGC CGCCGCCCCG GCTAGTCTCC GGCGCTGGCG CCTATGGTCG     180
   GCCTCCGACA GCGCTCCGGA GGGACCGGGG GAGCTCCCAG GCGCCCGGGA CTGGAGACTG     240
   ATGCATGAGG GGCCTACGGA GGCGCAGGAG CGGTGGTGAT GGTCTGGGAA GCGGAGCTGA     300
   AGTCCCCTGG GCTTTGGTGA GGCGTGACAG TTTATC ATG ACC GTG TTC AGG CAG       354
                                           Met Thr Val Phe Arg Gln
                                               105
   GAA AAC GTG GAT GAT TAC TAC GAC ACC GGC GAG GAA CTT GGC AGT GGA       402
   Glu Asn Val Asp Asp Tyr Tyr Asp Thr Gly Glu Glu Leu Gly Ser Gly
   110                 115                 120                 125
   CAG TTT GCG GTT GTG AAG AAA TGC CGT GAG AAA AGT ACC GGC CTC CAG       450
   Gln Phe Ala Val Val Lys Lys Cys Arg Glu Lys Ser Thr Gly Leu Gln
                   130                 135                 140
   TAT CCC GCC AAA TTC ATC AAG AAA AGG AGG ACT AAG TCC AGC CGG CGG       498
   Tyr Pro Ala Lys Phe Ile Lys Lys Arg Arg Thr Lys Ser Ser Arg Arg
               145                 150                 155
   GGT GTG AGC CGC GAG GAC ATC GAG CGG GAG GTC AGC ATC CTG AAG GAG       546
   Gly Val Ser Arg Glu Asp Ile Glu Arg Glu Val Ser Ile Leu Lys Glu
           160                 165                 170
   ATC CAG CAC CCC AAT GTC ATC ACC CTG CAC GAG GTC TAT GAG AAC AAG       594
   Ile Gln His Pro Asn Val Ile Thr Leu His Glu Val Tyr Glu Asn Lys
       175                 180                 185
   ACG GAC GTC ATC CTG ATC TTG GAA CTC GTT GCA GGT GGC GAG CTG TTT       642
   Thr Asp Val Ile Leu Ile Leu Glu Leu Val Ala Gly Gly Glu Leu Phe
   190                 195                 200                 205
   GAC TTC TTA GCT GAA AAG GAA TCT TTA ACT GAA GAG GAA GCA ACT GAA       690
   Asp Phe Leu Ala Glu Lys Glu Ser Leu Thr Glu Glu Glu Ala Thr Glu
                   210                 215                 220
   TTT CTC AAA CAA ATT CTT AAT GGT GTT TAC TAC CTG CAC TCC CTT CAA       738
   Phe Leu Lys Gln Ile Leu Asn Gly Val Tyr Tyr Leu His Ser Leu Gln
               225                 230                 235
   ATC GCC CAC TTT GAT CTT AAG CCT GAG AAC ATA ATG CTT TTG GAT AGA       786
   Ile Ala His Phe Asp Leu Lys Pro Glu Asn Ile Met Leu Leu Asp Arg
           240                 245                 250
   AAT GTC CCC AAA CCT CGG ATC AAG ATC ATT GAC TTT GGA AAT GAA TTT       834
   Asn Val Pro Lys Pro Arg Ile Lys Ile Ile Asp Phe Gly Asn Glu Phe
       255                 260                 265
   AAA AAC ATA TTT GGG ACT CCA GAG TTT GTC GCT CCT GAG ATA GTC AAC       882
   Lys Asn Ile Phe Gly Thr Pro Glu Phe Val Ala Pro Glu Ile Val Asn
   270                 275                 280                 285
   TAT GAA CCT CTT GGT CTT GAG GCA GAT ATG TGG AGT ATC GGG GTA ATA       930
   Tyr Glu Pro Leu Gly Leu Glu Ala Asp Met Trp Ser Ile Gly Val Ile
                   290                 295                 300
   ACC TAT ATC CTC CTA AGT GGG GCC TCC CCA TTT CTT GGA GAC ACT AAG       978
   Thr Tyr Ile Leu Leu Ser Gly Ala Ser Pro Phe Leu Gly Asp Thr Lys
               305                 310                 315
   CAA GAA ACG TTA GCA AAT GTA TCC GCT GTC AAC TAC GAA TTT GAG GAT      1026
   Gln Glu Thr Leu Ala Asn Val Ser Ala Val Asn Tyr Glu Phe Glu Asp
           320                 325                 330
   GAA TAC TTC AGT AAT ACC AGT GCC CTA GCC AAA GAT TTC ATA AGA AGA      1074
   Glu Tyr Phe Ser Asn Thr Ser Ala Leu Ala Lys Asp Phe Ile Arg Arg
       335                 340                 345
   CTT CTG GTC AAG GAT CCA AAG AAG AGA ATG ACA ATT CAA GAT AGT TTG       1122
   Leu Leu Val Lys Asp Pro Lys Lys Arg Met Thr Ile Gln Asp Ser Leu
   350                 355                 360                 365
   CAG CAT CCC TGG ATC AAG CCT AAA GAT ACA CAA CAG GCA CTT AGT AGA       1170
   Gln His Pro Trp Ile Lys Pro Lys Asp Thr Gln Gln Ala Leu Ser Arg
                   370                 375                 380
   AAA GCA TCA GCA GTA AAC ATG GAG AAA TTC AAG AAG TTT GCA GCC CGG       1218
   Lys Ala Ser Ala Val Asn Met Glu Lys Phe Lys Lys Phe Ala Ala Arg
               385                 390                 395
   AAA AAA TGG AAA CAA TCC GTT CGC TTG ATA TCA CTG TGC CAA AGA TTA       1266
   Lys Lys Trp Lys Gln Ser Val Arg Leu Ile Ser Leu Cys Gln Arg Leu
           400                 405                 410
   TCC AGG TCA TTC CTG TCC AGA AGT AAC ATG AGT GTT GCC AGA AGC GAT       1314
   Ser Arg Ser Phe Leu Ser Arg Ser Asn Met Ser Val Ala Arg Ser Asp
       415                 420                 425
   GAT ACT CTG GAT GAG GAA GAC TCC TTT GTG ATG AAA GCC ATC ATC CAT       1362
   Asp Thr Leu Asp Glu Glu Asp Ser Phe Val Met Lys Ala Ile Ile His
   430                 435                 440                 445
   GCC ATC AAC GAT GAC AAT GTC CCA GGC CTG CAG CAC CTT CTG GGC TCA       1410
   Ala Ile Asn Asp Asp Asn Val Pro Gly Leu Gln His Leu Leu Gly Ser
                   450                 455                 460
   TTA TCC AAC TAT GAT GTT AAC CAA CCC AAC AAG CAC GGG ACA CCT CCA       1458
   Leu Ser Asn Tyr Asp Val Asn Gln Pro Asn Lys His Gly Thr Pro Pro
               465                 470                 475
   TTA CTC ATT GCT GCT GGC TGT GGG AAT ATT CAA ATA CTA CAG TTG CTC       1506
   Leu Leu Ile Ala Ala Gly Cys Gly Asn Ile Gln Ile Leu Gln Leu Leu
           480                 485                 490
   ATT AAA AGA GGC TCG AGA ATC GAT GTC CAG GAT AAG GGC GGG TCC AAT       1554
   Ile Lys Arg Gly Ser Arg Ile Asp Val Gln Asp Lys Gly Gly Ser Asn
       495                 500                 505
   GCC GTC TAC TGG GCT GCT CGG CAT GGC CAC GTC GAT ACC TTG AAA TTT       1602
   Ala Val Tyr Trp Ala Ala Arg His Gly His Val Asp Thr Leu Lys Phe
   510                 515                 520                 525
   CTC AGT GAG AAC AAA TGC CCT TTG GAT GTG AAA GAC AAG TCT GGA GAG       1650
   Leu Ser Glu Asn Lys Cys Pro Leu Asp Val Lys Asp Lys Ser Gly Glu
                   530                 535                 540
   ATG GCC CTC CAC GTG GCA GCT CGC TAT GGC CAT GCT GAC GTG GCT CAA       1698
   Met Ala Leu His Val Ala Ala Arg Tyr Gly His Ala Asp Val Ala Gln
               545                 550                 555
   GTT ACT TGT GCA GCT TCG GCT CAA ATC CCA ATA TCC AGG ACA AAG GAA       1746
   Val Thr Cys Ala Ala Ser Ala Gln Ile Pro Ile Ser Arg Thr Lys Glu
           560                 565                 570
   GAA GAA ACC CCC CTG CAC TGT GCT GCT TGG CAC GGC TAT TAC TCT GTG       1794
   Glu Glu Thr Pro Leu His Cys Ala Ala Trp His Gly Tyr Tyr Ser Val
       575                 580                 585
   GCC AAA GCC CTT TGT GAA GCC GGC TGT AAC GTG AAC ATC AAG AAC CGA      1842
   Ala Lys Ala Leu Cys Glu Ala Gly Cys Asn Val Asn Ile Lys Asn Arg
   590                 595                 600                 605
   GAA GGA GAG ACG CCC CTC CTG ACA GCC TCT GCC AGG GGC TAC CAC GAC      1890
   Glu Gly Glu Thr Pro Leu Leu Thr Ala Ser Ala Arg Gly Tyr His Asp
                   610                 615                 620
   ATC GTG GAG TGT CTG GCC GAA CAT GGA GCC GAC CTT AAT GCT TGC GAC      1938
   Ile Val Glu Cys Leu Ala Glu His Gly Ala Asp Leu Asn Ala Cys Asp
               625                 630                 635
   AAG GAC GGA CAC ATT GCC CTT CAT CTG GCT GTA AGA CGG TGT CAG ATG      1986
   Lys Asp Gly His Ile Ala Leu His Leu Ala Val Arg Arg Cys Gln Met
           640                 645                 650
   GAG GTA ATC AAG ACT CTC CTC AGC CAA GGG TGT TTC GTC GAT TAT CAA      2034
   Glu Val Ile Lys Thr Leu Leu Ser Gln Gly Cys Phe Val Asp Tyr Gln
       655                 660                 665
   GAC AGG CAC GGC AAT ACT CCC CTC CAT GTG GCA TGT AAA GAT GGC AAC      2082
   Asp Arg His Gly Asn Thr Pro Leu His Val Ala Cys Lys Asp Gly Asn
   670                 675                 680                 685
   ATG CCT ATC GTG GTG GCC CTC TGT GAA GCA AAC TGC AAT TTG GAC ATC      2130
   Met Pro Ile Val Val Ala Leu Cys Glu Ala Asn Cys Asn Leu Asp Ile
                   690                 695                 700
   TCC AAC AAG TAT GGG CGA ACG CCT CTG CAC CTT GCG GCC AAC AAC GGA      2178
   Ser Asn Lys Tyr Gly Arg Thr Pro Leu His Leu Ala Ala Asn Asn Gly
               705                 710                 715
   ATC CTA GAC GTG GTC CGG TAT CTC TGT CTG ATG GGA GCC AGC GTT GAG      2226
   Ile Leu Asp Val Val Arg Tyr Leu Cys Leu Met Gly Ala Ser Val Glu
           720                 725                 730
   GCG CTG ACC ACG GAC GGA AAG ACG GCA GAA GAT CTT GCT AGA TCG GAA      2274
   Ala Leu Thr Thr Asp Gly Lys Thr Ala Glu Asp Leu Ala Arg Ser Glu
       735                 740                 745
   CAG CAC GAG CAC GTA GCA GGT CTC CTT GCA AGA CTT CGA AAG GAT ACG      2322
   Gln His Glu His Val Ala Gly Leu Leu Ala Arg Leu Arg Lys Asp Thr
   750                 755                 760                 765
   CAC CGA GGA CTC TTC ATC CAG CAG CTC CGA CCC ACA CAG AAC CTG CAG      2370
   His Arg Gly Leu Phe Ile Gln Gln Leu Arg Pro Thr Gln Asn Leu Gln
                   770                 775                 780
   CCA AGA ATT AAG CTC AAG CTG TTT GGC CAC TCG GGA TCC GGG AAA ACC      2418
   Pro Arg Ile Lys Leu Lys Leu Phe Gly His Ser Gly Ser Gly Lys Thr
               785                 790                 795
   ACC CTT GTA GAA TCT CTC AAG TGT GGG CTG CTG AGG AGC TTT TTC AGA      2466
   Thr Leu Val Glu Ser Leu Lys Cys Gly Leu Leu Arg Ser Phe Phe Arg
           800                 805                 810
   AGG CGT CGG CCC AGA CTG TCT TCC ACC AAC TCC AGC AGG TTC CCA CCT      2514
   Arg Arg Arg Pro Arg Leu Ser Ser Thr Asn Ser Ser Arg Phe Pro Pro
       815                 820                 825
   TCA CCC CTG GCT TCT AAG CCC ACA GTC TCA GTG AGC ATC AAC AAC CTG        2562
   Ser Pro Leu Ala Ser Lys Pro Thr Val Ser Val Ser Ile Asn Asn Leu
   830                 835                 840                 845
   TAC CCA GGC TGC GAG AAC GTG AGT GTG AGG AGC CGC AGC ATG ATG TTC        2610
   Tyr Pro Gly Cys Glu Asn Val Ser Val Arg Ser Arg Ser Met Met Phe
                   850                 855                 860
   GAG CCG GGT CTT ACC AAA GGG ATG CTG GAG GTG TTT GTG GCC CCG ACC        2658
   Glu Pro Gly Leu Thr Lys Gly Met Leu Glu Val Phe Val Ala Pro Thr
               865                 870                 875
   CAC CAC CCG CAC TGC TCG GCC GAT GAC CAG TCC ACC AAG GCC ATC GAC        2706
   His His Pro His Cys Ser Ala Asp Asp Gln Ser Thr Lys Ala Ile Asp
           880                 885                 890
   ATC CAG AAC GCT TAT TTG AAT GGA GTT GGC GAT TTC AGC GTG TGG GAG        2754
   Ile Gln Asn Ala Tyr Leu Asn Gly Val Gly Asp Phe Ser Val Trp Glu
       895                 900                 905
   TTC TCT GGA AAT CCT GTG TAT TTC TGC TGT TAT GAC TAT TTT GCT GCA        2802
   Phe Ser Gly Asn Pro Val Tyr Phe Cys Cys Tyr Asp Tyr Phe Ala Ala
   910                 915                 920                 925
   AAT GAT CCC ACG TCA ATC CAT GTT GTT GTC TTT AGT CTA GAA GAG CCC        2850
   Asn Asp Pro Thr Ser Ile His Val Val Val Phe Ser Leu Glu Glu Pro
                   930                 935                 940
   TAT GAG ATC CAG CTG AAC CCA GTG ATT TTC TGG CTC AGT TTC CTG AAG        2898
   Tyr Glu Ile Gln Leu Asn Pro Val Ile Phe Trp Leu Ser Phe Leu Lys
               945                 950                 955
   TCC CTT GTC CCA GTT GAA GAA CCC ATA GCC TTC GGT GGC AAG CTG AAG        2946
   Ser Leu Val Pro Val Glu Glu Pro Ile Ala Phe Gly Gly Lys Leu Lys
           960                 965                 970
   AAC CCA CTC CAA GTT GTC CTG GTG GCC ACC CAC GCT GAC ATC ATG AAT        2994
   Asn Pro Leu Gln Val Val Leu Val Ala Thr His Ala Asp Ile Met Asn
       975                 980                 985
   GTT CCT CGA CCG GCT GGA GGC GAG TTT GGA TAT GAC AAA GAC ACA TCG        3042
   Val Pro Arg Pro Ala Gly Gly Glu Phe Gly Tyr Asp Lys Asp Thr Ser
   990                 995                 1000                1005
   TTG CTG AAA GAG ATT AGG AAC AGG TTT GGA AAT GAT CTT CAC ATT TCA        3090
   Leu Leu Lys Glu Ile Arg Asn Arg Phe Gly Asn Asp Leu His Ile Ser
                   1010                1015                1020
   AAT AAG CTG TTT GTT CTG GAT GCT GGG GCT TCT GGG TCA AAG GAC ATG        3138
   Asn Lys Leu Phe Val Leu Asp Ala Gly Ala Ser Gly Ser Lys Asp Met
               1025                1030                1035
   AAG GTA CTT CGA AAT CAT CTG CAA GAA ATA CGA AGC CAG ATT GTT TCG        3186
   Lys Val Leu Arg Asn His Leu Gln Glu Ile Arg Ser Gln Ile Val Ser
           1040                1045                1050
   GTC TGT CCT CCC ATG ACT CAC CTG TGT GAG AAA ATC ATC TCC ACG CTG        3234
   Val Cys Pro Pro Met Thr His Leu Cys Glu Lys Ile Ile Ser Thr Leu
       1055                1060                1065
   CCT TCC TGG AGG AAG CTC AAT GGA CCC AAC CAG CTG ATG TCG CTG CAG      3282
   Pro Ser Trp Arg Lys Leu Asn Gly Pro Asn Gln Leu Met Ser Leu Gln
   1070                1075                1080                1085
   CAG TTT GTG TAC GAC GTG CAG GAC CAG CTG AAC CCC CTG GCC AGC GAG      3330
   Gln Phe Val Tyr Asp Val Gln Asp Gln Leu Asn Pro Leu Ala Ser Glu
                   1090                1095                1100
   GAG GAC CTC AGG CGC ATT GCT CAG CAG CTC CAC AGC ACA GGC GAG ATC      3378
   Glu Asp Leu Arg Arg Ile Ala Gln Gln Leu His Ser Thr Gly Glu Ile
               1105                1110                1115
   AAC ATC ATG CAA AGT GAA ACA GTT CAG GAC GTG CTG CTC CTG GAC CCC      3426
   Asn Ile Met Gln Ser Glu Thr Val Gln Asp Val Leu Leu Leu Asp Pro
           1120                1125                1130
   CGC TGG CTC TGC ACA AAC GTC CTG GGG AAG TTG CTG TCC GTG GAG ACC      3474
   Arg Trp Leu Cys Thr Asn Val Leu Gly Lys Leu Leu Ser Val Glu Thr
       1135                1140                1145
   CCA CGG GCG CTG CAC CAC TAC CGG GGC CGC TAC ACC GTG GAG GAC ATC      3522
   Pro Arg Ala Leu His His Tyr Arg Gly Arg Tyr Thr Val Glu Asp Ile
   1150                1155                1160                1165
   CAG CGC CTG GTG CCC GAC AGC GAC GTG GAG GAG CTG CTG CAG ATC CTC      3570
   Gln Arg Leu Val Pro Asp Ser Asp Val Glu Glu Leu Leu Gln Ile Leu
                   1170                1175                1180
   GAT GCC ATG GAC ATC TGC GCC CGG GAC CTG AGC AGC GGG ACC ATG GTG      3618
   Asp Ala Met Asp Ile Cys Ala Arg Asp Leu Ser Ser Gly Thr Met Val
               1185                1190                1195
   GAC GTC CCA GCC CTG ATC AAG ACA GAC AAC CTG CAC CGC TCC TGG GCT      3666
   Asp Val Pro Ala Leu Ile Lys Thr Asp Asn Leu His Arg Ser Trp Ala
           1200                1205                1210
   GAT GAG GAG GAC GAG GTG ATG GTG TAT GGT GGC GTG CGC ATC GTG CCC      3714
   Asp Glu Glu Asp Glu Val Met Val Tyr Gly Gly Val Arg Ile Val Pro
       1215                1220                1225
   GTG GAA CAC CTC ACC CCC TTC CCA TGT GGC ATC TTT CAC AAG GTC CAG      3762
   Val Glu His Leu Thr Pro Phe Pro Cys Gly Ile Phe His Lys Val Gln
   1230                1235                1240                1245
   GTG AAC CTG TGC CGG TGG ATC CAC CAG CAA AGC ACA GAG GGC GAC GCG      3810
   Val Asn Leu Cys Arg Trp Ile His Gln Gln Ser Thr Glu Gly Asp Ala
                   1250                1255                1260
   GAC ATC CGC CTG TGG GTG AAT GGC TGC AAG CTG GCC AAC CGT GGG GCC      3858
   Asp Ile Arg Leu Trp Val Asn Gly Cys Lys Leu Ala Asn Arg Gly Ala
               1265                1270                1275
   GAG CTG CTG GTG CTG CTG GTC AAC CAC GGC CAG GGC ATT GAG GTC CAG      3906
   Glu Leu Leu Val Leu Leu Val Asn His Gly Gln Gly Ile Glu Val Gln
           1280                1285                1290
   GTC CGT GGC CTG GAG ACG GAG AAG ATC AAG TGC TGC CTG CTG CTG GAC      3954
   Val Arg Gly Leu Glu Thr Glu Lys Ile Lys Cys Cys Leu Leu Leu Asp
       1295                1300                1305
   TCG GTG TGC AGC ACC ATT GAG AAC GTC ATG GCC ACC ACG CTG CCA GGG      4002
   Ser Val Cys Ser Thr Ile Glu Asn Val Met Ala Thr Thr Leu Pro Gly
   1310                1315                1320                1325
   CTC CTG ACC GTG AAG CAT TAC CTG AGC CCC CAG CAG CTG CGG GAG CAC      4050
   Leu Leu Thr Val Lys His Tyr Leu Ser Pro Gln Gln Leu Arg Glu His
                   1330                1335                1340
   CAT GAG CCC GTC ATG ATC TAC CAG CCA CGG GAC TTC TTC CGG GCA CAG      4098
   His Glu Pro Val Met Ile Tyr Gln Pro Arg Asp Phe Phe Arg Ala Gln
               1345                1350                1355
   ACT CTG AAG GAA ACC TCA CTG ACC AAC ACC ATG GGG GGG TAC AAG GAA      4146
   Thr Leu Lys Glu Thr Ser Leu Thr Asn Thr Met Gly Gly Tyr Lys Glu
           1360                1365                1370
   AGC TTC AGC AGC ATC ATG TGC TTC GGG TGT CAC GAC GTC TAC TCA CAG      4194
   Ser Phe Ser Ser Ile Met Cys Phe Gly Cys His Asp Val Tyr Ser Gln
       1375                1380                1385
   GCC AGC CTC GGC ATG GAC ATC CAT GCA TCA GAC CTG AAC CTC CTC ACT      4242
   Ala Ser Leu Gly Met Asp Ile His Ala Ser Asp Leu Asn Leu Leu Thr
   1390                1395                1400                1405
   CGG AGG AAA CTG AGT CGC CTG CTG GAC CCG CCC GAC CCC CTG GGG AAG      4290
   Arg Arg Lys Leu Ser Arg Leu Leu Asp Pro Pro Asp Pro Leu Gly Lys
                   1410                1415                1420
   GAC TGG TGC CTT CTC GCC ATG AAC TTA GGC CTC CCT GAC CTC GTG GCA      4338
   Asp Trp Cys Leu Leu Ala Met Asn Leu Gly Leu Pro Asp Leu Val Ala
               1425                1430                1435
   AAG TAC AAC ACC AAT AAC GGG GCT CCC AAG GAT TTC CTC CCC AGC CCC      4386
   Lys Tyr Asn Thr Asn Asn Gly Ala Pro Lys Asp Phe Leu Pro Ser Pro
           1440                1445                1450
   CTC CAC GCC CTG CTG CGG GAA TGG ACC ACC TAC CCT GAG AGC ACA GTG      4434
   Leu His Ala Leu Leu Arg Glu Trp Thr Thr Tyr Pro Glu Ser Thr Val
       1455                1460                1465
   GGC ACC CTC ATG TCC AAA CTG AGG GAG CTG GGT CGC CGG GAT GCC GCA      4482
   Gly Thr Leu Met Ser Lys Leu Arg Glu Leu Gly Arg Arg Asp Ala Ala
   1470                1475                1480                1485
   GAC CTT TTG CTG AAG GCA TCC TCT GTG TTC AAA ATC AAC CTG GAT GGC      4530
   Asp Leu Leu Leu Lys Ala Ser Ser Val Phe Lys Ile Asn Leu Asp Gly
                   1490                1495                1500
   AAT GGC CAG GAG GCC TAT GCC TCG AGC TGC AAC AGC GGC ACC TCT TAC      4578
   Asn Gly Gln Glu Ala Tyr Ala Ser Ser Cys Asn Ser Gly Thr Ser Tyr
               1505                1510                1515
   AAT TCC ATT AGC TCT GTT GTA TCC CGG TGAGGGCAGC CTCTGGCTTG            4625
   Asn Ser Ile Ser Ser Val Val Ser Arg
           1520                1525
   GACAGGGTCT GTTTGGACTG CAGAACCAAG GGGGTGATGT AGCCCATCCT TCCCTTTGGA    4685
   GATGCTGAGG GTGTTTCTTC CTGCACCCAC AGCCAGGGGG ATGCCACTCC TCCCTCCGGC    4745
   TTGACCTGTT TCTCTGCCGC TACCTCCCTC CCCGTCTCAT TCCGTTGTCT GTGGATGGTC    4805
   ATTGCAGTTT AAGAGCAGAA CAGATCTTTT ACTTTGGCCG CTTGAAAAGC TAGTGTACCT    4865
   CCTCTCAGTG TTTTGGACTC CATCTCTCAT CCTCCAGTAC CTTGCTTCTT ACTGATAATT    4925
   TTGCTGGAAT TCCTAACTTT TCAATGACAT TTTTTTTAAC TATCATATTG ATTGTCCTTT    4985
   AAAAAAGAAA AGTGCATATT TATCCAAAAT GTGTATTTCT TATACGCTTT TCTGTGTTAT    5045
   ACCATTTCCT CAGCTTATCT CTTTTATATT TGTAGGAGAA ACTCCCATGT ATGGAATCCC    5105
   ACTGTATGAT TTATAAACAG ACAATATGTG AGTGCCTTTT GCAGAAGAGG GTGTGTTTGA    5165
   AATCATCGGA GTCAGCCAGG AGCTGTCACC AAGGAAACGC TACCTCTCTG TCCCTTGCTG    5225
   TATGCTGATC ATCGCCAGAG GTGCTTCACC CTGAGTTTTG TTTTGTATTG TTTTCTGACA    5285
   GTTTTTCTGT TTTGTTTGGC AAGGAAAGGG GAGAAGGGAA TCCTCCTCCA GGGTGATTTT    5345
   ATGATCAGTG TTGTTGCTCT AGGAAGACAT TTTTCCGTTT GCTTTTGTTC CAATGTCAAT    5405
   GTGAACGTCC ACATGAAACC TACACACTGT CATGCTTCAT CATTCCCTCT CATCTCAGGT    5465
   AGAAGGTTGA CACAGTTGTA GGGTTACAGA GACCTATGTA AGAATTCAGA AGACCCCTGA    5525
   CTCATCATTT GTGGCAGTCC CTTATAATTG GTGCATAGCA GATGGTTTCC ACATTTAGAT    5585
   CCTGGTTTCA TAACTTCTCG TACTTGAAGT CTAAAAGCAG AAAATAAAGG AAGCAAGTTT    5645
   TCTTCCATGA TTTTAAATTG TGATCGAGTT TTAAATTGAT AGGAGGGAAC ATGTCCTAAT    5705
   TCTTCTGTCC TGAGAAGCAT GTAATGTTAA TGTTATATCA TATGTATATA TATATATGCA    5765
   CTATGTATAT ACATATATAT TAATACTGGT ATTTTTACTT AATCTATAAA ATGTCGTTAA    5825
   AAAGTTGTTT GTTTTTTTCT TTTTTTATAA ATAAACTGTT GCTCGTTAAA AAAAAAAAAA    5885
   A                                                                   5886
(2) INFORMATION FOR SEQ ID NO: 12:
   (i) SEQUENCE CHARACTERISTICS:
      (A) LENGTH: 1423 amino acids
      (B) TYPE: amino acid
      (D) TOPOLOGY: linear
   (ii) MOLECULE TYPE: protein
   (xi) SEQUENCE DESCRIPTION: SEQ ID NO: 12:
   Met Thr Val Phe Arg Gln Glu Asn Val Asp Asp Tyr Tyr Asp Thr Gly
    1               5                  10                  15
   Glu Glu Leu Gly Ser Gly Gln Phe Ala Val Val Lys Lys Cys Arg Glu
               20                  25                  30
   Lys Ser Thr Gly Leu Gln Tyr Pro Ala Lys Phe Ile Lys Lys Arg Arg
           35                  40                  45
   Thr Lys Ser Ser Arg Arg Gly Val Ser Arg Glu Asp Ile Glu Arg Glu
       50                  55                  60
   Val Ser Ile Leu Lys Glu Ile Gln His Pro Asn Val Ile Thr Leu His
    65                  70                  75                  80
   Glu Val Tyr Glu Asn Lys Thr Asp Val Ile Leu Ile Leu Glu Leu Val
                    85                  90                  95
   Ala Gly Gly Glu Leu Phe Asp Phe Leu Ala Glu Lys Glu Ser Leu Thr
               100                 105                 110
   Glu Glu Glu Ala Thr Glu Phe Leu Lys Gln Ile Leu Asn Gly Val Tyr
           115                 120                 125
   Tyr Leu His Ser Leu Gln Ile Ala His Phe Asp Leu Lys Pro Glu Asn
       130                 135                 140
   Ile Met Leu Leu Asp Arg Asn Val Pro Lys Pro Arg Ile Lys Ile Ile
   145                 150                 155                 160
   Asp Phe Gly Asn Glu Phe Lys Asn Ile Phe Gly Thr Pro Glu Phe Val
                   165                 170                 175
   Ala Pro Glu Ile Val Asn Tyr Glu Pro Leu Gly Leu Glu Ala Asp Met
               180                 185                 190
   Trp Ser Ile Gly Val Ile Thr Tyr Ile Leu Leu Ser Gly Ala Ser Pro
           195                 200                 205
   Phe Leu Gly Asp Thr Lys Gln Glu Thr Leu Ala Asn Val Ser Ala Val
       210                 215                 220
   Asn Tyr Glu Phe Glu Asp Glu Tyr Phe Ser Asn Thr Ser Ala Leu Ala
   225                 230                 235                 240
   Lys Asp Phe Ile Arg Arg Leu Leu Val Lys Asp Pro Lys Lys Arg Met
                   245                 250                 255
   Thr Ile Gln Asp Ser Leu Gln His Pro Trp Ile Lys Pro Lys Asp Thr
               260                 265                 270
   Gln Gln Ala Leu Ser Arg Lys Ala Ser Ala Val Asn Met Glu Lys Phe
           275                 280                 285
   Lys Lys Phe Ala Ala Arg Lys Lys Trp Lys Gln Ser Val Arg Leu Ile
       290                 295                 300
   Ser Leu Cys Gln Arg Leu Ser Arg Ser Phe Leu Ser Arg Ser Asn Met
   305                 310                 315                 320
   Ser Val Ala Arg Ser Asp Asp Thr Leu Asp Glu Glu Asp Ser Phe Val
                   325                 330                 335
   Met Lys Ala Ile Ile His Ala Ile Asn Asp Asp Asn Val Pro Gly Leu
               340                 345                 350
   Gln His Leu Leu Gly Ser Leu Ser Asn Tyr Asp Val Asn Gln Pro Asn
           355                 360                 365
   Lys His Gly Thr Pro Pro Leu Leu Ile Ala Ala Gly Cys Gly Asn Ile
       370                 375                 380
   Gln Ile Leu Gln Leu Leu Ile Lys Arg Gly Ser Arg Ile Asp Val Gln
   385                 390                 395                 400
   Asp Lys Gly Gly Ser Asn Ala Val Tyr Trp Ala Ala Arg His Gly His
                   405                 410                 415
   Val Asp Thr Leu Lys Phe Leu Ser Glu Asn Lys Cys Pro Leu Asp Val
               420                 425                 430
   Lys Asp Lys Ser Gly Glu Met Ala Leu His Val Ala Ala Arg Tyr Gly
           435                 440                 445
   His Ala Asp Val Ala Gln Val Thr Cys Ala Ala Ser Ala Gln Ile Pro
       450                 455                 460
   Ile Ser Arg Thr Lys Glu Glu Glu Thr Pro Leu His Cys Ala Ala Trp
   465                 470                 475                 480
   His Gly Tyr Tyr Ser Val Ala Lys Ala Leu Cys Glu Ala Gly Cys Asn
                   485                 490                 495
   Val Asn Ile Lys Asn Arg Glu Gly Glu Thr Pro Leu Leu Thr Ala Ser
               500                 505                 510
   Ala Arg Gly Tyr His Asp Ile Val Glu Cys Leu Ala Glu His Gly Ala
           515                 520                 525
   Asp Leu Asn Ala Cys Asp Lys Asp Gly His Ile Ala Leu His Leu Ala
       530                 535                 540
   Val Arg Arg Cys Gln Met Glu Val Ile Lys Thr Leu Leu Ser Gln Gly
   545                 550                 555                 560
   Cys Phe Val Asp Tyr Gln Asp Arg His Gly Asn Thr Pro Leu His Val
                   565                 570                 575
   Ala Cys Lys Asp Gly Asn Met Pro Ile Val Val Ala Leu Cys Glu Ala
               580                 585                 590
   Asn Cys Asn Leu Asp Ile Ser Asn Lys Tyr Gly Arg Thr Pro Leu His
           595                 600                 605
   Leu Ala Ala Asn Asn Gly Ile Leu Asp Val Val Arg Tyr Leu Cys Leu
       610                 615                 620
   Met Gly Ala Ser Val Glu Ala Leu Thr Thr Asp Gly Lys Thr Ala Glu
   625                 630                 635                 640
   Asp Leu Ala Arg Ser Glu Gln His Glu His Val Ala Gly Leu Leu Ala
                   645                 650                 655
   Arg Leu Arg Lys Asp Thr His Arg Gly Leu Phe Ile Gln Gln Leu Arg
               660                 665                 670
   Pro Thr Gln Asn Leu Gln Pro Arg Ile Lys Leu Lys Leu Phe Gly His
           675                 680                 685
   Ser Gly Ser Gly Lys Thr Thr Leu Val Glu Ser Leu Lys Cys Gly Leu
       690                 695                 700
   Leu Arg Ser Phe Phe Arg Arg Arg Arg Pro Arg Leu Ser Ser Thr Asn
   705                 710                 715                 720
   Ser Ser Arg Phe Pro Pro Ser Pro Leu Ala Ser Lys Pro Thr Val Ser
                   725                 730                 735
   Val Ser Ile Asn Asn Leu Tyr Pro Gly Cys Glu Asn Val Ser Val Arg
               740                 745                 750
   Ser Arg Ser Met Met Phe Glu Pro Gly Leu Thr Lys Gly Met Leu Glu
           755                 760                 765
   Val Phe Val Ala Pro Thr His His Pro His Cys Ser Ala Asp Asp Gln
       770                 775                 780
   Ser Thr Lys Ala Ile Asp Ile Gln Asn Ala Tyr Leu Asn Gly Val Gly
   785                 790                 795                 800
   Asp Phe Ser Val Trp Glu Phe Ser Gly Asn Pro Val Tyr Phe Cys Cys
                   805                 810                 815
   Tyr Asp Tyr Phe Ala Ala Asn Asp Pro Thr Ser Ile His Val Val Val
               820                 825                 830
   Phe Ser Leu Glu Glu Pro Tyr Glu Ile Gln Leu Asn Pro Val Ile Phe
           835                 840                 845
   Trp Leu Ser Phe Leu Lys Ser Leu Val Pro Val Glu Glu Pro Ile Ala
       850                 855                 860
   Phe Gly Gly Lys Leu Lys Asn Pro Leu Gln Val Val Leu Val Ala Thr
   865                 870                 875                 880
   His Ala Asp Ile Met Asn Val Pro Arg Pro Ala Gly Gly Glu Phe Gly
                   885                 890                 895
   Tyr Asp Lys Asp Thr Ser Leu Leu Lys Glu Ile Arg Asn Arg Phe Gly
               900                 905                 910
   Asn Asp Leu His Ile Ser Asn Lys Leu Phe Val Leu Asp Ala Gly Ala
           915                 920                 925
   Ser Gly Ser Lys Asp Met Lys Val Leu Arg Asn His Leu Gln Glu Ile
       930                 935                 940
   Arg Ser Gln Ile Val Ser Val Cys Pro Pro Met Thr His Leu Cys Glu
   945                 950                 955                 960
   Lys Ile Ile Ser Thr Leu Pro Ser Trp Arg Lys Leu Asn Gly Pro Asn
                   965                 970                 975
   Gln Leu Met Ser Leu Gln Gln Phe Val Tyr Asp Val Gln Asp Gln Leu
               980                 985                 990
   Asn Pro Leu Ala Ser Glu Glu Asp Leu Arg Arg Ile Ala Gln Gln Leu
           995                 1000                1005
   His Ser Thr Gly Glu Ile Asn Ile Met Gln Ser Glu Thr Val Gln Asp
       1010                1015                1020
   Val Leu Leu Leu Asp Pro Arg Trp Leu Cys Thr Asn Val Leu Gly Lys
   1025                1030                1035                1040
   Leu Leu Ser Val Glu Thr Pro Arg Ala Leu His His Tyr Arg Gly Arg
                   1045                1050                1055
   Tyr Thr Val Glu Asp Ile Gln Arg Leu Val Pro Asp Ser Asp Val Glu
               1060                1065                1070
   Glu Leu Leu Gln Ile Leu Asp Ala Met Asp Ile Cys Ala Arg Asp Leu
           1075                1080                1085
   Ser Ser Gly Thr Met Val Asp Val Pro Ala Leu Ile Lys Thr Asp Asn
       1090                1095                1100
   Leu His Arg Ser Trp Ala Asp Glu Glu Asp Glu Val Met Val Tyr Gly
   1105                1110                1115                1120
   Gly Val Arg Ile Val Pro Val Glu His Leu Thr Pro Phe Pro Cys Gly
                   1125                1130                1135
   Ile Phe His Lys Val Gln Val Asn Leu Cys Arg Trp Ile His Gln Gln
               1140                1145                1150
   Ser Thr Glu Gly Asp Ala Asp Ile Arg Leu Trp Val Asn Gly Cys Lys
           1155                1160                1165
   Leu Ala Asn Arg Gly Ala Glu Leu Leu Val Leu Leu Val Asn His Gly
       1170                1175                1180
   Gln Gly Ile Glu Val Gln Val Arg Gly Leu Glu Thr Glu Lys Ile Lys
   1185                1190                1195                1200
   Cys Cys Leu Leu Leu Asp Ser Val Cys Ser Thr Ile Glu Asn Val Met
                   1205                1210                1215
   Ala Thr Thr Leu Pro Gly Leu Leu Thr Val Lys His Tyr Leu Ser Pro
               1220                1225                1230
   Gln Gln Leu Arg Glu His His Glu Pro Val Met Ile Tyr Gln Pro Arg
           1235                1240                1245
   Asp Phe Phe Arg Ala Gln Thr Leu Lys Glu Thr Ser Leu Thr Asn Thr
       1250                1255                1260
   Met Gly Gly Tyr Lys Glu Ser Phe Ser Ser Ile Met Cys Phe Gly Cys
   1265                1270                1275                1280
   His Asp Val Tyr Ser Gln Ala Ser Leu Gly Met Asp Ile His Ala Ser
                   1285                1290                1295
   Asp Leu Asn Leu Leu Thr Arg Arg Lys Leu Ser Arg Leu Leu Asp Pro
               1300                1305                1310
   Pro Asp Pro Leu Gly Lys Asp Trp Cys Leu Leu Ala Met Asn Leu Gly
           1315                1320                1325
   Leu Pro Asp Leu Val Ala Lys Tyr Asn Thr Asn Asn Gly Ala Pro Lys
       1330                1335                1340
   Asp Phe Leu Pro Ser Pro Leu His Ala Leu Leu Arg Glu Trp Thr Thr
   1345                1350                1355                1360
   Tyr Pro Glu Ser Thr Val Gly Thr Leu Met Ser Lys Leu Arg Glu Leu
                   1365                1370                1375
   Gly Arg Arg Asp Ala Ala Asp Leu Leu Leu Lys Ala Ser Ser Val Phe
               1380                1385                1390
   Lys Ile Asn Leu Asp Gly Asn Gly Gln Glu Ala Tyr Ala Ser Ser Cys
           1395                1400                1405
   Asn Ser Gly Thr Ser Tyr Asn Ser Ile Ser Ser Val Val Ser Arg
       1410                1415                1420
(2) INFORMATION FOR SEQ ID NO: 13:
   (i) SEQUENCE CHARACTERISTICS:
      (A) LENGTH: 1568 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION:74..1270
      (D) OTHER INFORMATION:/note= "prevention of IFN-2 promoted cell death"
   (xi) SEQUENCE DESCRIPTION: SEQ ID NO: 13:
   GAATTCCGCC GGCCCCAGGC AGCGTGTGTC GGTCGCCTAG GCTGGAGAAC TAGTCCTCGA      60
   CTCACGTGCA AGG ATG ATG CTG AAA GGA ATA ACA AGG CTT ATC TCT AGG        109
                  Met Met Leu Lys Gly Ile Thr Arg Leu Ile Ser Arg
                      1425                1430                1435
   ATC CAT AAG TTG GAC CCT GGG CGT TTT TTA CAC ATG GGG ACC CAG GCT        157
   Ile His Lys Leu Asp Pro Gly Arg Phe Leu His Met Gly Thr Gln Ala
                   1440                1445                1450
   CGC CAA AGC ATT GCT GCT CAC CTA GAT AAC CAG GTT CCA GTT GAG AGT        205
   Arg Gln Ser Ile Ala Ala His Leu Asp Asn Gln Val Pro Val Glu Ser
               1455                1460                1465
   CCG AGA GCT ATT TCC CGC ACC AAT GAG AAT GAC CCG GCC AAG CAT GGG        253
   Pro Arg Ala Ile Ser Arg Thr Asn Glu Asn Asp Pro Ala Lys His Gly
           1470                1475                1480
   GAT CAG CAC GAG GGT CAG CAC TAC AAC ATC TCC CCC CAG GAT TTG GAG        301
   Asp Gln His Glu Gly Gln His Tyr Asn Ile Ser Pro Gln Asp Leu Glu
       1485                1490                1495
   ACT GTA TTT CCC CAT GGC CTT CCT CCT CGC TTT GTG ATG CAG GTG AAG        349
   Thr Val Phe Pro His Gly Leu Pro Pro Arg Phe Val Met Gln Val Lys
   1500                1505                1510                1515
   ACA TTC AGT GAA GCT TGC CTG ATG GTA AGG AAA CCA GCC CTA GAA CTT        397
   Thr Phe Ser Glu Ala Cys Leu Met Val Arg Lys Pro Ala Leu Glu Leu
                   1520                1525                1530
   CTG CAT TAC CTG AAA AAC ACC AGT TTT GCT TAT CCA GCT ATA CGA TAT        445
   Leu His Tyr Leu Lys Asn Thr Ser Phe Ala Tyr Pro Ala Ile Arg Tyr
               1535                1540                1545
   CTT CTG TAT GGA GAG AAG GGA ACA GGA AAA ACC CTA AGT CTT TGC CAT        493
   Leu Leu Tyr Gly Glu Lys Gly Thr Gly Lys Thr Leu Ser Leu Cys His
           1550                1555                1560
   GTT ATT CAT TTC TGT GCA AAA CAG GAC TGG CTG ATA CTA CAT ATT CCA        541
   Val Ile His Phe Cys Ala Lys Gln Asp Trp Leu Ile Leu His Ile Pro
       1565                1570                1575
   GAT GCT CAT CTT TGG GTG AAA AAT TGT CGG GAT CTT CTG CAG TCC AGC        589
   Asp Ala His Leu Trp Val Lys Asn Cys Arg Asp Leu Leu Gln Ser Ser
   1580                1585                1590                1595
   TAC AAC AAA CAG CGC TTT GAT CAA CCT TTA GAG GCT TCA ACC TGG CTG        637
   Tyr Asn Lys Gln Arg Phe Asp Gln Pro Leu Glu Ala Ser Thr Trp Leu
                   1600                1605                1610
   AAG AAT TTC AAA ACT ACA AAT GAG CGC TTC CTG AAC CAG ATA AAA GTT        685
   Lys Asn Phe Lys Thr Thr Asn Glu Arg Phe Leu Asn Gln Ile Lys Val
               1615                1620                1625
   CAA GAG AAG TAT GTC TGG AAT AAG AGA GAA AGC ACT GAG AAA GGG AGT        733
   Gln Glu Lys Tyr Val Trp Asn Lys Arg Glu Ser Thr Glu Lys Gly Ser
           1630                1635                1640
   CCT CTG GGA GAA GTG GTT GAA CAG GGC ATA ACA CGG GTG AGG AAC GCC        781
   Pro Leu Gly Glu Val Val Glu Gln Gly Ile Thr Arg Val Arg Asn Ala
       1645                1650                1655
   ACA GAT GCA GTT GGA ATT GTG CTG AAA GAG CTA AAG AGG CAA AGT TCT        829
   Thr Asp Ala Val Gly Ile Val Leu Lys Glu Leu Lys Arg Gln Ser Ser
   1660                1665                1670                1675
   TTG GGT ATG TTT CAC CTC CTA GTG GCC GTG GAT GGA ATC AAT GCT CTT        877
   Leu Gly Met Phe His Leu Leu Val Ala Val Asp Gly Ile Asn Ala Leu
                   1680                1685                1690
   TGG GGA AGA ACC ACT CTG AAA AGA GAA GAT AAA AGC CCG ATT GCC CCC        925
   Trp Gly Arg Thr Thr Leu Lys Arg Glu Asp Lys Ser Pro Ile Ala Pro
               1695                1700                1705
   GAG GAA TTA GCA CTT GTT CAC AAC TTG AGG AAA ATG ATG AAA AAT GAT        973
   Glu Glu Leu Ala Leu Val His Asn Leu Arg Lys Met Met Lys Asn Asp
           1710                1715                1720
   TGG CAT GGA GGC GCC ATT GTG TCG GCT TTG AGC CAG ACT GGG TCT CTC       1021
   Trp His Gly Gly Ala Ile Val Ser Ala Leu Ser Gln Thr Gly Ser Leu
       1725                1730                1735
   TTT AAG CCC CGG AAA GCC TAT CTG CCC CAG GAG TTG CTG GGA AAG GAA       1069
   Phe Lys Pro Arg Lys Ala Tyr Leu Pro Gln Glu Leu Leu Gly Lys Glu
   1740                1745                1750                1755
   GGA TTT GAT GCC CTG GAT CCC TTT ATT CCC ATC CTG GTT TCC AAC TAT       1117
   Gly Phe Asp Ala Leu Asp Pro Phe Ile Pro Ile Leu Val Ser Asn Tyr
                   1760                1765                1770
   AAC CCA AAG GAA TTT GAA AGT TGT ATT CAG TAT TAT TTG GAA AAC AAT      1165
   Asn Pro Lys Glu Phe Glu Ser Cys Ile Gln Tyr Tyr Leu Glu Asn Asn
               1775                1780                1785
   TGG CTT CAA CAT GAG AAA GCT CCT ACA GAA GAA GGG AAA AAA GAG CTG      1213
   Trp Leu Gln His Glu Lys Ala Pro Thr Glu Glu Gly Lys Lys Glu Leu
           1790                1795                1800
   CTG TTC CTA AGT AAC GCG AAC CCC TCG CTG CTG GAG CGG CAC TGT GCC      1261
   Leu Phe Leu Ser Asn Ala Asn Pro Ser Leu Leu Glu Arg His Cys Ala
       1805                1810                1815
   TAC CTC TAA GCCAAGATCA CAGCATGTGA GGAAGACAGT GGACATCTGC              1310
   Tyr Leu  *
   1820
   TTTATGCTGG ACCCAGTAAG ATGAGGAAGT CGGGCAGTAC ACAGGAAGAG GAGCCAGGCC    1370
   CTTGTACCTA TGGGATTGGA CAGGACTGCA GTTGGCTCTG GACCTGCATT AAAATGGGTT    1430
   TCACTGTGAA TGCGTGACAA TAAGATATTC CCTTGTTCCT AAAACTTTAT ATCAGTTTAT    1490
   TGGATGTGGG TTTTTCACAT TTAAGATAAT TATGGCTCTT TTCCTAAAAA ATAAAATATC    1550
   TTTCTAAAAA AAAAAAAA                                                  1568
(2) INFORMATION FOR SEQ ID NO: 14:
   (i) SEQUENCE CHARACTERISTICS:
      (A) LENGTH: 399 amino acids
      (B) TYPE: amino acid
      (D) TOPOLOGY: linear
   (ii) MOLECULE TYPE: protein
   (xi) SEQUENCE DESCRIPTION: SEQ ID NO: 14:
   Met Met Leu Lys Gly Ile Thr Arg Leu Ile Ser Arg Ile His Lys Leu
    1               5                  10                  15
   Asp Pro Gly Arg Phe Leu His Met Gly Thr Gln Ala Arg Gln Ser Ile
               20                  25                  30
   Ala Ala His Leu Asp Asn Gln Val Pro Val Glu Ser Pro Arg Ala Ile
           35                  40                  45
   Ser Arg Thr Asn Glu Asn Asp Pro Ala Lys His Gly Asp Gln His Glu
       50                  55                  60
   Gly Gln His Tyr Asn Ile Ser Pro Gln Asp Leu Glu Thr Val Phe Pro
   65                  70                  75                  80
   His Gly Leu Pro Pro Arg Phe Val Met Gln Val Lys Thr Phe Ser Glu
                   85                  90                  95
   Ala Cys Leu Met Val Arg Lys Pro Ala Leu Glu Leu Leu His Tyr Leu
               100                 105                 110
   Lys Asn Thr Ser Phe Ala Tyr Pro Ala Ile Arg Tyr Leu Leu Tyr Gly
           115                 120                 125
   Glu Lys Gly Thr Gly Lys Thr Leu Ser Leu Cys His Val Ile His Phe
       130                 135                 140
   Cys Ala Lys Gln Asp Trp Leu Ile Leu His Ile Pro Asp Ala His Leu
   145                 150                 155                 160
   Trp Val Lys Asn Cys Arg Asp Leu Leu Gln Ser Ser Tyr Asn Lys Gln
                   165                 170                 175
   Arg Phe Asp Gln Pro Leu Glu Ala Ser Thr Trp Leu Lys Asn Phe Lys
               180                 185                 190
   Thr Thr Asn Glu Arg Phe Leu Asn Gln Ile Lys Val Gln Glu Lys Tyr
           195                 200                 205
   Val Trp Asn Lys Arg Glu Ser Thr Glu Lys Gly Ser Pro Leu Gly Glu
       210                 215                 220
   Val Val Glu Gln Gly Ile Thr Arg Val Arg Asn Ala Thr Asp Ala Val
   225                 230                 235                 240
   Gly Ile Val Leu Lys Glu Leu Lys Arg Gln Ser Ser Leu Gly Met Phe
                   245                 250                 255
   His Leu Leu Val Ala Val Asp Gly Ile Asn Ala Leu Trp Gly Arg Thr
               260                 265                 270
   Thr Leu Lys Arg Glu Asp Lys Ser Pro Ile Ala Pro Glu Glu Leu Ala
           275                 280                 285
   Leu Val His Asn Leu Arg Lys Met Met Lys Asn Asp Trp His Gly Gly
       290                 295                 300
   Ala Ile Val Ser Ala Leu Ser Gln Thr Gly Ser Leu Phe Lys Pro Arg
   305                 310                 315                 320
   Lys Ala Tyr Leu Pro Gln Glu Leu Leu Gly Lys Glu Gly Phe Asp Ala
                   325                 330                 335
   Leu Asp Pro Phe Ile Pro Ile Leu Val Ser Asn Tyr Asn Pro Lys Glu
               340                 345                 350
   Phe Glu Ser Cys Ile Gln Tyr Tyr Leu Glu Asn Asn Trp Leu Gln His
           355                 360                 365
   Glu Lys Ala Pro Thr Glu Glu Gly Lys Lys Glu Leu Leu Phe Leu Ser
       370                 375                 380
   Asn Ala Asn Pro Ser Leu Leu Glu Arg His Cys Ala Tyr Leu  *
   385                 390                 395
(2) INFORMATION FOR SEQ ID NO: 15:
   (i) SEQUENCE CHARACTERISTICS:
      (A) LENGTH: 132 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO: 15:
   CTAGATGAGG CAGATATAAG AGTCATGGAA AAAAGGACAG AGAAAAAAAA CAGACAAATC      60
   AGTTGTCAGT ATCCATGGCC TCTGATTCTG TCTCAACCAT GAAACAGAAG TGACACATAT     120
   ACCTGCTAAA AG                                                         132
(2) INFORMATION FOR SEQ ID NO: 16:
   (i) SEQUENCE CHARACTERISTICS:
      (A) LENGTH: 255 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO: 16:
   AACTCAGTCC CTTTCCTTCC AAAATCTCCA ACATGCGGTC CTTATTCTGA TCAATTTCTG      60
   GGAGCATTTT CTGCATATTG ACCTTGCTTT GTTGAAAAAG TTCTGTTAAC CATTCTCGAT     120
   CTTGTAATTT AGCTAACTGC TGAAGACAAA GTAGGAAGAG AGGAAAATGG GTGCCACTTT     180
   CTAGTGGTTG AGCTAGTTCT GAAATGCTCA CCAGCTCTGA AATGATGGCA CGAGCTGCAA     240
   ACTGTGCTAA ATAGC                                                      255
(2) INFORMATION FOR SEQ ID NO: 17:
   (i) SEQUENCE CHARACTERISTICS:
      (A) LENGTH: 2038 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION:52..1230
   (xi) SEQUENCE DESCRIPTION: SEQ ID NO: 17:
   GGCTATAAGC GCACGGCCTC GGCGACCCTC TCCGACCCGG CCGCCGCCGC C ATG CAG        57
                                                            Met Gln
                                                            400
   CCC TCC AGC CTT CTG CCG CTC GCC CTC TGC CTG CTG GCT GCA CCC GCC        105
   Pro Ser Ser Leu Leu Pro Leu Ala Leu Cys Leu Leu Ala Ala Pro Ala
               405                 410                 415
   TCC GCG CTC GTC AGG ATC CCG CTG CAC AAG TTC ACG TCC ATC CGC CGG        153
   Ser Ala Leu Val Arg Ile Pro Leu His Lys Phe Thr Ser Ile Arg Arg
           420                 425                 430
   ACC ATG TCG GAG GTT GGG GGC TCT GTG GAG GAC CTG ATT GCC AAA GGC        201
   Thr Met Ser Glu Val Gly Gly Ser Val Glu Asp Leu Ile Ala Lys Gly
       435                 440                 445
   CCC GTC TCA AAG TAC TCC CAG GCG GTG CCA GCC GTG ACC GAG GGG CCC        249
   Pro Val Ser Lys Tyr Ser Gln Ala Val Pro Ala Val Thr Glu Gly Pro
   450                 455                 460                 465
   ATT CCC GAG GTG CTC AAG AAC TAC ATG GAC GCC CAG TAC TAC GGG GAG        297
   Ile Pro Glu Val Leu Lys Asn Tyr Met Asp Ala Gln Tyr Tyr Gly Glu
                   470                 475                 480
   ATT GGC ATC GGG ACG CCC CCC CAG TGC TTC ACA GTC GTC TTC GAC ACG        345
   Ile Gly Ile Gly Thr Pro Pro Gln Cys Phe Thr Val Val Phe Asp Thr
               485                 490                 495
   GGC TCC TCC AAC CTG TGG GTC CCC TCC ATC CAC TGC AAA CTG CTG GAC        393
   Gly Ser Ser Asn Leu Trp Val Pro Ser Ile His Cys Lys Leu Leu Asp
           500                 505                 510
   ATC GCT TGC TGG ATC CAC CAC AAG TAC AAC AGC GAC AAG TCC AGC ACC        441
   Ile Ala Cys Trp Ile His His Lys Tyr Asn Ser Asp Lys Ser Ser Thr
       515                 520                 525
   TAC GTG AAG AAT GGT ACC TCG TTT GAC ATC CAC TAT GGC TCG GGC AGC        489
   Tyr Val Lys Asn Gly Thr Ser Phe Asp Ile His Tyr Gly Ser Gly Ser
   530                 535                 540                 545
   CTC TCC GGG TAC CTG AGC CAG GAC ACT GTG TCG GTG CCC TGC CAG TCA        537
   Leu Ser Gly Tyr Leu Ser Gln Asp Thr Val Ser Val Pro Cys Gln Ser
                   550                 555                 560
   GCG TCG TCA GCC TCT GCC CTG GGC GGT GTC AAA GTG GAG AGG CAG GTC        585
   Ala Ser Ser Ala Ser Ala Leu Gly Gly Val Lys Val Glu Arg Gln Val
               565                 570                 575
   TTT GGG GAG GCC ACC AAG CAG CCA GGC ATC ACC TTC ATC GCA GCC AAG        633
   Phe Gly Glu Ala Thr Lys Gln Pro Gly Ile Thr Phe Ile Ala Ala Lys
           580                 585                 590
   TTC GAT GGC ATC CTG GGC ATG GCC TAC CCC CGC ATC TCC GTC AAC AAC        681
   Phe Asp Gly Ile Leu Gly Met Ala Tyr Pro Arg Ile Ser Val Asn Asn
       595                 600                 605
   GTG CTG CCC GTC TTC GAC AAC CTG ATG CAG CAG AAG CTG GTG GAC CAG        729
   Val Leu Pro Val Phe Asp Asn Leu Met Gln Gln Lys Leu Val Asp Gln
   610                 615                 620                 625
   AAC ATC TTC TCC TTC TAC CTG AGC AGG GAC CCA GAT GCG CAG CCT GGG        777
   Asn Ile Phe Ser Phe Tyr Leu Ser Arg Asp Pro Asp Ala Gln Pro Gly
                   630                 635                 640
   GGT GAG CTG ATG CTG GGT GGC ACA GAC TCC AAG TAT TAC AAG GGT TCT        825
   Gly Glu Leu Met Leu Gly Gly Thr Asp Ser Lys Tyr Tyr Lys Gly Ser
               645                 650                 655
   CTG TCC TAC CTG AAT GTC ACC CGC AAG GCC TAC TGG CAG GTC CAC CTG        873
   Leu Ser Tyr Leu Asn Val Thr Arg Lys Ala Tyr Trp Gln Val His Leu
           660                 665                 670
   GAC CAG GTG GAG GTG GCC AGC GGG CTG ACC CTG TGC AAG GAG GGC TGT        921
   Asp Gln Val Glu Val Ala Ser Gly Leu Thr Leu Cys Lys Glu Gly Cys
       675                 680                 685
   GAG GCC ATT GTG GAC ACA GGC ACT TCC CTC ATG GTG GGC CCG GTG GAT        969
   Glu Ala Ile Val Asp Thr Gly Thr Ser Leu Met Val Gly Pro Val Asp
   690                 695                 700                 705
   GAG GTG CGC GAG CTG CAG AAG GCC ATC GGG GCC GTG CCG CTG ATT CAG       1017
   Glu Val Arg Glu Leu Gln Lys Ala Ile Gly Ala Val Pro Leu Ile Gln
                   710                 715                 720
   GGC GAG TAC ATG ATC CCC TGT GAG AAG GTG TCC ACC CTG CCC GCG ATC       1065
   Gly Glu Tyr Met Ile Pro Cys Glu Lys Val Ser Thr Leu Pro Ala Ile
               725                 730                 735
   ACA CTG AAG CTG GGA GGC AAA GGC TAC AAG CTG TCC CCA GAG GAC TAC       1113
   Thr Leu Lys Leu Gly Gly Lys Gly Tyr Lys Leu Ser Pro Glu Asp Tyr
           740                 745                 750
   ACG CTC AAG GTG TCG CAG GCC GGG AAG ACC CTC TGC CTG AGC GGC TTC       1161
   Thr Leu Lys Val Ser Gln Ala Gly Lys Thr Leu Cys Leu Ser Gly Phe
       755                 760                 765
   ATG GGC ATG GAC ATC CCG CCA CCC AGC GGG CCA CTC TGG ATC CTG GGC       1209
   Met Gly Met Asp Ile Pro Pro Pro Ser Gly Pro Leu Trp Ile Leu Gly
   770                 775                 780                 785
   GAC GTC TTC ATC GGC CGC TAC TACACTGTGT TTGACCGTGA CAACAACAGG          1260
   Asp Val Phe Ile Gly Arg Tyr
                   790
   GTGGGCTTCG CCGAGGCTGC CCGCCTCTAG TTCCCAAGGC GTCCGCGCGC CAGCACAGAA    1320
   ACAGAGGAGA GTCCCAGAGC AGGAGGCCCC TGGCCCAGCG GCCCCTCCCA CACACACCCA    1380
   CACACTCGCC CGCCCACTGT CCTGGGCGCC CTGGAAGCCG GCGGCCCAAG CCCGACTTGC    1440
   TGTTTTGTTC TGTGGTTTTC CCCTCCCTGG GTTCAGAAAT GCTGCCTGCC TGTCTGTCTC    1500
   TCCATCTGTT TGGTGGGGGT AGAGCTGATC CAGAGCACAG ATCTGTTTCG TGCATTGGAA    1560
   GACCCCACCC AAGCTTGGCA GCCGAGCTCG TGTATCCTGG GGCTCCCTTC ATCTCCAGGG    1620
   AGTCCCCTCC CCGGCCCTAC CAGCGCCCGC TGGGCTGAGC CCCTACCCCA CACCAGGCCG    1680
   TCCTCCCGGG CCCTCCCTTG GAAACCTGCC CTGCCTGAGG GCCCCTCTGC CCAGCTTGGG    1740
   CCCAGCTGGG CTCTGCCACC CTACCTGTTC AGTGTCCCGG GCCCGTTGAG GATGAGGCCG    1800
   CTAGAGGCCT GAGGATGAGC TGGAAGGAGT GAGAGGGGAC AAAACCCACC TTGTTGGAGC    1860
   CTGCAGGGTG GTGCTGGGAC TGAGCCAGTC CCAGGGGCAT GTATTGGCCT GGAGGTGGGG    1920
   TTGGGATTGG GGGCTGGTGC CAGCCTTCCT CTGCAGCTGA CCTCTGTTGT CCTCCCCTTG    1980
   GGCGGCTGAG AGCCCCAGCT GACATGGAAA TACAGTTGTT GGCCTCCGGC CTCCCCTC      2038
(2) INFORMATION FOR SEQ ID NO: 18:
   (i) SEQUENCE CHARACTERISTICS:
      (A) LENGTH: 393 amino acids
      (B) TYPE: amino acid
      (D) TOPOLOGY: linear
   (ii) MOLECULE TYPE: protein
   (xi) SEQUENCE DESCRIPTION: SEQ ID NO: 18:
   Met Gln Pro Ser Ser Leu Leu Pro Leu Ala Leu Cys Leu Leu Ala Ala
     1               5                  10                  15
   Pro Ala Ser Ala Leu Val Arg Ile Pro Leu His Lys Phe Thr Ser Ile
               20                  25                  30
   Arg Arg Thr Met Ser Glu Val Gly Gly Ser Val Glu Asp Leu Ile Ala
           35                  40                  45
   Lys Gly Pro Val Ser Lys Tyr Ser Gln Ala Val Pro Ala Val Thr Glu
       50                  55                  60
   Gly Pro Ile Pro Glu Val Leu Lys Asn Tyr Met Asp Ala Gln Tyr Tyr
    65                  70                  75                  80
   Gly Glu Ile Gly Ile Gly Thr Pro Pro Gln Cys Phe Thr Val Val Phe
                   85                  90                  95
   Asp Thr Gly Ser Ser Asn Leu Trp Val Pro Ser Ile His Cys Lys Leu
               100                 105                 110
   Leu Asp Ile Ala Cys Trp Ile His His Lys Tyr Asn Ser Asp Lys Ser
           115                 120                 125
   Ser Thr Tyr Val Lys Asn Gly Thr Ser Phe Asp Ile His Tyr Gly Ser
       130                 135                 140
   Gly Ser Leu Ser Gly Tyr Leu Ser Gln Asp Thr Val Ser Val Pro Cys
   145                 150                 155                 160
   Gln Ser Ala Ser Ser Ala Ser Ala Leu Gly Gly Val Lys Val Glu Arg
                   165                 170                 175
   Gln Val Phe Gly Glu Ala Thr Lys Gln Pro Gly Ile Thr Phe Ile Ala
               180                 185                 190
   Ala Lys Phe Asp Gly Ile Leu Gly Met Ala Tyr Pro Arg Ile Ser Val
           195                 200                 205
   Asn Asn Val Leu Pro Val Phe Asp Asn Leu Met Gln Gln Lys Leu Val
       210                 215                 220
   Asp Gln Asn Ile Phe Ser Phe Tyr Leu Ser Arg Asp Pro Asp Ala Gln
   225                 230                 235                 240
   Pro Gly Gly Glu Leu Met Leu Gly Gly Thr Asp Ser Lys Tyr Tyr Lys
                   245                 250                 255
   Gly Ser Leu Ser Tyr Leu Asn Val Thr Arg Lys Ala Tyr Trp Gln Val
               260                 265                 270
   His Leu Asp Gln Val Glu Val Ala Ser Gly Leu Thr Leu Cys Lys Glu
           275                 280                 285
   Gly Cys Glu Ala Ile Val Asp Thr Gly Thr Ser Leu Met Val Gly Pro
       290                 295                 300
   Val Asp Glu Val Arg Glu Leu Gln Lys Ala Ile Gly Ala Val Pro Leu
   305                 310                 315                 320
   Ile Gln Gly Glu Tyr Met Ile Pro Cys Glu Lys Val Ser Thr Leu Pro
                   325                 330                 335
   Ala Ile Thr Leu Lys Leu Gly Gly Lys Gly Tyr Lys Leu Ser Pro Glu
               340                 345                 350
   Asp Tyr Thr Leu Lys Val Ser Gln Ala Gly Lys Thr Leu Cys Leu Ser
           355                 360                 365
   Gly Phe Met Gly Met Asp Ile Pro Pro Pro Ser Gly Pro Leu Trp Ile
       370                 375                 380
   Leu Gly Asp Val Phe Ile Gly Arg Tyr
   385                 390
(2) INFORMATION FOR SEQ ID NO: 19:
   (i) SEQUENCE CHARACTERISTICS:
      (A) LENGTH: 331 amino acids
      (B) TYPE: amino acid
      (C) STRANDEDNESS: double
      (D) TOPOLOGY: linear
   (ii) MOLECULE TYPE: DNA (genomic)
   (xi) SEQUENCE DESCRIPTION: SEQ ID NO: 19:
   Met Thr Val Phe Arg Gln Glu Asn Val Asp Asp Tyr Tyr Asp Thr Gly
   1               5                   10                  15
   Glu Glu Leu Gly Ser Gly Gln Phe Ala Val Val Lys Lys Cys Arg Glu
               20                  25                  30
   Lys Ser Thr Gly Leu Gln Tyr Pro Ala Lys Phe Ile Lys Lys Arg Arg
           35                  40                  45
   Thr Lys Ser Ser Arg Arg Gly Val Ser Arg Glu Asp Ile Glu Arg Glu
       50                  55                  60
   Val Ser Ile Leu Lys Glu Ile Gln His Pro Asn Val Ile Thr Leu His
   65                  70                  75                  80
   Glu Val Tyr Glu Asn Lys Thr Asp Val Ile Leu Ile Leu Glu Leu Val
                   85                  90                  95
   Ala Gly Gly Glu Leu Phe Asp Phe Leu Ala Glu Lys Glu Ser Leu Thr
               100                 105                 110
   Glu Glu Glu Ala Thr Glu Phe Leu Lys Gln Ile Leu Asn Gly Val Tyr
           115                 120                 125
   Tyr Leu His Ser Leu Gln Ile Ala His Phe Asp Leu Lys Pro Glu Asn
       130                 135                 140
   Ile Met Leu Leu Asp Arg Asn Val Pro Lys Pro Arg Ile Lys Ile Ile
   145                 150                 155                 160
   Asp Phe Gly Asn Glu Phe Lys Asn Ile Phe Gly Thr Pro Glu Phe Val
                   165                 170                 175
   Ala Pro Glu Ile Val Asn Tyr Glu Pro Leu Gly Leu Glu Ala Asp Met
               180                 185                 190
   Trp Ser Ile Gly Val Ile Thr Tyr Ile Leu Leu Ser Gly Ala Ser Pro
           195                 200                 205
   Phe Leu Gly Asp Thr Lys Gln Glu Thr Leu Ala Asn Val Ser Ala Val
       210                 215                 220
   Asn Tyr Glu Phe Glu Asp Glu Tyr Phe Ser Asn Thr Ser Ala Leu Ala
   225                 230                 235                 240
   Lys Asp Phe Ile Arg Arg Leu Leu Val Lys Asp Pro Lys Lys Arg Met
                   245                 250                 255
   Thr Ile Gln Asp Ser Leu Gln His Pro Trp Ile Lys Pro Lys Asp Thr
               260                 265                 270
   Gln Gln Ala Leu Ser Arg Lys Ala Ser Ala Val Asn Met Glu Lys Phe
           275                 280                 285
   Lys Lys Phe Ala Ala Arg Lys Lys Trp Lys Gln Ser Val Arg Leu Ile
       290                 295                 300
   Ser Leu Cys Gln Arg Leu Ser Arg Ser Phe Leu Ser Arg Ser Asn Met
   305                 310                 315                 320
   Ser Val Ala Arg Ser Asp Asp Thr Leu Asp Glu
                   325                 330
(2) INFORMATION FOR SEQ ID NO: 20:
   (i) SEQUENCE CHARACTERISTICS:
      (A) LENGTH: 33 amino acids
      (B) TYPE: amino acid
      (C) STRANDEDNESS: double
      (D) TOPOLOGY: linear
   (ii) MOLECULE TYPE: DNA (genomic)
   (ix) FEATURE:
      (A) NAME/KEY: Domain
      (B) LOCATION:1..33
      (D) OTHER INFORMATION:/note= "AR2"
   (xi) SEQUENCE DESCRIPTION: SEQ ID NO: 20:
   Asp Val Gln Asp Lys Gly Gly Ser Asn Ala Val Tyr Trp Ala Ala Arg
   1               5                   10                  15
   His Gly His Val Asp Thr Leu Lys Phe Leu Ser Glu Asn Lys Cys Pro
               20                  25                  30
   Leu
(2) INFORMATION FOR SEQ ID NO: 21:
   (i) SEQUENCE CHARACTERISTICS:
      (A) LENGTH: 33 amino acids
      (B) TYPE: amino acid
      (C) STRANDEDNESS: double
      (D) TOPOLOGY: linear
   (ii) MOLECULE TYPE: DNA (genomic)
   (ix) FEATURE:
      (A) NAME/KEY: Domain
      (B) LOCATION:1..33
      (D) OTHER INFORMATION:/note= "ar3"
   (xi) SEQUENCE DESCRIPTION: SEQ ID NO: 21:
   Asp Val Lys Asp Lys Ser Gly Glu Met Ala Leu His Val Ala Ala Arg
   1               5                   10                  15
   Tyr Gly His Ala Asp Val Ala Gln Val Thr Cys Ala Ala Ser Ala Gln
               20                  25                  30
   Ile
(2) INFORMATION FOR SEQ ID NO: 22:
   (i) SEQUENCE CHARACTERISTICS:
      (A) LENGTH: 33 amino acids
      (B) TYPE: amino acid
      (C) STRANDEDNESS: double
      (D) TOPOLOGY: linear
   (ii) MOLECULE TYPE: DNA (genomic)
   (ix) FEATURE:
      (A) NAME/KEY: Domain
      (B) LOCATION:1..33
      (D) OTHER INFORMATION:/note= "ar4"
   (xi) SEQUENCE DESCRIPTION: SEQ ID NO: 22:
   Ile Ser Arg Thr Lys Glu Glu Glu Thr Pro Leu His Cys Ala Ala Trp
   1               5                   10                  15
   His Gly Tyr Tyr Ser Val Ala Lys Ala Leu Cys Glu Ala Gly Cys Asn
               20                  25                  30
   Val
(2) INFORMATION FOR SEQ ID NO: 23:
   (i) SEQUENCE CHARACTERISTICS:
      (A) LENGTH: 33 amino acids
      (B) TYPE: amino acid
      (C) STRANDEDNESS: double
      (D) TOPOLOGY: linear
   (ii) MOLECULE TYPE: DNA (genomic)
   (ix) FEATURE:
      (A) NAME/KEY: Domain
      (B) LOCATION:1..33
      (D) OTHER INFORMATION:/note= "ar5"
   (xi) SEQUENCE DESCRIPTION: SEQ ID NO: 23:
   Asn Ile Lys Asn Arg Glu Gly Glu Thr Pro Leu Leu Thr Ala Ser Ala
   1               5                   10                  15
   Arg Gly Tyr Met Asp Ile Val Glu Cys Leu Ala Glu His Gly Ala Asp
               20                  25                  30
   Leu
(2) INFORMATION FOR SEQ ID NO: 24:
   (i) SEQUENCE CHARACTERISTICS:
      (A) LENGTH: 33 amino acids
      (B) TYPE: amino acid
      (C) STRANDEDNESS: double
      (D) TOPOLOGY: linear
   (ii) MOLECULE TYPE: DNA (genomic)
   (ix) FEATURE:
      (A) NAME/KEY: Domain
      (B) LOCATION:1..33
      (D) OTHER INFORMATION:/note= "ar6"
   (xi) SEQUENCE DESCRIPTION: SEQ ID NO: 24:
   Asn Ala Cys Asp Lys Asp Gly His Ile Ala Leu His Leu Ala Val Arg
   1               5                   10                  15
   Arg Cys Gln Met Glu Val Ile Lys Thr Leu Leu Ser Gln Gly Cys Phe
               20                  25                  30
   Val
(2) INFORMATION FOR SEQ ID NO: 25:
   (i) SEQUENCE CHARACTERISTICS:
      (A) LENGTH: 33 amino acids
      (B) TYPE: amino acid
      (C) STRANDEDNESS: double
      (D) TOPOLOGY: linear
   (ii) MOLECULE TYPE: DNA (genomic)
   (ix) FEATURE:
      (A) NAME/KEY: Domain
      (B) LOCATION:1..33
      (D) OTHER INFORMATION:/note= "ar7"
   (xi) SEQUENCE DESCRIPTION: SEQ ID NO: 25:
   Asp Tyr Gln Asp Arg His Gly Asn Thr Pro Leu His Val Ala Cys Lys
   1               5                   10                  15
   Asp Gly Asn Met Pro Ile Val Val Ala Leu Cys Glu Ala Asn Cys Asn
               20                  25                  30
   Leu
(2) INFORMATION FOR SEQ ID NO: 26:
   (i) SEQUENCE CHARACTERISTICS:
      (A) LENGTH: 33 amino acids
      (B) TYPE: amino acid
      (C) STRANDEDNESS: double
      (D) TOPOLOGY: linear
   (ii) MOLECULE TYPE: DNA (genomic)
   (ix) FEATURE:
      (A) NAME/KEY: Domain
      (B) LOCATION:1..33
      (D) OTHER INFORMATION:/note= "ar8"
   (xi) SEQUENCE DESCRIPTION: SEQ ID NO: 26:
   Asp Ile Ser Asn Lys Tyr Gly Arg Thr Pro Leu His Leu Ala Ala Asn
   1               5                   10                  15
   Asn Gly Ile Leu Asp Val Val Arg Tyr Leu Cys Leu Met Gly Ala Ser
               20                  25                  30
   Val